# EUROPÄISCHE PATENTANMELDUNG

(11) **EP 2 639 308 A1**
(43) Veröffentlichungstag der Anmeldung: **18.09.2013**
(21) Anmeldenummer: 12159087.1
(22) Anmeldetag: 12.03.2012
(51) Int. Cl.: C12N 15/52, C12N 9/00, C12N 9/10, C12P 7/42, C12P 7/62, C12P 7/64, C12P 13/00

(54) **Enzymatische omega-Oxidation und -Aminierung von Fettsäuren**

(71) Anmelder: Evonik Industries AG, 45128 Essen (DE)
(72) Erfinder: Schaffer, Steffen, Dr., 45699 Herten (DE); Hauberg, Michaela, 45130 Essen (DE); Wessel, Mirja, Dr., 44799 Bochum (DE); Hennemann, Hans-Georg, Dr., 45770 Marl (DE); Pfeffer, Jan-Christoph, Dr., 45355 Essen (DE); Hass, Thomas, Dr., 48161 Münster (DE); Häger, Harald, Dr., 59348 Lüdinghausen (DE)

(57) **Zusammenfassung**

Die Erfindung betrifft ein Verfahren zur Oxidation einer Fettsäure oder eines Esters davon der Formel (I)

H₃C - (CH₂)ₙ - COOR (I),

wobei R aus der Gruppe ausgewählt ist, die H, Methyl, Ethyl, Propyl und Butyl umfasst,
wobei n 0 bis 30, bevorzugt 6 bis 24 ist,
umfassend den Schritt Oxidieren der Fettsäure oder des Esters davon durch Kontaktieren mit einer Cytochrom P450-Monooxygenase der CYP153-Familie in Anwesenheit von molekularem Sauerstoff und NAD(P)H und einen Ganzzellkatalysator exprimierend eine rekombinante Cytochrom P450-Monooxygenase der CYP153-Familie, eine rekombinante Alkoholdehydrogenase, eine rekombinante Transaminase und optional ein oder mehr als ein rekombinantes Enzym aus der Gruppe umfassend Alanindehydrogenase, Ferredoxin und Ferredoxin-Reduktase sowie die Verwendung dieses Ganzzellkatalysators zur Oxidation einer Fettsäure oder eines Esters davon.

SEQUENCE LISTING

<110>  Evonik Industries AG

<120>  Endständige Oxidation und Aminierung von Fettsäuren

<130>  2012E00056 DE

<160>  49

<170>  PatentIn version 3.5

<210>  1
<211>  230
<212>  PRT
<213>  Pseudomonas oleovorans

<400>  1

Met Ser Phe Ser Asn Tyr Lys Val Ile Ala Met Pro Val Leu Val Ala
1               5                   10                  15

Asn Phe Val Leu Gly Ala Ala Thr Ala Trp Ala Asn Glu Asn Tyr Pro
            20                  25                  30

Ala Lys Ser Ala Gly Tyr Asn Gln Gly Asp Trp Val Ala Ser Phe Asn
        35                  40                  45

Phe Ser Lys Val Tyr Val Gly Glu Glu Leu Gly Asp Leu Asn Val Gly
    50                  55                  60

Gly Gly Ala Leu Pro Asn Ala Asp Val Ser Ile Gly Asn Asp Thr Thr
65                  70                  75                  80

Leu Thr Phe Asp Ile Ala Tyr Phe Val Ser Ser Asn Ile Ala Val Asp
                85                  90                  95

Phe Phe Val Gly Val Pro Ala Arg Ala Lys Phe Gln Gly Glu Lys Ser
            100                 105                 110

Ile Ser Ser Leu Gly Arg Val Ser Glu Val Asp Tyr Gly Pro Ala Ile
        115                 120                 125

Leu Ser Leu Gln Tyr His Tyr Asp Ser Phe Glu Arg Leu Tyr Pro Tyr
    130                 135                 140

Val Gly Val Gly Val Gly Arg Val Leu Phe Phe Asp Lys Thr Asp Gly
145                 150                 155                 160

Ala Leu Ser Ser Phe Asp Ile Lys Asp Lys Trp Ala Pro Ala Phe Gln
                165                 170                 175

Val Gly Leu Arg Tyr Asp Leu Gly Asn Ser Trp Met Leu Asn Ser Asp
            180                 185                 190

Val Arg Tyr Ile Pro Phe Lys Thr Asp Val Thr Gly Thr Leu Gly Pro
        195                 200                 205

Val Pro Val Ser Thr Lys Ile Glu Val Asp Pro Phe Ile Leu Ser Leu
    210                 215                 220

Gly Ala Ser Tyr Val Phe
225                 230

<210>  2
<211>  693
<212>  DNA
<213>  Pseudomonas oleovorans

<400>  2
atgagttttt ctaattataa agtaatcgcg atgccggtgt tggttgctaa ttttgttttg        60

ggggcggcca ctgcatgggc gaatgaaaat tatccggcga aatctgctgg ctataatcag       120

ggtgactggg tcgctagctt caatttttct aaggtctatg tgggtgagga gcttggcgat       180

ctaaatgttg gagggggggc tttgccaaat gctgatgtaa gtattggtaa tgatacaaca       240

cttacgtttg atatcgccta ttttgttagc tcaaatatag cggtggattt ttttgttggg       300

gtgccagcta gggctaaatt tcaaggtgag aaatcaatct cctcgctggg aagagtcagt       360

gaagttgatt acggccctgc aattctttcg cttcaatatc attacgatag ctttgagcga       420

ctttatccat atgttggggt tggtgttggt cgggtgctat tttttgataa aaccgacggt       480

gctttgagtt cgtttgatat taaggataaa tgggcgcctg cttttcaggt tggccttaga       540

tatgaccttg gtaactcatg gatgctaaat tcagatgtgc gttatattcc tttcaaaacg       600

gacgtcacag gtactcttgg cccggttcct gtttctacta aaattgaggt tgatcctttc       660

attctcagtc ttggtgcgtc atatgttttc taa                                    693

<210>  3
<211>  230
<212>  PRT
<213>  Pseudomonas putida

<400>  3

Met Asn Pro Pro Ile Leu Lys Lys Leu Ala Met Ser Ile Leu Ala Thr
1               5                   10                  15

Ser Phe Val Leu Gly Gly Ala Ser Ala Trp Ser Gly Glu Ile Tyr Ser
            20                  25                  30

Thr Glu Thr Ala Gly Tyr Asn Gln Gly Asp Trp Val Ala Ser Phe Asn
        35                  40                  45

Met Ser Lys Val Tyr Val Asp Glu Thr Leu Gly Ser Leu Asn Val Gly

50                  55                  60

Gly Ala Thr Val Pro Asn Ala Ala Val Ser Ile Gly Asn Asp Thr Thr
65                  70                  75                  80

Val Ser Phe Asp Ile Ser Tyr Phe Ile Ser Asn Asn Val Ala Leu Asp
                85                  90                  95

Phe Phe Val Gly Ile Pro Ala Lys Ala Lys Phe Gln Gly Glu Lys Ser
            100                 105                 110

Ile Ser Ala Leu Gly Arg Val Ser Glu Val Asp Tyr Gly Pro Ala Ile
        115                 120                 125

Leu Ser Leu Gln Tyr His Phe Asp Asn Phe Glu Arg Leu Tyr Pro Tyr
    130                 135                 140

Val Gly Leu Gly Val Gly Arg Val Phe Phe Phe Asp Lys Thr Asp Gly
145                 150                 155                 160

Ala Leu Thr Ser Phe Asp Ile Lys Asp Lys Trp Ala Pro Ala Val Gln
                165                 170                 175

Val Gly Leu Arg Tyr Asp Phe Gly Asn Ser Trp Met Leu Asn Ser Asp
            180                 185                 190

Val Arg Tyr Ile Pro Phe Lys Thr Asp Val Ser Gly Thr Leu Gly Ala
        195                 200                 205

Ala Pro Val Ser Thr Lys Ile Glu Ile Asp Pro Phe Ile Leu Ser Leu
    210                 215                 220

Gly Ala Ser Tyr Lys Phe
225                 230

<210>  4
<211>  693
<212>  DNA
<213>  Pseudomonas putida

<400>  4
atgaatccgc ctattttaaa aaaactcgct atgtcgatat tagcaactag ttttgtgttg      60

ggtggggcca gtgcgtggtc aggtgaaatc tattcgactg aaactgctgg ctacaatcag     120

ggcgactggg ttgctagctt taatatgtct aaagtttatg tagacgagac gctaggctcc     180

ctaaatgtag gtggggctac tgtacccaat gctgctgtaa gcatcggtaa tgatacaaca     240

gtttcttttg atatttccta ttttattagt aacaatgtag ctttggattt tttcgtcggg     300

attccagcta aagctaagtt tcaaggtgaa aaatccatct ctgcgctggg aagagtcagt     360

gaagttgatt atggccctgc aattttgtca cttcagtatc attttgataa ttttgagcga 420

ctttatccat atgtcggact aggtgtcggt cgagtgtttt tcttcgacaa aactgatggt 480

gccttgactt catttgatat caaagataaa tgggcgcctg ctgttcaggt cggccttaga 540

tatgattttg gtaactcatg gatgttaaat tcagatgtgc gctatattcc tttcaaaaca 600

gatgtttctg gtacacttgg ggctgcacct gtttctacca agattgagat tgatcctttc 660

attctgagtc ttggagcatc atataagttc tga 693

<210> 5
<211> 246
<212> PRT
<213> Marinobacter aquaeolei VT8

<400> 5

Met Cys Tyr Glu Lys Leu Gln Phe Tyr Leu Lys Pro Ile Cys Phe Asn
1               5                   10                  15

Thr Lys Lys Asp Asn Lys Lys Met Lys Pro Lys Ile Ile Ser Lys Val
            20                  25                  30

Ser Leu Val Ala Phe Leu Leu Leu Ser Leu Ala Ala Ser Leu Ala Asn
        35                  40                  45

Ala Gln Ser Glu Pro Val Tyr Ser Arg Gly Asp Trp Val Val Gly Leu
    50                  55                  60

Asn Ala Thr Arg Val Leu Thr Asp Glu Asp Leu Arg Ser Ala Ser Ala
65                  70                  75                  80

Gly Gly Ala Pro Val Pro Asn Ser Asn Leu Ser Ile Asn Asn Asp Thr
                85                  90                  95

Thr Val Ser Phe Asp Val Ser Tyr Phe Leu Ser Asn Gln Leu Ala Phe
            100                 105                 110

Asn Ile Phe Gly Gly Ile Pro Ala Ser Ala Asp Leu Gln Gly Glu Glu
        115                 120                 125

Ser Leu Ser Gly Leu Phe Leu Gly Gln Thr Asp Tyr Gly Pro Val Ile
    130                 135                 140

Leu Ser Leu Gln Tyr His Val Leu Thr Gly Ser Asn Phe Ser Pro Tyr
145                 150                 155                 160

Phe Gly Ala Gly Val Gly Arg Ile Leu Phe Leu Asp Glu Lys Asp Arg
                165                 170                 175

Ala Leu Thr Asp Phe Asp Val Glu Asp Thr Trp Ala Pro Ala Ile Gln

180                 185                 190

Ala Gly Phe Arg Trp Arg Ile His Asn Asn Trp Ser Ala Asn Phe Asp
        195                 200                 205

Val Arg Tyr Ala Pro Phe Lys Ala Asp Ile Thr Gly Asn Leu Gly Pro
    210                 215                 220

Ala Pro Val Gln Ala Glu Val Glu Val Asp Pro Thr Ile Val Ser Ile
225                 230                 235                 240

Gly Val Ala Tyr Arg Phe
                245

<210>  6
<211>  741
<212>  DNA
<213>  Marinobacter aquaeolei

<400>  6
ttgtgttacg aaaaattgca gttttaccta aagccaattt gttttaatac caaaaaggat      60

aataaaaaaa tgaaacctaa aataattagt aaagtctcgt tagtggcgtt ccttttactt     120

tcacttgctg cgagcctggc caacgctcaa tctgagccgg tttacagtag aggcgactgg     180

gtggttggac tgaatgccac tagagtttta accgatgaag atttgcgatc agcctctgcg     240

gggggtgccc ccgttccaaa ttccaacctg tctattaaca acgatacgac cgtttcattc     300

gacgtgtcgt attttctgag taatcagctg gcatttaaca tttttggcgg cattcccgct     360

agtgcggacc tccagggcga agagtctctc tccggtcttt ttcttggtca aacagattat     420

ggtccggtaa ttctttcgct tcagtatcat gtcttaacgg gtagcaactt ctctccgtac     480

tttggagcgg gtgtaggacg gattctcttt ttagatgaga aggatcgcgc actaaccgac     540

ttcgacgtcg aagatacatg ggcccctgcg attcaggctg gttttcgctg gaggatacac     600

aataactggt cggcaaattt tgacgttaga tatgcaccct tcaaagcgga tatcaccggt     660

aacctaggcc cggcccctgt tcaggcagaa gtggaagtgg accccactat cgtgagcatc     720

ggtgtcgcat atcgctttta a                                               741

<210>  7
<211>  228
<212>  PRT
<213>  Oceanicaulis alexandrii HTCC2633

<400>  7

Met Lys Gln Ser Asn Val Lys Ser Lys Gly Pro Phe Ala Ser Lys Phe
1               5                   10                  15

Val Leu Ile Thr Met Ile Gly Gly Phe Ser Ser Phe Ser Val Ala Asn
            20                  25                  30

Ala Glu Pro Leu Tyr Ser Lys Gly Asp Trp Leu Phe Gly Leu Asn Ala
        35                  40                  45

Ala Lys Val Phe Thr Asn Glu Thr Leu Asp Ser Ile Ser Ala Gly Gly
    50                  55                  60

Ala Pro Ile Pro Gly Ala Gly Val Asn Ile Thr Asp Asp Thr Thr Leu
65                  70                  75                  80

Ser Phe Asp Val Ser Tyr Phe Leu Asn Ser Ser Val Ala Leu Asn Phe
                85                  90                  95

Phe Gly Gly Leu Pro Ala Ser Ala Asn Leu Ala Gly Ser Gly Ser Leu
            100                 105                 110

Ala Gly Leu Pro Val Gly Glu Thr Glu Tyr Gly Pro Ala Val Leu Ser
        115                 120                 125

Leu Gln Tyr His Phe Ser Thr Asn Ser Ser Val Ser Pro Tyr Val Gly
    130                 135                 140

Ala Gly Ile Ala Arg Ile Leu Phe Leu Glu Glu Gln Gly Asp Ala Leu
145                 150                 155                 160

Ala Asp Phe Asp Leu Lys Asp Ala Trp Ala Pro Ala Val Gln Val Gly
                165                 170                 175

Met Arg Tyr Gln Met Ser Asp Asn Trp Phe Ala Asn Ala Asp Ile Arg
            180                 185                 190

Tyr Thr Pro Phe Glu Thr Asp Ile Ser Gly Thr Leu Gly Gly Ala Pro
        195                 200                 205

Val Arg Gly Lys Ile Ser Val Asp Pro Thr Ile Leu Asn Ile Gly Ile
    210                 215                 220

Ala Tyr Arg Phe
225

<210>  8
<211>  741
<212>  DNA
<213>  Oceanicaulis alexandrii HTCC2633

<400>  8
ttaaaagcga tatgcgacac cgatgctcac gatagtgggg tccacttcca cttctgcctg      60

aacaggggcc gggcctaggt taccggtgat atccgctttg aagggtgcat atctaacgtc     120

aaaatttgcc gaccagttat tgtgtatcct ccagcgaaaa ccagcctgaa tcgcaggggc     180

ccatgtatct tcgacgtcga agtcggttag tgcgcgatcc ttctcatcta aaaagagaat 240

ccgtcctaca cccgctccaa agtacggaga gaagttgcta cccgttaaga catgatactg 300

aagcgaaaga attaccggac cataatctgt ttgaccaaga aaaagaccgg agagagactc 360

ttcgccctgg aggtccgcac tagcgggaat gccgccaaaa atgttaaatg ccagctgatt 420

actcagaaaa tacgacacgt cgaatgaaac ggtcgtatcg ttgttaatag acaggttgga 480

atttggaacg gggcaccccc ccgcagaggc tgatcgcaaa tcttcatcgg ttaaaactct 540

agtggcattc agtccaacca cccagtcgcc tctactgtaa accggctcag attgagcgtt 600

ggccaggctc gcagcaagtg aaagtaaaag gaacgccact aacgagactt tactaattat 660

tttaggtttc atttttttat tatccttttt ggtattaaaa caaattggct ttaggtaaaa 720

ctgcaatttt tcgtaacaca a 741

<210> 9
<211> 223
<212> PRT
<213> Marinobacter manganoxydans MnI7-9

<400> 9

Met Lys Pro Lys Ile Ile Ser Lys Val Ser Leu Val Ala Phe Leu Leu
1               5                   10                  15

Leu Ser Leu Ala Ala Ser Leu Ala Asn Ala Gln Ser Glu Pro Val Tyr
            20                  25                  30

Ser Arg Gly Asp Trp Val Val Gly Leu Asn Ala Thr Arg Val Leu Thr
        35                  40                  45

Asp Glu Asp Leu Arg Ser Ala Ser Ala Gly Ser Ala Pro Val Pro Asn
    50                  55                  60

Ser Asn Leu Ser Ile Asn Asn Asp Thr Thr Val Ser Phe Asp Val Ser
65                  70                  75                  80

Tyr Phe Leu Ser Asn Gln Leu Ala Phe Asn Ile Phe Gly Gly Ile Pro
                85                  90                  95

Ala Ser Ala Asp Leu Gln Gly Glu Glu Ser Leu Ser Gly Leu Phe Leu
            100                 105                 110

Gly Gln Thr Asp Tyr Gly Pro Val Ile Leu Ser Leu Gln Tyr His Val
        115                 120                 125

Leu Thr Gly Ser Asn Phe Ser Pro Tyr Phe Gly Ala Gly Val Gly Arg
    130                 135                 140

Ile Leu Phe Leu Asp Glu Lys Asp Arg Ala Leu Thr Asp Phe Asp Val
145                 150                 155                 160

Glu Asp Thr Trp Ala Pro Ala Val Gln Ala Gly Phe Arg Trp Arg Ile
                165                 170                 175

His Asn Asn Trp Ser Ala Asn Phe Asp Val Arg Tyr Ala Pro Phe Glu
            180                 185                 190

Ala Asp Ile Thr Gly Asn Leu Gly Pro Ala Pro Val Gln Ala Lys Val
        195                 200                 205

Glu Val Asp Pro Thr Ile Val Ser Ile Gly Val Ala Tyr Arg Phe
    210                 215                 220

<210>  10
<211>  687
<212>  DNA
<213>  Marinobacter manganoxydans MnI7-9

<400>  10
atgaaacaat ctaatgtaaa atccaagggg ccttttgcct cgaagtttgt gttaatcacg      60

atgatcggtg gcttttcgtc gttcagcgtc gcaaatgcgg aaccgcttta ctccaagggc     120

gactggcttt ttggcctgaa tgcggcgaaa gtattcacga acgaaacgtt ggattcaatc     180

agcgcgggcg gtgcgccaat acccggggct ggtgtcaata tcaccgatga caccacgctg     240

agtttcgacg tttcctattt tttgaattca tctgtagcgt tgaacttctt tggtggtttg     300

cctgccagcg ctaatcttgc agggagcggc agtttggcag gactgcctgt tggagagacg     360

gaatatggcc ctgctgtttt gtcgcttcaa tatcactttt cgactaattc atccgttagc     420

ccttatgttg gtgccggtat cgctcgaatc ttgtttctgg aagaacaagg ggatgcactc     480

gcggacttcg acttgaaaga cgcctgggcg cccgctgtcc aagttggaat gcgctatcaa     540

atgagcgata attggtttgc caatgccgat atacgttaca cgccattcga aacagatatc     600

tctgggacac tcggcggggc gccagtcaga ggcaagattt cggtggaccc aacaattctc     660

aatatcggta ttgcctaccg gttttaa                                         687

<210>  11
<211>  227
<212>  PRT
<213>  Caulobacter sp. K31

<400>  11

Met Arg Lys Asn Gly Ala Tyr Met Tyr Ala Arg Leu Ala Gln Tyr Ala
1               5                   10                  15

Thr Ala Leu Ala Val Leu Ser Val Phe Pro Gln Ala Ala Leu Ala Gln
            20                  25                  30

Asn Ser Glu Gly Phe Lys Leu Trp Ala Val Ser Leu Asn Ala Thr Arg
        35                  40                  45

Val Phe Val Asp Glu Asp Ala Pro Asp Ile Thr Leu Ala Gly Gly Pro
    50                  55                  60

Val Pro Gly Ser Asn Val Arg Ile Gly Asp Ala Thr Ser Ala Thr Ile
65                  70                  75                  80

Asp Ile Gly Tyr Phe Phe Thr Pro Asn Val Ala Gly Asn Leu Phe Leu
                85                  90                  95

Gly Val Pro Ala Thr Ala Gln Ile Asp Gly Ala Gly Ser Leu Glu Pro
            100                 105                 110

Leu Gly Thr Leu Ala Lys Val Asn Tyr Gly Pro Ile Ile Leu Ser Ala
        115                 120                 125

Gln Tyr His Phe Asn Asn Leu Gly Lys Val His Pro Tyr Leu Gly Val
    130                 135                 140

Gly Val Gly Arg Ile Val Phe Leu Asn Glu Arg Asp Arg Ala Leu Leu
145                 150                 155                 160

Asn Phe Ser Ile Asp Asp Ser Trp Ala Pro Ala Ala Gln Val Gly Val
                165                 170                 175

Arg Tyr Glu Leu Gly Ala Glu Trp Met Leu Asn Ala Asp Val Arg Tyr
            180                 185                 190

Val Pro Phe Ser Thr His Ala Thr Gly Ser Leu Gly Gly Ala Pro Val
        195                 200                 205

Arg Thr Arg Leu Asp Ile Asp Pro Ile Leu Thr Ser Ala Gly Val Thr
    210                 215                 220

Tyr Arg Phe
225

<210>  12
<211>  684
<212>  DNA
<213>  Caulobacter sp. K31

<400>  12
atgagaaaaa atggagcata tatgtatgca aggcttgcgc aatacgccac cgcgctcgcc      60

gtcttgagcg tcttccccca ggctgcattg gcccagaatt cggaaggatt caagctgtgg     120

gccgtaagtc tgaatgcaac tagggtcttc gtggatgagg atgcgcccga catcaccttg     180

gctggagggc ctgttccggg ctcgaatgtg aggatcgggg atgccacgtc ggcgaccatt 240

gatatcggat atttcttcac gcccaatgtt gctggtaatt tgtttctcgg cgtgccggcg 300

accgcgcaaa ttgacggcgc tgggtcactt gagccgctcg gaactctagc caaggtcaac 360

tatggaccca tcatcttgtc ggcccagtac catttcaaca atcttggcaa ggttcatccc 420

tatctgggag tgggcgtcgg gcggatcgtc tttctgaatg agcgtgacag agctttgctt 480

aatttcagta tcgacgacag ttgggcgcct gcggctcagg tgggtgttcg gtacgagctt 540

ggcgcagaat ggatgctgaa cgctgacgtt cgatacgttc ccttctccac gcacgctacc 600

ggttcactgg gtggagcgcc tgtccggaca cgtttggaca tcgacccgat cctgacgagc 660

gccggagtga cttaccggtt ttag 684

<210> 13
<211> 410
<212> PRT
<213> Alcanivorax borkumensis SK2

<400> 13

Met Glu Asn Glu Lys Gln Asp Ala Thr Val Ile Val Gly Gly Gly His
1               5                   10                  15

Ala Ala Gly Ala Leu Met Thr Ala Leu Ile Gln Lys Lys Tyr Pro His
            20                  25                  30

Glu Val Val Leu Val Gly Glu Glu Pro Tyr Pro Pro Tyr Gln Arg Pro
        35                  40                  45

Pro Leu Ser Lys Thr Tyr Leu Ser Gly Glu Val Asn Glu Glu Ser Leu
    50                  55                  60

Tyr Leu Lys Pro Arg Ser Val Tyr Glu Gly Ala Gly His Gln Leu Arg
65                  70                  75                  80

Leu Gly Val Arg Val Glu Asn Ile Asp Arg Asp Asn Lys Thr Leu Thr
                85                  90                  95

Leu Ser Asp Gln Ser Thr Leu Lys Tyr Gly Arg Leu Ile Leu Ala Thr
            100                 105                 110

Gly Ser His Val Arg Arg Leu Asn Ala Pro Gly Ser Glu Leu Lys Gly
        115                 120                 125

Ile His Tyr Leu His Asp Ile Ala Asp Thr Asp Thr Leu Arg Asp Gln
    130                 135                 140

Leu Ser Pro Gly Ala Arg Leu Val Ile Val Gly Gly Gly Tyr Ile Gly
145                 150                 155                 160

Leu Glu Val Ala Ala Ser Ala Ser Lys Lys Gly Val Asn Val Thr Val
                165                 170                 175

Leu Glu Gly Ala Glu Arg Leu Met Gln Arg Val Thr Gly Val Glu Met
            180                 185                 190

Ser Ser Phe Leu Tyr Ala Lys His Ser Gly Ser Gly Val Asp Val Arg
        195                 200                 205

Leu Asn Thr Ala Val Thr Gly Phe Lys Ala Gly Asp Gln Gly Arg Val
    210                 215                 220

Ala Gly Val Thr Leu Ala Asn Gly Glu Thr Val Asp Ala Asp Val Val
225                 230                 235                 240

Leu Val Ser Ile Gly Val Ile Pro Glu Thr Ala Leu Ala Glu Ala Ala
                245                 250                 255

Gly Leu Ser Cys Glu Asp Gly Ile Leu Val Asp Glu Tyr Val Arg Thr
            260                 265                 270

Ser Asp Pro Ser Ile Leu Ala Ile Gly Asp Cys Thr Arg His Arg Asn
        275                 280                 285

Leu Phe Phe Glu Lys Met Gln Arg Leu Glu Ser Val Ala Asn Ala Val
    290                 295                 300

Asp Gln Ala Arg Thr Ala Ala Ala Thr Leu Met Gly Glu Asp Lys Pro
305                 310                 315                 320

Tyr Asp Ser Ala Pro Trp Phe Trp Ser Asn Gln Tyr Asp Val Arg Leu
                325                 330                 335

Gln Met Val Gly Leu Ser Gln Asp His Asp Glu Arg Val Met Arg Gly
            340                 345                 350

Ser Thr Glu Asp Lys Ala Phe Ala Val Phe Tyr Leu Arg Glu Gly Cys
        355                 360                 365

Val Ile Ala Val Asp Ala Val Asn Met Pro Ile Ala Phe Met Val Gly
    370                 375                 380

Lys Gln Leu Val Gln His Arg Lys Ser Ile Ser Ala Asp Val Leu Ser
385                 390                 395                 400

Asp Leu Asp Val Glu Leu Lys Ser Leu Ile
                405                 410

<210>  14
<211>  1233
<212>  DNA
<213>  Alcanivorax borkumensis SK2

<400>  14
atggaaaacg aaaaacaaga tgccactgtc atcgttggag gcgggcacgc agcaggtgcg        60

ttgatgacag ccttgataca aaagaaatat ccacacgagg tggttctggt gggcgaagag       120

ccttatccgc cctaccagcg cccgccttta tccaaaacgt atctgtcagg agaggttaac       180

gaggaatctc tctatttgaa accgcgctcg gtgtatgaag gtgcggggca tcagttgcga       240

cttggtgtgc gcgttgagaa cattgatcga gacaacaaaa cccttacatt gtcagatcag       300

agcacactga aatatggccg actgattctt gccacaggtt cacacgttag gcgtcttaat       360

gcgcctggat ctgaattaaa aggcatccat tatctgcatg acattgctga tacggataca       420

ttgcgcgatc aactgtcacc aggtgcccgt ttggttattg tcggtggcgg ctacattggc       480

cttgaggttg cagccagtgc gagcaagaaa ggcgttaatg ttacggtgct ggaaggcgct       540

gagcgtctaa tgcagcgagt tacgggcgtt gagatgtctt cgttcctgta tgctaagcac       600

agtggttctg gcgtggacgt gcgtcttaat actgctgtca ccggcttcaa agctggagat       660

caggggcgag tggctggcgt aacgttagca aatggcgaaa cggttgacgc agatgttgtg       720

cttgtctcga ttggcgttat acccgaaacg gctttggctg aggctgccgg cctatcctgt       780

gaagacggta tcctggtgga cgaatatgtc cgcacttctg acccaagcat cctggcgata       840

ggtgattgca ctcgtcaccg aaaccttttc ttcgagaaaa tgcagaggct cgagtccgtt       900

gctaacgctg tcgatcaagc acgtactgcg gcagcgacct tgatgggaga ggataagccc       960

tacgatagcg ctccatggtt ttggtcgaat caatatgatg ttcgtttgca aatggtgggg      1020

ctctcgcagg accatgatga acgagtcatg cgtggcagca cggaagacaa agcgtttgcg      1080

gtgttctatc tccgtgaggg ctgtgtgatt gccgttgatg cggtgaatat gcccattgcg      1140

tttatggttg gaaagcagtt ggttcagcac cgtaagagta ttagcgctga cgtgttgagt      1200

gatctggatg ttgaattaaa gtctttgatc tga                                   1233

<210>  15
<211>  106
<212>  PRT
<213>  Alcanivorax borkumensis SK2

<400>  15

Met Gly Lys Ile Thr Phe Ile Glu Asn Asp Lys Thr Glu His Val Thr
1               5                   10                  15

Glu Phe Glu Ala Gly Ile Thr Leu Met Gln Val Ala Leu Asp Asn Ala
            20                  25                  30

Val Pro Gly Ile Asp Gly Asp Cys Gly Gly Glu Cys Ala Cys Gly Thr

35                  40                  45

Cys His Leu Ile Val Pro Glu Glu Trp Phe Asp Lys Thr Gly Pro Ile
    50                  55                  60

Asn Asp Ala Glu Glu Gln Met Leu Ser Met Thr Pro Glu Arg Ala Lys
65                  70                  75                  80

Thr Ser Arg Leu Gly Cys Gln Val Lys Ala Thr Glu Ala Met Asp Gly
                85                  90                  95

Met Thr Val Gln Leu Pro Glu Phe Gln Met
            100                 105

<210>  16
<211>  321
<212>  DNA
<213>  Alcanivorax borkumensis SK2

<400>  16
gtgggaaaaa tcacctttat tgagaatgat aaaactgaac atgtaacaga atttgaggca      60

ggtattactt tgatgcaagt tgccttagac aacgccgttc ccggtattga tggggattgc     120

ggcggggagt gtgcctgtgg tacctgtcac ctgattgttc cagaagaatg gttcgataaa     180

accgggccga ttaatgatgc tgaagaacaa atgttgtcca tgacacctga gcgtgcaaaa     240

acctctcggt tggggtgtca ggttaaggcc actgaggcaa tggacggaat gactgttcaa     300

ttgccagaat ttcaaatgta a                                               321

<210>  17
<211>  336
<212>  PRT
<213>  Escherichia coli

<400>  17

Met Lys Ala Ala Val Val Thr Lys Asp His His Val Asp Val Thr Tyr
1               5                   10                  15

Lys Thr Leu Arg Ser Leu Lys His Gly Glu Ala Leu Leu Lys Met Glu
            20                  25                  30

Cys Cys Gly Val Cys His Thr Asp Leu His Val Lys Asn Gly Asp Phe
        35                  40                  45

Gly Asp Lys Thr Gly Val Ile Leu Gly His Glu Gly Ile Gly Val Val
    50                  55                  60

Ala Glu Val Gly Pro Gly Val Thr Ser Leu Lys Pro Gly Asp Arg Ala
65                  70                  75                  80

Ser Val Ala Trp Phe Tyr Glu Gly Cys Gly His Cys Glu Tyr Cys Asn
                85                  90                  95

Ser Gly Asn Glu Thr Leu Cys Arg Ser Val Lys Asn Ala Gly Tyr Ser
            100                 105                 110

Val Asp Gly Gly Met Ala Glu Glu Cys Ile Val Val Ala Asp Tyr Ala
        115                 120                 125

Val Lys Val Pro Asp Gly Leu Asp Ser Ala Ala Ala Ser Ser Ile Thr
    130                 135                 140

Cys Ala Gly Val Thr Thr Tyr Lys Ala Val Lys Leu Ser Lys Ile Arg
145                 150                 155                 160

Pro Gly Gln Trp Ile Ala Ile Tyr Gly Leu Gly Gly Leu Gly Asn Leu
                165                 170                 175

Ala Leu Gln Tyr Ala Lys Asn Val Phe Asn Ala Lys Val Ile Ala Ile
            180                 185                 190

Asp Val Asn Asp Glu Gln Leu Lys Leu Ala Thr Glu Met Gly Ala Asp
        195                 200                 205

Leu Ala Ile Asn Ser His Thr Glu Asp Ala Ala Lys Ile Val Gln Glu
    210                 215                 220

Lys Thr Gly Gly Ala His Ala Ala Val Val Thr Ala Val Ala Lys Ala
225                 230                 235                 240

Ala Phe Asn Ser Ala Val Asp Ala Val Arg Ala Gly Gly Arg Val Val
                245                 250                 255

Ala Val Gly Leu Pro Pro Glu Ser Met Ser Leu Asp Ile Pro Arg Leu
            260                 265                 270

Val Leu Asp Gly Ile Glu Val Val Gly Ser Leu Val Gly Thr Arg Gln
        275                 280                 285

Asp Leu Thr Glu Ala Phe Gln Phe Ala Ala Glu Gly Lys Val Val Pro
    290                 295                 300

Lys Val Ala Leu Arg Pro Leu Ala Asp Ile Asn Thr Ile Phe Thr Glu
305                 310                 315                 320

Met Glu Glu Gly Lys Ile Arg Gly Arg Met Val Ile Asp Phe Arg His
                325                 330                 335

<210>  18

<211>  1011
<212>  DNA
<213>  Escherichia coli

<400>  18
atgaaggctg cagttgttac gaaggatcat catgttgacg ttacgtataa aacactgcgc      60

tcactgaaac atggcgaagc cctgctgaaa atggagtgtt gtggtgtatg tcataccgat     120

cttcatgtta agaatggcga ttttggtgac aaaaccggcg taattctggg ccatgaaggc     180

atcggtgtgg tggcagaagt gggtccaggt gtcacctcat taaaaccagg cgatcgtgcc     240

agcgtggcgt ggttctacga aggatgcggt cattgcgaat actgtaacag tggtaacgaa     300

acgctctgcc gttcagttaa aaatgccgga tacagcgttg atggcgggat ggcggaagag     360

tgcatcgtgg tcgccgatta cgcggtaaaa gtgccagatg gtctggactc ggcggcggcc     420

agcagcatta cctgtgcggg agtcaccacc tacaaagccg ttaagctgtc aaaaattcgt     480

ccagggcagt ggattgctat ctacggtctt ggcggtctgg gtaacctcgc cctgcaatac     540

gcgaagaatg tctttaacgc caaagtgatc gccattgatg tcaatgatga gcagttaaaa     600

ctggcaaccg aaatgggcgc agatttagcg attaactcac acaccgaaga cgccgccaaa     660

attgtgcagg agaaaactgg tggcgctcac gctgcggtgg taacagcggt agctaaagct     720

gcgtttaact cggcagttga tgctgtccgt gcaggcggtc gtgttgtggc tgtcggtcta     780

ccgccggagt ctatgagcct ggatatccca cgtcttgtgc tggatggtat tgaagtggtc     840

ggttcgctgg tcggcacgcg ccaggattta actgaagcct tccagtttgc cgccgaaggt     900

aaagtggtgc cgaaagtcgc cctgcgtccg ttagcggaca tcaacaccat ctttactgag     960

atggaagaag gcaaaatccg tggccgcatg gtgattgatt tccgtcacta a             1011


<210>  19
<211>  470
<212>  PRT
<213>  Alcanivorax borkumensis SK2

<400>  19

Met Ser Thr Ser Ser Ser Thr Ser Asn Asp Ile Gln Ala Lys Ile Ile
1               5                   10                  15


Asn Ala Thr Ser Lys Val Val Pro Met His Leu Gln Ile Lys Ala Leu
            20                  25                  30


Lys Asn Leu Met Lys Val Lys Arg Lys Thr Ile Gly Thr Ser Arg Pro
        35                  40                  45


Gln Val His Phe Val Glu Thr Asp Leu Pro Asp Val Asn Asp Leu Ala
    50                  55                  60


Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Arg Ala Val Gln
145                 150                 155                 160

Gly Val Val Ala Pro Lys Asn Leu Lys Glu Met Glu Gly Leu Ile Arg
                165                 170                 175

Lys Arg Thr Gly Asp Val Leu Asp Ser Leu Pro Leu Asp Thr Pro Phe
            180                 185                 190

Asn Trp Val Pro Val Val Ser Lys Glu Leu Thr Gly Arg Met Leu Ala
        195                 200                 205

Ser Leu Leu Asp Phe Pro Tyr Asp Glu Arg Glu Lys Leu Val Gly Trp
    210                 215                 220

Ser Asp Arg Leu Ser Gly Ala Ser Ser Ala Thr Gly Gly Glu Phe Thr
225                 230                 235                 240

Asn Glu Asp Val Phe Phe Asp Asp Ala Ala Asp Met Ala Trp Ala Phe
                245                 250                 255

Ser Lys Leu Trp Arg Asp Lys Glu Ala Arg Gln Lys Ala Gly Glu Glu
            260                 265                 270

Pro Gly Phe Asp Leu Ile Ser Met Leu Gln Ser Asn Glu Asp Thr Lys
        275                 280                 285

Asp Leu Ile Asn Arg Pro Leu Glu Phe Ile Gly Asn Leu Ala Leu Leu
    290                 295                 300

Ile Val Gly Gly Asn Asp Thr Thr Arg Asn Ser Met Ser Gly Gly Val
305                 310                 315                 320

Leu Ala Leu Asn Gln Phe Pro Glu Gln Phe Glu Lys Leu Lys Ala Asn
                325                 330                 335

Pro Lys Leu Ile Pro Asn Met Val Ser Glu Ile Ile Arg Trp Gln Thr
            340                 345                 350

Pro Leu Ala Tyr Met Arg Arg Val Ala Lys Gln Asp Val Glu Leu Asn
        355                 360                 365

Gly Gln Thr Ile Lys Lys Gly Asp Arg Val Leu Met Trp Tyr Ala Ser
    370                 375                 380

Gly Asn Gln Asp Glu Arg Lys Phe Glu Asn Pro Glu Gln Phe Ile Ile
385                 390                 395                 400

Asp Arg Lys Asp Thr Arg Asn His Val Ser Phe Gly Tyr Gly Val His
                405                 410                 415

Arg Cys Met Gly Asn Arg Leu Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470

<210> 20
<211> 1413
<212> DNA
<213> Alcanivorax borkumensis SK2

<400> 20
atgtcaacga gttcaagtac aagtaatgac atccaggcaa aaataattaa cgccacatcc 60

aaagtcgtgc caatgcatct acagatcaag gcactaaaaa acttgatgaa ggtgaagcgg 120

aagaccattg gcacttcccg ccctcaggtg cactttgttg aaaccgattt gcctgacgtc 180

aatgatttgg cgatagaaga tatcgatacg agtaaccctt ttttataccg acaaggtaag 240

gcgaatgcgt actttaagcg gttgcgtgat gaagcgccgg tgcactatca gaagaacagt 300

gctttcgggc cgttctggtc ggtaacacgc tacgaagata tcgtcttcgt ggacaagagc 360

catgatttgt tttccgccga accccaaatt atcttgggtg atcctccgga aggcctgtcg 420

gttgaaatgt tcatcgctat ggatcctccc aagcacgacg tacagcgtcg ggcagtccag 480

ggtgttgttg cgcccaagaa cctgaaagaa atggaaggac tgatccgcaa gcgcaccggg 540

gacgtactgg atagcctgcc gttggacact ccgttcaact gggtgccggt ggtgtcgaaa 600

gagctgaccg ggcgcatgct agcctcactg ttagatttcc cgtatgacga acgcgaaaaa 660

ctggttggct ggtcggatcg attgtccggc gcgtcctcgg caaccggcgg cgagtttacg 720

aatgaagatg tgttttttga tgacgcggca gatatggcgt gggctttctc caagctttgg 780

cgtgataaag aagcccgtca aaaagcaggt gaagagccgg gtttcgattt gatcagcatg 840

cttcagtcca atgaagacac aaaagatctg atcaatcgtc ctttggaatt cattggtaat 900

ctcgcgttgt tgattgttgg cggtaatgac accacgcgta actcaatgag cgggggggtg 960

ctggctttaa atcagttccc agagcaattc gagaagctaa aggcgaaccc aaagcttatc 1020

cccaatatgg tctctgaaat cattcgctgg caaacgccgc ttgcgtatat gcgccgggtt 1080

gccaagcagg atgtggagct gaacggacag accatcaaga agggtgatcg cgtgctgatg 1140

tggtatgcgt cgggcaacca ggatgagaga aaatttgaga atcctgagca attcatcatc 1200

gaccgcaaag atacgcgtaa ccatgtgtcg tttggttatg gggttcaccg ttgtatgggc 1260

aaccgccttg ccgaactgca gctgcgtatt ctgtgggaag agcttctccc tcgctttgaa 1320

aacatcgaag tgatcggtga gccggagcgc gtgcaatcga actttgtgcg gggctattcc 1380

aagatgatgg ttaagttgac ggctaaaaaa taa 1413

<210> 21
<211> 12
<212> PRT
<213> artificial

<220>
<223> sequence motif from CYP153

<220>
<221> alternatives
<222> (3)..(4)
<223> Ile3 may be leucin; val4 may be isoleucin

<400> 21

Leu Leu Ile Ile Gly Gly Asn Asp Thr Thr Arg Asn
1               5                   10

<210> 22
<211> 378
<212> PRT
<213> Bacillus subtilis subsp. subtilis str. 168

<400> 22

Met Ile Ile Gly Val Pro Lys Glu Ile Lys Asn Asn Glu Asn Arg Val
1               5                   10                  15

Ala Leu Thr Pro Gly Gly Val Ser Gln Leu Ile Ser Asn Gly His Arg
            20                  25                  30

Val Leu Val Glu Thr Gly Ala Gly Leu Gly Ser Gly Phe Glu Asn Glu
        35                  40                  45

Ala Tyr Glu Ser Ala Gly Ala Glu Ile Ile Ala Asp Pro Lys Gln Val
    50                  55                  60

Trp Asp Ala Glu Met Val Met Lys Val Lys Glu Pro Leu Pro Glu Glu
65                  70                  75                  80

Tyr Val Tyr Phe Arg Lys Gly Leu Val Leu Phe Thr Tyr Leu His Leu
                85                  90                  95

Ala Ala Glu Pro Glu Leu Ala Gln Ala Leu Lys Asp Lys Gly Val Thr
            100                 105                 110

Ala Ile Ala Tyr Glu Thr Val Ser Glu Gly Arg Thr Leu Pro Leu Leu
        115                 120                 125

Thr Pro Met Ser Glu Val Ala Gly Arg Met Ala Ala Gln Ile Gly Ala
    130                 135                 140

Gln Phe Leu Glu Lys Pro Lys Gly Gly Lys Gly Ile Leu Leu Ala Gly
145                 150                 155                 160

Val Pro Gly Val Ser Arg Gly Lys Val Thr Ile Ile Gly Gly Gly Val
                165                 170                 175

Val Gly Thr Asn Ala Ala Lys Met Ala Val Gly Leu Gly Ala Asp Val
            180                 185                 190

Thr Ile Ile Asp Leu Asn Ala Asp Arg Leu Arg Gln Leu Asp Asp Ile
        195                 200                 205

Phe Gly His Gln Ile Lys Thr Leu Ile Ser Asn Pro Val Asn Ile Ala
    210                 215                 220

Asp Ala Val Ala Glu Ala Asp Leu Leu Ile Cys Ala Val Leu Ile Pro
225                 230                 235                 240

Gly Ala Lys Ala Pro Thr Leu Val Thr Glu Glu Met Val Lys Gln Met
                245                 250                 255

Lys Pro Gly Ser Val Ile Val Asp Val Ala Ile Asp Gln Gly Gly Ile
            260                 265                 270

Val Glu Thr Val Asp His Ile Thr Thr His Asp Gln Pro Thr Tyr Glu
        275                 280                 285

Lys His Gly Val Val His Tyr Ala Val Ala Asn Met Pro Gly Ala Val
    290                 295                 300

Pro Arg Thr Ser Thr Ile Ala Leu Thr Asn Val Thr Val Pro Tyr Ala
305                 310                 315                 320

Leu Gln Ile Ala Asn Lys Gly Ala Val Lys Ala Leu Ala Asp Asn Thr
                325                 330                 335

Ala Leu Arg Ala Gly Leu Asn Thr Ala Asn Gly His Val Thr Tyr Glu
            340                 345                 350

Ala Val Ala Arg Asp Leu Gly Tyr Glu Tyr Val Pro Ala Glu Lys Ala
        355                 360                 365

Leu Gln Asp Glu Ser Ser Val Ala Gly Ala
    370                 375

<210>  23
<211>  1137
<212>  DNA
<213>  Bacillus subtilis subsp. subtilis str. 168

<400>  23
atgatcatcg gcgtccctaa agaaatcaaa aacaacgaaa accgcgtggc actgaccccg      60

ggtggtgtgt cccaattgat cagcaacggc caccgtgtct tggtcgaaac cggcgctggt     120

ctgggtagcg gctttgagaa cgaggcatac gagtcggcag gtgcggagat tattgccgat     180

cctaagcagg tgtgggacgc cgagatggtt atgaaagtga aagaaccgct gccggaagaa     240

tacgtttact ttcgcaaagg tctggttctg ttcacctatc tgcacttggc cgctgagccg     300

gagctggcac aagcgctgaa ggataagggc gttacggcga tcgcgtatga aacggtgtct     360

gagggccgta ccctgccgct gctgaccccg atgagcgagg ttgccggtcg tatggcagcc     420

cagatcggtg cgcagttcct ggagaaaccg aaaggtggca agggcattct gctggcgggt     480

gtcccgggtg tttctcgtgg taaggtcact atcattggcg gtggcgtggt cggtaccaac     540

gcggcgaaga tggcggttgg cctgggtgct gacgttacga ttatcgactt gaacgctgat     600

cgcctgcgtc aattggacga catctttggc caccagatca agaccttgat ctccaatccg     660

gtgaatatcg cggacgcggt ggcggaggcg gatctgctga tttgcgcagt tctgattcct     720

ggcgcgaagg cgccgaccct ggtcacggaa gaaatggtga aacaaatgaa accgggtagc     780

gtgattgttg acgtagcgat tgatcagggt ggtatcgtgg aaactgttga ccacatcacg     840

actcatgatc agccgacgta cgagaaacat ggtgttgttc actatgcagt tgcaaatatg     900

ccgggtgcgg tcccgcgtac tagcacgatt gccctgacca atgtgaccgt tccgtatgca     960

ctgcaaattg caaataaggg tgcggtgaag gctttggcgg acaacaccgc gctgcgtgct    1020

ggtctgaata ccgcgaacgg tcatgtgacc tatgaggcgg tcgcacgtga cctgggttat    1080

gagtacgtgc ctgcagagaa ggcactgcag gacgagagct ccgtggcagg tgcgtaa       1137

<210>  24
<211>  459
<212>  PRT
<213>  Chromobacterium violaceum ATCC 12472

<400>  24

Met Gln Lys Gln Arg Thr Thr Ser Gln Trp Arg Glu Leu Asp Ala Ala
1               5                   10                  15

His His Leu His Pro Phe Thr Asp Thr Ala Ser Leu Asn Gln Ala Gly
            20                  25                  30

Ala Arg Val Met Thr Arg Gly Glu Gly Val Tyr Leu Trp Asp Ser Glu
        35                  40                  45

Gly Asn Lys Ile Ile Asp Gly Met Ala Gly Leu Trp Cys Val Asn Val
    50                  55                  60

Gly Tyr Gly Arg Lys Asp Phe Ala Glu Ala Ala Arg Arg Gln Met Glu
65                  70                  75                  80

Glu Leu Pro Phe Tyr Asn Thr Phe Phe Lys Thr Thr His Pro Ala Val
                85                  90                  95

Val Glu Leu Ser Ser Leu Leu Ala Glu Val Thr Pro Ala Gly Phe Asp
            100                 105                 110

Arg Val Phe Tyr Thr Asn Ser Gly Ser Glu Ser Val Asp Thr Met Ile
        115                 120                 125

Arg Met Val Arg Arg Tyr Trp Asp Val Gln Gly Lys Pro Glu Lys Lys
    130                 135                 140

Thr Leu Ile Gly Arg Trp Asn Gly Tyr His Gly Ser Thr Ile Gly Gly
145                 150                 155                 160

Ala Ser Leu Gly Gly Met Lys Tyr Met His Glu Gln Gly Asp Leu Pro
                165                 170                 175

Ile Pro Gly Met Ala His Ile Glu Gln Pro Trp Trp Tyr Lys His Gly
            180                 185                 190

Lys Asp Met Thr Pro Asp Glu Phe Gly Val Val Ala Ala Arg Trp Leu
        195                 200                 205

Glu Glu Lys Ile Leu Glu Ile Gly Ala Asp Lys Val Ala Ala Phe Val
    210                 215                 220

Gly Glu Pro Ile Gln Gly Ala Gly Gly Val Ile Val Pro Pro Ala Thr
225                 230                 235                 240

Tyr Trp Pro Glu Ile Glu Arg Ile Cys Arg Lys Tyr Asp Val Leu Leu
                245                 250                 255

Val Ala Asp Glu Val Ile Cys Gly Phe Gly Arg Thr Gly Glu Trp Phe
            260                 265                 270

Gly His Gln His Phe Gly Phe Gln Pro Asp Leu Phe Thr Ala Ala Lys
        275                 280                 285

Gly Leu Ser Ser Gly Tyr Leu Pro Ile Gly Ala Val Phe Val Gly Lys
    290                 295                 300

Arg Val Ala Glu Gly Leu Ile Ala Gly Gly Asp Phe Asn His Gly Phe
305                 310                 315                 320

Thr Tyr Ser Gly His Pro Val Cys Ala Ala Val Ala His Ala Asn Val
                325                 330                 335

Ala Ala Leu Arg Asp Glu Gly Ile Val Gln Arg Val Lys Asp Asp Ile
            340                 345                 350

Gly Pro Tyr Met Gln Lys Arg Trp Arg Glu Thr Phe Ser Arg Phe Glu
        355                 360                 365

His Val Asp Asp Val Arg Gly Val Gly Met Val Gln Ala Phe Thr Leu
    370                 375                 380

Val Lys Asn Lys Ala Lys Arg Glu Leu Phe Pro Asp Phe Gly Glu Ile
385                 390                 395                 400

Gly Thr Leu Cys Arg Asp Ile Phe Phe Arg Asn Asn Leu Ile Met Arg
                405                 410                 415

Ala Cys Gly Asp His Ile Val Ser Ala Pro Pro Leu Val Met Thr Arg
            420                 425                 430

Ala Glu Val Asp Glu Met Leu Ala Val Ala Glu Arg Cys Leu Glu Glu
        435                 440                 445

Phe Glu Gln Thr Leu Lys Ala Arg Gly Leu Ala
    450                 455

<210>  25
<211>  1380
<212>  DNA
<213>  Chromobacterium violaceum ATCC 12472

<400>  25
atgcagaaac agcgtaccac ctctcagtgg cgtgaactgg atgcggcgca tcatctgcat        60

ccgtttaccg ataccgcgag cctgaatcag gcgggtgcgc gtgtgatgac ccgtggcgaa 120

ggcgtgtatc tgtgggatag cgaaggcaac aaaattattg atggcatggc gggcctgtgg 180

tgcgtgaacg tgggctatgg ccgtaaagat tttgcggaag cggcgcgtcg tcagatggaa 240

gaactgccgt tttataacac cttctttaaa accacccatc cggcggtggt ggaactgagc 300

agcctgctgg ccgaagttac cccggcaggt tttgatcgtg tgttttatac caacagcggc 360

agcgaaagcg tggataccat gattcgtatg gtgcgtcgtt attgggatgt gcagggcaaa 420

ccggaaaaaa aaaccctgat tggccgttgg aacggctatc acggcagcac cattggcggt 480

gcgagcctgg gcggcatgaa atatatgcat gaacagggcg atctgccgat tccgggcatg 540

gcgcatattg aacagccgtg gtggtataaa catggcaaag atatgacccc ggatgaattt 600

ggcgtggttg cggcgcgttg gctggaagaa aaaattctgg aaatcggcgc ggataaagtg 660

gcggcgtttg tgggcgaacc gattcagggt gcgggcggtg tgattgttcc gccggcaacc 720

tattggccgg aaattgaacg tatttgccgc aaatatgatg tgctgctggt tgcggatgaa 780

gtgatttgcg gctttggccg taccggcgaa tggtttggcc atcagcattt tggctttcag 840

ccggacctgt ttaccgcggc gaaaggcctg agcagcggct atctgccgat tggcgcggtg 900

tttgtgggca aacgtgttgc ggaaggtctg attgcgggcg gtgattttaa ccatggcttt 960

acctatagcg gccatccggt gtgtgcggcg gtggcgcatg cgaatgttgc ggcgctgcgt 1020

gatgaaggca ttgtgcagcg tgtgaaagat gatattggcc cgtatatgca gaaacgttgg 1080

cgtgaaacct ttagccgttt tgaacatgtg gatgatgtgc gtggcgtggg catggtgcag 1140

gcgtttaccc tggtgaaaaa caaagcgaaa cgtgaactgt ttccggattt tggcgaaatt 1200

ggcaccctgt gccgcgatat tttttttcgc aacaacctga ttatgcgtgc gtgcggcgat 1260

cacattgtgt ctgcaccgcc gctggttatg acccgtgcgg aagtggatga aatgctggcc 1320

gtggcggaac gttgcctgga agaatttgaa cagaccctga aagcgcgtgg cctggcctaa 1380

<210> 26
<211> 552
<212> PRT
<213> Pseudomonas oleovorans

<400> 26

Met Tyr Asp Tyr Ile Ile Val Gly Ala Gly Ser Ala Gly Cys Val Leu
1               5                   10                  15

Ala Asn Arg Leu Ser Ala Asp Pro Ser Lys Arg Val Cys Leu Leu Glu
            20                  25                  30

Ala Gly Pro Arg Asp Thr Asn Pro Leu Ile His Met Pro Leu Gly Ile
        35                  40                  45

Ala Leu Leu Ser Asn Ser Lys Lys Leu Asn Trp Ala Phe Gln Thr Ala
    50                  55                  60

Pro Gln Gln Asn Leu Asn Gly Arg Ser Leu Phe Trp Pro Arg Gly Lys
65                  70                  75                  80

Thr Leu Gly Gly Ser Ser Ser Ile Asn Ala Met Val Tyr Ile Arg Gly
                85                  90                  95

His Glu Asp Asp Tyr His Ala Trp Glu Gln Ala Ala Gly Arg Tyr Trp
            100                 105                 110

Gly Trp Tyr Arg Ala Leu Glu Leu Phe Lys Arg Leu Glu Cys Asn Gln
        115                 120                 125

Arg Phe Asp Lys Ser Glu His His Gly Val Asp Gly Glu Leu Ala Val
    130                 135                 140

Ser Asp Leu Lys Tyr Ile Asn Pro Leu Ser Lys Ala Phe Val Gln Ala
145                 150                 155                 160

Gly Met Glu Ala Asn Ile Asn Phe Asn Gly Asp Phe Asn Gly Glu Tyr
                165                 170                 175

Gln Asp Gly Val Gly Phe Tyr Gln Val Thr Gln Lys Asn Gly Gln Arg
            180                 185                 190

Trp Ser Ser Ala Arg Ala Phe Leu His Gly Val Leu Ser Arg Pro Asn
        195                 200                 205

Leu Asp Ile Ile Thr Asp Ala His Ala Ser Lys Ile Leu Phe Glu Asp
    210                 215                 220

Arg Lys Ala Val Gly Val Ser Tyr Ile Lys Lys Asn Met His His Gln
225                 230                 235                 240

Val Lys Thr Thr Ser Gly Gly Glu Val Leu Leu Ser Leu Gly Ala Val
                245                 250                 255

Gly Thr Pro His Leu Leu Met Leu Ser Gly Val Gly Ala Ala Ala Glu
            260                 265                 270

Leu Lys Glu His Gly Val Ser Leu Val His Asp Leu Pro Glu Val Gly
        275                 280                 285

Lys Asn Leu Gln Asp His Leu Asp Ile Thr Leu Met Cys Ala Ala Asn
    290                 295                 300

Ser Arg Glu Pro Ile Gly Val Ala Leu Ser Phe Ile Pro Arg Gly Val

305                 310                 315                 320

Ser Gly Leu Phe Ser Tyr Val Phe Lys Arg Glu Gly Phe Leu Thr Ser
                325                 330                 335

Asn Val Ala Glu Ser Gly Gly Phe Val Lys Ser Ser Pro Asp Arg Asp
            340                 345                 350

Arg Pro Asn Leu Gln Phe His Phe Leu Pro Thr Tyr Leu Lys Asp His
        355                 360                 365

Gly Arg Lys Ile Ala Gly Gly Tyr Gly Tyr Thr Leu His Ile Cys Asp
    370                 375                 380

Leu Leu Pro Lys Ser Arg Gly Arg Ile Gly Leu Lys Ser Ala Asn Pro
385                 390                 395                 400

Leu Gln Pro Pro Leu Ile Asp Pro Asn Tyr Leu Ser Asp His Glu Asp
                405                 410                 415

Ile Lys Thr Met Ile Ala Gly Ile Lys Ile Gly Arg Ala Ile Leu Gln
            420                 425                 430

Ala Pro Ser Met Ala Lys His Phe Lys His Glu Val Val Pro Gly Gln
        435                 440                 445

Ala Val Lys Thr Asp Asp Glu Ile Ile Glu Asp Ile Arg Arg Arg Ala
    450                 455                 460

Glu Thr Ile Tyr His Pro Val Gly Thr Cys Arg Met Gly Lys Asp Pro
465                 470                 475                 480

Ala Ser Val Val Asp Pro Cys Leu Lys Ile Arg Gly Leu Ala Asn Ile
                485                 490                 495

Arg Val Val Asp Ala Ser Ile Met Pro His Leu Val Ala Gly Asn Thr
            500                 505                 510

Asn Ala Pro Thr Ile Met Ile Ala Glu Asn Ala Ala Glu Ile Ile Met
        515                 520                 525

Arg Asn Leu Asp Val Glu Ala Leu Glu Ala Ser Ala Glu Phe Ala Arg
    530                 535                 540

Glu Gly Ala Glu Leu Glu Leu Ala
545                 550

<210>  27
<211>  1656

<212>  DNA
<213>  Pseudomonas oleovorans

<400>  27
atgtacgact atataatcgt tggtgctgga tctgcaggat gtgtgcttgc taatcgtctt      60

tcggccgacc cctctaaaag agtttgttta cttgaagctg ggccgcgaga tacgaatccg     120

ctaattcata tgccgttagg tattgctttg ctttcaaata gtaaaaagtt gaattgggct     180

tttcaaactg cgccacagca aaatctcaac ggccggagcc ttttctggcc acgaggaaaa     240

acgttaggtg gttcaagctc aatcaacgca atggtctata tccgagggca tgaagacgat     300

taccacgcat gggagcaggc ggccggccgc tactggggtt ggtaccgggc tcttgagttg     360

ttcaaaaggc ttgaatgcaa ccagcgattc gataagtccg agcaccatgg ggttgacgga     420

gaattagctg ttagtgattt aaaatatatc aatccgctta gcaaagcatt cgtgcaagcc     480

ggcatggagg ccaatattaa tttcaacgga gatttcaacg gcgagtacca ggacggcgta     540

gggttctatc aagtaaccca aaaaaatgga caacgctgga gctcggcgcg tgcattcttg     600

cacggtgtac tttccagacc aaatctagac atcattactg atgcgcatgc atcaaaaatt     660

ctttttgaag accgtaaggc ggttggtgtt tcttatataa agaaaaatat gcaccatcaa     720

gtcaagacaa cgagtggtgg tgaagtactt cttagtcttg gcgcagtcgg cacgcctcac     780

cttctaatgc tttctggtgt tggggctgca gccgagctta aggaacatgg tgtttctcta     840

gtccatgatc ttcctgaggt ggggaaaaat cttcaagatc atttggacat cacattgatg     900

tgcgcagcaa attcgagaga gccgataggt gttgctcttt ctttcatccc tcgtggtgtc     960

tcgggtttgt tttcatatgt gtttaagcgc gaggggtttc tcactagtaa cgtggcagag    1020

tcgggtggtt ttgtaaaaag ttctcctgat cgtgatcggc ccaatttgca gtttcatttc    1080

cttccaactt atcttaaaga tcacggtcga aaaatagcgg gtggttatgg ttatacgcta    1140

catatatgtg atcttttgcc taagagccga ggcagaattg gcctaaaaag cgccaatcca    1200

ttacagccgc ctttaattga cccgaactat cttagcgatc atgaagatat taaaaccatg    1260

attgcgggta ttaagatagg gcgcgctatt ttgcaggccc catcgatggc gaagcatttt    1320

aagcatgaag tagtaccggg ccaggctgtt aaaactgatg atgaaataat cgaagatatt    1380

cgtaggcgag ctgagactat ataccatccg gtaggtactt gtaggatggg taaagatcca    1440

gcgtcagttg ttgatccgtg cctgaagatc cgtgggttgg caaatattag agtcgttgat    1500

gcgtcaatta tgccgcactt ggtcgcgggt aacacaaacg ctccaactat tatgattgca    1560

gaaaatgcgg cagaaataat tatgcggaat cttgatgtgg aagcattaga ggctagcgct    1620

gagtttgctc gcgagggtgc agagctagag ttggcc                              1656

<210>  28
<211>  535
<212>  PRT
<213>  Alcanivorax borkumensis str. SK2

<400>  28

Met Tyr Asp Tyr Ile Ile Val Gly Ala Gly Ser Ala Gly Cys Val Leu
1               5                   10                  15

Ala Asn Arg Leu Ser Ala Asp Thr Ser Lys Arg Val Ala Leu Ile Glu
            20                  25                  30

Ala Gly Pro Arg Asp Lys Asn Pro Leu Ile His Met Pro Ile Gly Ile
        35                  40                  45

Ala Leu Leu Ala Asn Asn Arg Lys Leu Asn Trp Ala Leu Glu Thr Glu
    50                  55                  60

Pro Gln Glu His Leu Lys Gly Arg Gln Leu Phe Trp Pro Arg Gly Lys
65                  70                  75                  80

Thr Leu Gly Gly Ser Ser Ser Ile Asn Ala Met Val Tyr Ile Arg Gly
                85                  90                  95

His Lys Ala Asp Tyr Asp His Trp Gly Gln Val Ala Gly Asn Asn Asn
            100                 105                 110

Leu Trp Gly Trp Asp Arg Ala Leu Thr Leu Phe Arg Arg Val Glu Asp
        115                 120                 125

Asn Gln Arg Leu Gly Ala Asp Pro Tyr His Gly Lys Asp Gly Glu Leu
    130                 135                 140

Thr Val Ser Glu Leu Lys Ser Ile Asn Pro Leu Ser Arg Asp Phe Val
145                 150                 155                 160

Arg Ala Ala Pro His Val Asp Leu Pro Val Asn Thr Asp Phe Asn Gly
                165                 170                 175

Lys Ser Gln Asp Gly Leu Gly Leu Tyr Gln Val Thr Gln Lys Asn Gly
            180                 185                 190

Gln Arg Trp Ser Ser Ala Gln Ala Phe Leu Arg Ala Ala Glu Ser Arg
        195                 200                 205

Ser Asn Leu Asp Val Leu Thr Asp Ala Arg Val Thr Arg Val Ala Met
    210                 215                 220

Glu Gly Lys Arg Ala Val Gly Val Thr Leu Lys Gln Gly Ser Glu Tyr
225                 230                 235                 240

Arg Gln Leu Arg Leu Asn Ala Gly Gly Glu Val Ile Leu Ser Gly Gly
                245                 250                 255

Ala Val Asn Ser Pro Gln Leu Leu Leu Leu Ser Gly Ile Gly Asp Ser
            260                 265                 270

Lys Glu Leu Ala Lys His Gly Ile Pro Leu Val His His Leu Pro Glu
        275                 280                 285

Val Gly Gln Asn Leu Ala Asp His Leu Asp Ile Thr Ile Met His Thr
    290                 295                 300

Ala Asn Ser Arg Leu Pro Ile Gly Val Ala Pro Ser Phe Leu Phe Arg
305                 310                 315                 320

Gly Val Ser Ala Leu Phe Ser Tyr Ile Phe Ala Arg Arg Gly Phe Leu
                325                 330                 335

Thr Ser Asn Val Ala Glu Ser Gly Gly Phe Val Lys Ser Asp Pro Ser
            340                 345                 350

Ser Glu Arg Pro Asn Val Gln Phe His Phe Leu Pro Thr Tyr Leu Lys
        355                 360                 365

Asp His Gly Arg Lys Val Met Ala Gly Tyr Gly Tyr Thr Leu His Ile
    370                 375                 380

Cys Asp Leu Leu Pro Lys Ser Arg Gly Phe Ile Gly Leu Gln Ser Pro
385                 390                 395                 400

Asp Pro Leu Ala Asn Pro Leu Ile Gln Pro Asn Tyr Leu Ser Asp Pro
                405                 410                 415

Glu Asp Ile Lys Thr Met Ile Ser Ala Ile Lys Phe Gly Arg Arg Ile
            420                 425                 430

Leu Gly Ala Pro Thr Met Ala Leu His Ser Lys Arg Glu Val Met Pro
        435                 440                 445

Gly Glu Ser Val Ser Thr Asp Ala Gln Leu Ala Asp Phe Ile Arg Glu
    450                 455                 460

Asn Ala Glu Thr Ile Tyr His Pro Val Gly Thr Cys Arg Met Gly Ala
465                 470                 475                 480

Asp Pro Asp Ser Val Val Asp Pro Glu Leu Lys Val Arg Gly Val Glu
                485                 490                 495

Gly Leu Arg Val Val Asp Ala Ser Ile Met Pro Ser Leu Val Ala Gly
            500                 505                 510

Asn Thr Asn Ala Pro Thr Met Met Ile Ala Glu Asn Ala Ala Asp Ile
        515                 520                 525

Leu Leu Gly Lys Val Gln Val
    530                 535

<210>  29
<211>  1608
<212>  DNA
<213>  Oxidoreduktase der Glucose-Methanol-Cholin-Oxidoreduktase-Familie
YP_694430 (Ab_AlkJ)

<400>  29
atgtacgatt acattattgt aggagccggg tcggctggct gtgtgttggc taaccgcctt      60

agtgcggata cttcaaaacg cgtagcgctg atcgaagccg gcccacggga taaaaatccc     120

ctaattcaca tgcctattgg catcgctctc ctcgccaaca atagaaaact caactgggcg     180

ctagaaaccg agcctcaaga acacttgaaa gggcgccaac tgttttggcc ccgcggaaag     240

acgcttggtg gctcttcttc tattaatgcc atggtgtata tcagggggca taaagccgac     300

tacgatcatt ggggccaagt tgccggtaac aacaacctct ggggttggga tcgtgcatta     360

acattatttc gtcgggtgga agacaaccaa cgccttggcg cagacccata ccacggcaaa     420

gatggtgagc tcactgtaag cgaattaaaa tcgatcaacc cgctgagccg tgattttgtt     480

cgagcagcgc ctcacgtaga cctgcccgtg aacacagact ttaacggcaa atcacaagac     540

ggattggggc tttaccaagt aacgcagaaa aatggccagc gctggagttc agcgcaagca     600

tttttgcgtg ccgctgagag ccgctctaat cttgacgtgc taaccgatgc tcgagtaacc     660

cgcgtggcta tggagggtaa gcgagcggtt ggcgtgaccc tgaaacaggg aagtgaatat     720

cgccagctga gactcaatgc cggcggcgaa gtcatcctgt ctggtggtgc agttaattca     780

ccacagctcc tcctgctgtc tggcatcggg gatagtaaag agcttgcgaa acacggcatt     840

ccgctagttc atcaccttcc cgaagtcggc cagaatttgg ccgatcatct ggacatcacg     900

atcatgcaca cggcgaactc tcgtttgccc attggcgttg cacccagctt cttattccgt     960

ggggtgagcg cacttttctc ctatatcttt gcgcgacgtg gttttcttac cagtaatgtt    1020

gccgagtctg gcggctttgt gaaatcggac ccttcgtctg agcgacccaa tgtgcaattt    1080

cactttttgc ccacttacct gaaggatcat ggccgaaaag taatggcagg ctatggctac    1140

actttgcaca tttgcgattt gttgccgaaa agccgaggct tcattggatt gcaaagccct    1200

gacccattgg ccaatccgct gattcagcct aactatctga gcgaccccga agatatcaaa    1260

acaatgatat ccgccattaa gtttggcgca cgcattctcg gagcaccaac aatggcgctt    1320

catagtaaac gggaagttat gccgggagag tccgtatcta cggacgctca actagcagac    1380

tttatccgtg aaaatgctga aaccatctac caccctgttg gcacttgtcg tatgggggcc    1440

gaccctgatt ccgttgtcga tccggaactg aaagtcagag gcgttgaagg gctaagagtt    1500

gtcgatgcct cgataatgcc cagcttggtg gcgggtaaca cgaacgcacc cacaatgatg 1560

attgccgaaa atgcggccga catcctgctg ggaaaggtcc aagtgtaa 1608

<210> 30
<211> 416
<212> PRT
<213> Marinobacter aquaeolei VT8

<400> 30

Met Val Ser Lys Arg Lys Glu Arg Thr Val Ile Val Gly Gly Gly His
1               5                   10                  15

Ala Ala Gly Ala Leu Leu Thr Ala Leu Leu Gln Lys Lys Tyr Gln His
            20                  25                  30

Glu Val Val Leu Val Gly Asn Glu Pro His Pro Pro Tyr His Arg Pro
        35                  40                  45

Pro Leu Ser Lys Asn Tyr Leu Thr Gly Asp Val Asp Gln Glu Ser Leu
    50                  55                  60

Tyr Leu Lys Pro Arg Ser Val Tyr Glu Asn Ala Gly His Gln Leu Arg
65                  70                  75                  80

Leu Gly Val Arg Val Glu Gln Ile Asp Arg Asp Ser Ser Thr Ile Ser
                85                  90                  95

Leu Ser Asp Gln Ser Arg Leu Gln Tyr Asp Arg Leu Val Leu Ala Thr
            100                 105                 110

Gly Ser His Leu Arg His Leu Asn Ala Pro Gly Ala Asp Leu Asn Gly
        115                 120                 125

Ile His Tyr Leu His Asp Ile Ala Asp Ser Glu Val Leu Arg Glu Gln
    130                 135                 140

Leu Val Ala Gly Lys Arg Leu Val Val Val Gly Gly Gly Tyr Ile Gly
145                 150                 155                 160

Leu Glu Val Ala Ala Ser Ala Asn Lys Lys Gly Val Asn Val Thr Val
                165                 170                 175

Leu Glu Ala Ala Glu Arg Leu Met Gln Arg Val Thr Gly Pro Glu Ile
            180                 185                 190

Ser Ala Phe Leu Tyr Asp Lys His Arg Gly Ala Gly Val Asp Val Arg
        195                 200                 205

Leu Asn Thr Ala Val Thr Gly Phe Glu Ala Gly Asp Gln Gly His Val
    210                 215                 220

Ala Gly Val Thr Leu Ala Asp Gly Ser Thr Val Pro Ala Asp Ile Val
225                 230                 235                 240

Leu Val Ser Ile Gly Ile Ile Pro Glu Thr Ala Leu Ala Lys Asp Ala
                245                 250                 255

Gly Leu Pro Cys Asp Asn Gly Ile Ile Val Asp Glu Phe Thr Arg Thr
            260                 265                 270

Glu Asp Pro Ala Ile Leu Ala Ile Gly Asp Cys Thr Arg His Arg Asn
        275                 280                 285

Leu Phe Phe Glu Lys Met Gln Arg Leu Glu Ser Val Ala Asn Ala Val
    290                 295                 300

Asp Gln Ala Arg Thr Ala Ala Ala Thr Leu Met Gly Glu Glu Lys Pro
305                 310                 315                 320

Tyr Asp Ser Val Pro Trp Phe Trp Ser Asn Gln Tyr Asp Val Arg Leu
                325                 330                 335

Gln Met Val Gly Leu Ser Gln Asn His Asp Gln Arg Val Val Arg Gly
            340                 345                 350

Thr Pro Glu Asp Lys Gly Phe Ala Val Phe Tyr Leu Arg Glu Gly Cys
        355                 360                 365

Val Ile Ala Val Asp Ala Val Asn Leu Pro Leu Ala Phe Leu Val Gly
    370                 375                 380

Lys Thr Leu Val Gln Gln Arg Arg Thr Ile Asn Pro Glu Leu Ile Glu
385                 390                 395                 400

Asp Pro Asp Thr Glu Leu Lys Ser Leu Val Asn Gly Arg Leu Gln Ser
                405                 410                 415

<210>  31
<211>  1251
<212>  DNA
<213>  Marinobacter aquaeolei VT8

<400>  31
atggtaagca aacgtaaaga gaggacggtc attgttggcg gtgggcacgc agcaggtgcc      60

ctcctgacag ccttactcca aaaaaaatat caacatgagg tcgttctggt ggggaatgaa     120

cctcatccgc cctaccatcg accgccgctg tccaagaatt acctgacagg agacgttgat     180

caggagtcgc tgtacctgaa accgcgctcg gtatacgaga acgcaggcca tcagttgcgg     240

ctcggtgtgc gcgtcgaaca aattgatcgg gacagtagca ccatcagctt gtcggatcag 300

agcaggctgc aatacgatcg actggtcctg gccaccgggt cacaccttcg acacctgaac 360

gcgcccgggg ctgacttaaa tggcattcat tacctgcacg acatagctga ttcagaggta 420

ctgcgtgaac agttagttgc tggaaagcgc ctggtcgtcg tgggtggtgg ttacatcggc 480

cttgaggtgg cggccagtgc caacaaaaaa ggtgttaatg tcacggtgct agaagccgcc 540

gaacgtctta tgcagcgcgt tacgggcccg gaaatatcag cgttccttta cgacaaacac 600

cgtggcgccg gcgtggacgt acgtctgaac acagcggtaa ccggcttcga agcgggcgat 660

caggggcatg tggctggcgt gacgttggcg gacggaagca ccgtaccggc cgacatcgtc 720

cttgtgtcga tcggcattat cccggaaacc gctctggcta aggacgccgg cctgccctgt 780

gataacggta ttattgttga cgaatttacc cgtaccgagg accccgccat cttggcgatc 840

ggtgactgca cccggcaccg gaatcttttc ttcgagaaga tgcaacgact cgagtctgtc 900

gccaatgctg tcgatcaggc tcgtacagcc gcggcaaccc tgatgggtga ggagaaaccc 960

tatgatagcg ttccatggtt ctggtcaaac cagtacgatg ttcgtctgca gatggtagga 1020

ttgtcgcaaa atcatgatca gcgagtggtt cgaggcaccc ccgaggataa aggatttgcc 1080

gtgttctatc tccgcgaagg ctgtgttatt gctgttgacg cggtcaacct gccccttgct 1140

tttttggtag gcaagacact cgttcaacaa cgcagaacga tcaacccgga actaatagag 1200

gatccggata ctgaactgaa atctttggtg aacggaaggc tccagagttg a 1251

<210> 32
<211> 404
<212> PRT
<213> Acinetobacter sp. OC4

<400> 32

Met Gln Thr Ile Val Ile Ile Gly Ala Ser His Ala Ala Ala Gln Leu
1               5                   10                  15

Ala Ala Ser Leu Arg Pro Asp Gly Trp Gln Gly Glu Ile Val Val Ile
            20                  25                  30

Gly Asp Glu Pro Tyr Leu Pro Tyr His Arg Pro Pro Leu Ser Lys Thr
        35                  40                  45

Phe Leu Arg Gly Ala Gln Leu Val Asp Glu Leu Leu Ile Arg Pro Ala
    50                  55                  60

Ala Phe Tyr Gln Lys Asn Gln Ile Glu Phe Arg His Gly Arg Val Val
65                  70                  75                  80

Ala Ile Asp Arg Ala Ala Arg Ser Val Thr Leu Gln Asp Gly Ser Thr
                85                  90                  95

Leu Ala Tyr Asp Gln Leu Ala Leu Cys Thr Gly Ala Arg Val Arg Thr
            100                 105                 110

Val Ser Leu Ala Gly Ser Asp Leu Ala Gly Val His Tyr Leu Arg Asn
        115                 120                 125

Ile Ser Asp Val Gln Ala Ile Gln Pro Phe Val Gln Pro Asn Gly Lys
    130                 135                 140

Ala Val Val Ile Gly Gly Gly Tyr Ile Gly Leu Glu Thr Ala Ala Ala
145                 150                 155                 160

Leu Thr Glu Gln Gly Met Gln Val Val Val Leu Glu Ala Ala Glu Arg
                165                 170                 175

Ile Leu Gln Arg Val Thr Ala Pro Glu Val Ser Asp Phe Tyr Thr Arg
            180                 185                 190

Ile His Arg Glu Gln Gly Val Thr Ile His Thr Gly Val Ser Val Thr
        195                 200                 205

Ala Ile Thr Gly Glu Gly Arg Ala Gln Ala Val Leu Cys Ala Asp Gly
    210                 215                 220

Ser Met Phe Asp Ala Asp Leu Val Ile Ile Gly Val Gly Val Val Pro
225                 230                 235                 240

Asn Ile Glu Leu Ala Leu Asp Ala Gly Leu Gln Val Asp Asn Gly Ile
                245                 250                 255

Val Ile Asp Glu Tyr Cys Arg Thr Ser Ala Pro Glu Ile Val Ala Ile
            260                 265                 270

Gly Asp Cys Ala Asn Ala Phe Asn Pro Ile Tyr Gln Arg Arg Met Arg
        275                 280                 285

Leu Glu Ser Val Pro Asn Ala Asn Glu Gln Ala Lys Ile Ala Ser Ala
    290                 295                 300

Thr Leu Cys Gly Leu Gln Arg Thr Ser Lys Ser Leu Pro Trp Phe Trp
305                 310                 315                 320

Ser Asp Gln Tyr Asp Leu Lys Leu Gln Ile Ala Gly Leu Ser Gln Gly
                325                 330                 335

Tyr Asp Gln Ile Val Ile Arg Gly Asp Val Gln Gln Arg Arg Ser Phe
            340                 345                 350

Ala Ala Phe Tyr Leu Gln Ala Gly Arg Leu Ile Ala Ala Asp Cys Val
        355                 360                 365

Asn Arg Pro Gln Glu Phe Met Leu Ser Lys Lys Leu Ile Thr Ala Gly
    370                 375                 380

Thr Ala Val Asp Pro Leu Arg Leu Ala Asp Glu Ser Ile Ala Val Gln
385                 390                 395                 400

Ala Leu Met Gly

<210>  33
<211>  1215
<212>  DNA
<213>  Acinetobacter sp. OC4

<400>  33
atgcagacca tcgtaatcat cggcgccagc cacgcagcag cccaactggc cgcgagcttg      60

cgtccggatg gttggcaggg tgagatcgtt gtcatcggtg atgaaccgta tctgccatac     120

caccgtccgc cgctgagcaa aaccttcctg cgtggtgcac aactggttga tgaactgctg     180

attcgtcctg ccgcttttta ccaaaagaac cagattgagt tccgtcacgg tcgtgtggtc     240

gcaattgatc gtgcagcgcg tagcgttacc ctgcaagacg gtagcaccct ggcttatgat     300

cagctggcac tgtgcactgg tgcacgtgtg cgtaccgtta gcttggctgg cagcgacctg     360

gctggtgttc actacctgcg caacattagc gatgtccaag caatccagcc gttcgtgcaa     420

ccgaatggta aagcggttgt gattggtggc ggctacatcg gtctggaaac ggctgcggcc     480

ctgacggaac aaggcatgca ggtggttgtt ttggaggcgg ctgagcgcat cctgcaacgc     540

gttacggcgc ctgaagttag cgacttttac acccgtattc accgtgaaca gggcgttacc     600

attcacacgg gtgtctcggt gacggccatt accggcgaag gtcgtgcgca ggcggtgctg     660

tgcgcggatg gtagcatgtt tgatgctgat ttggttatca ttggtgtcgg cgtcgtcccg     720

aatatcgagc tggctctgga tgcgggtctg caggtggata atggcatcgt catcgacgag     780

tactgtcgca cctctgcgcc ggagatcgtc gccattggtg actgcgcgaa tgctttcaac     840

ccgatctacc agcgtcgtat gcgcttggaa tcggtgccga acgcgaacga acaagcaaag     900

attgcaagcg caaccctgtg cggtctgcag cgtaccagca aatccctgcc gtggttttgg     960

agcgatcaat atgatctgaa actgcaaatt gctggcctga gccaaggtta tgaccaaatt    1020

gtgattcgtg gcgacgttca acaacgccgc agcttcgcgg cgttctacct gcaggcgggt    1080

cgtctgatcg cggcagattg tgtaaatcgt ccgcaggagt ttatgctgag caagaagctg    1140

attaccgctg gtacggcggt ggacccgctg cgtctggctg acgagagcat cgctgtacag    1200

gcgctgatgg gctga                                                     1215

<210>  34
<211>  470
<212>  PRT
<213>  Marinobacter aquaeolei VT8

<400>  34

Met Pro Thr Leu Pro Arg Thr Phe Asp Asp Ile Gln Ser Arg Leu Ile
1               5                   10                  15

Asn Ala Thr Ser Arg Val Val Pro Met Gln Arg Gln Ile Gln Gly Leu
            20                  25                  30

Lys Phe Leu Met Ser Ala Lys Arg Lys Thr Phe Gly Pro Arg Arg Pro
        35                  40                  45

Met Pro Glu Phe Val Glu Thr Pro Ile Pro Asp Val Asn Thr Leu Ala
    50                  55                  60

Leu Glu Asp Ile Asp Val Ser Asn Pro Phe Leu Tyr Arg Gln Gly Gln
65                  70                  75                  80

Trp Arg Ala Tyr Phe Lys Arg Leu Arg Asp Glu Ala Pro Val His Tyr
                85                  90                  95

Gln Lys Asn Ser Pro Phe Gly Pro Phe Trp Ser Val Thr Arg Phe Glu
            100                 105                 110

Asp Ile Leu Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Ser Ser Val Gln
145                 150                 155                 160

Gly Val Val Ala Pro Lys Asn Leu Lys Glu Met Glu Gly Leu Ile Arg
                165                 170                 175

Ser Arg Thr Gly Asp Val Leu Asp Ser Leu Pro Thr Asp Lys Pro Phe
            180                 185                 190

Asn Trp Val Pro Ala Val Ser Lys Glu Leu Thr Gly Arg Met Leu Ala
        195                 200                 205

Thr Leu Leu Asp Phe Pro Tyr Glu Glu Arg His Lys Leu Val Glu Trp
    210                 215                 220

Ser Asp Arg Met Ala Gly Ala Ala Ser Ala Thr Gly Gly Glu Phe Ala

225                 230                 235                 240

Asp Glu Asn Ala Met Phe Asp Asp Ala Ala Asp Met Ala Arg Ser Phe
                245                 250                 255

Ser Arg Leu Trp Arg Asp Lys Glu Ala Arg Arg Ala Ala Gly Glu Glu
            260                 265                 270

Pro Gly Phe Asp Leu Ile Ser Leu Leu Gln Ser Asn Lys Glu Thr Lys
        275                 280                 285

Asp Leu Ile Asn Arg Pro Met Glu Phe Ile Gly Asn Leu Thr Leu Leu
    290                 295                 300

Ile Val Gly Gly Asn Asp Thr Thr Arg Asn Ser Met Ser Gly Gly Leu
305                 310                 315                 320

Val Ala Met Asn Glu Phe Pro Arg Glu Phe Glu Lys Leu Lys Ala Lys
                325                 330                 335

Pro Glu Leu Ile Pro Asn Met Val Ser Glu Ile Ile Arg Trp Gln Thr
            340                 345                 350

Pro Leu Ala Tyr Met Arg Arg Ile Ala Lys Gln Asp Val Glu Leu Gly
        355                 360                 365

Gly Gln Thr Ile Lys Lys Gly Asp Arg Val Val Met Trp Tyr Ala Ser
    370                 375                 380

Gly Asn Arg Asp Glu Arg Lys Phe Asp Asn Pro Asp Gln Phe Ile Ile
385                 390                 395                 400

Asp Arg Lys Asp Ala Arg Asn His Met Ser Phe Gly Tyr Gly Val His
                405                 410                 415

Arg Cys Met Gly Asn Arg Leu Ala Glu Leu Gln Leu Arg Ile Leu Trp
            420                 425                 430

Glu Glu Ile Leu Lys Arg Phe Asp Asn Ile Glu Val Val Glu Glu Pro
        435                 440                 445

Glu Arg Val Gln Ser Asn Phe Val Arg Gly Tyr Ser Arg Leu Met Val
    450                 455                 460

Lys Leu Thr Pro Asn Ser
465                 470

<210>  35
<211>  1413

<212>  DNA
<213>  Marinobacter aquaeolei VT8

<400>  35
atgccaacac tgcccagaac atttgacgac attcagtccc gactgattaa cgccacctcc      60

agggtggtgc cgatgcagag gcaaattcag ggactgaaat tcttaatgag cgccaagagg     120

aagaccttcg gcccacgccg accgatgccc gaattcgttg aaacacccat cccggacgtt     180

aacacgctgg cccttgagga catcgatgtc agcaatccgt tttataccgg gcagggtcag      240

tggcgcgcct atttcaaacg gttgcgtgat gaggcgccgg tccattacca gaagaacagc     300

cctttcggcc ccttctggtc ggtaactcgg tttgaagaca tcctgttcgt ggataagagt     360

cacgacctgt tttccgccga gccgcaaatc attctcggtg accctccgga ggggctgtcg     420

gtggaaatgt tcatagcgat ggatccgccg aaacacgatg tgcagcgcag ctcggtgcag     480

ggagtagtgg caccgaaaaa cctgaaggag atggaggggc tgatccgatc acgcaccggc     540

gatgtgcttg acagcctgcc tacagacaaa ccctttaact gggtacctgc tgtttccaag     600

gaactcacag gccgcatgct ggcgacgctt ctggattttc cttacgagga acgccacaag     660

ctggttgagt ggtcggacag aatggcaggt gcagcatcgg ccaccggcgg ggagtttgcc     720

gatgaaaatg ccatgtttga cgacgcggca gacatggccc ggtctttctc caggctttgg     780

cgggacaagg aggcgcgccg cgcagcaggc gaggagcccg gtttcgattt gatcagcctg     840

ttgcagagca acaaagaaac gaaagacctg atcaatcggc cgatggagtt tatcggtaat     900

ttgacgctgc tcatagtcgg cggcaacgat acgacgcgca actcgatgag tggtggcctg     960

gtggccatga acgaattccc cagggaattt gaaaaattga aggcaaaacc ggagttgatt    1020

ccgaacatgg tgtcggaaat catccgctgg caaacgccgc tggcctatat gcgccgaatc    1080

gccaagcagg atgtcgaact gggcggccag accatcaaga agggtgatcg agttgtcatg    1140

tggtacgcgt cgggtaaccg ggacgagcgc aaatttgaca accccgatca gttcatcatt    1200

gatcgcaagg acgcacgaaa ccacatgtcg ttcggctatg gggttcaccg ttgcatgggc    1260

aaccgtctgg ctgaactgca actgcgcatc ctctgggaag aaatactcaa gcgttttgac    1320

aacatcgaag tcgtcgaaga gcccgagcgg gtgcagtcca acttcgtgcg gggctattcc    1380

aggttgatgg tcaaactgac accgaacagt taa                                 1413

<210>  36
<211>  106
<212>  PRT
<213>  Acinetobacter sp. OC4

<400>  36

Met Gly Gln Ile Thr Phe Ile Ala His Asp Gly Ala Gln Thr Ser Val
1               5                   10                  15

Ala Ile Glu Ala Gly Lys Ser Leu Met Gln Leu Ala Val Glu Asn Gly

20                  25                  30

Val Ala Gly Ile Asp Gly Asp Cys Gly Gly Glu Cys Ala Cys Gly Thr
        35                  40                  45

Cys His Val Ile Val Ser Ala Glu Trp Ser Asp Val Ala Gly Thr Ala
    50                  55                  60

Gln Ala Asn Glu Gln Gln Met Leu Glu Met Thr Pro Glu Arg Ala Ala
65                  70                  75                  80

Thr Ser Arg Leu Ala Cys Cys Ile Gln Val Thr Asp Ala Met Asp Gly
                85                  90                  95

Met Thr Val His Leu Pro Glu Phe Gln Met
            100                 105

<210>  37
<211>  321
<212>  DNA
<213>  Acinetobacter sp. OC4

<400>  37
atgggccaaa ttactttcat cgcacacgac ggcgcacaga cgagcgttgc gatcgaggcg        60

ggtaagtctt tgatgcagct ggcggtagaa aacggtgtcg ccggcatcga tggcgactgc       120

ggtggcgagt gtgcgtgtgg cacctgtcat gtcattgtct ctgcggagtg gagcgatgtg       180

gccggtaccg cgcaagccaa tgagcagcag atgctggaga tgactccgga gcgtgcggcc       240

accagccgtc tggcatgttg catccaagtg acggacgcga tggatggtat gaccgtccat       300

ctgccggaat ttcagatgta a                                                 321

<210>  38
<211>  542
<212>  PRT
<213>  Caulobacter sp K31

<400>  38

Met Phe Asp Tyr Ile Ile Val Gly Ala Gly Ser Ala Gly Cys Leu Leu
1               5                   10                  15

Ala Glu Arg Leu Ser Ala Asn Pro Arg Thr Arg Val Cys Leu Leu Glu
            20                  25                  30

Ala Gly Pro Pro Asp Arg Ser Pro Leu Ile His Met Pro Ile Gly Ile
        35                  40                  45

Ala Leu Leu Ser Lys Ser Lys Ile Leu Asn Trp Ala Phe Glu Thr Gln
    50                  55                  60

Pro Gln Ala Asn Leu Asp Gly Arg Arg Leu Phe Trp Pro Arg Gly Lys
65                  70                  75                  80

Thr Leu Gly Gly Ser Ser Ser Ile Asn Ala Met Val Tyr Ile Arg Gly
                85                  90                  95

His Arg Asp Asp Tyr Asp Ser Trp Gly Glu Ala Ala Asp Pro Ile Trp
            100                 105                 110

Ser Tyr Asp Asn Val Leu Pro Leu Phe Lys Ala Met Glu Ser Asn Glu
        115                 120                 125

Arg Phe Gly Thr Asp Ala Phe His Gly Gly Asp Gly Glu Leu His Val
    130                 135                 140

Ser Asp Leu Arg Thr Arg Asn Pro Leu Ser Asp Ala Phe Val Glu Ala
145                 150                 155                 160

Gly Gln Gln Ala Gln Phe Pro His Ala Val Asp Phe Asn Gly Lys Met
                165                 170                 175

Gln Asp Gly Val Gly Leu Tyr Gln Val Thr Gln His Lys Gly Arg Arg
            180                 185                 190

Trp Ser Ser Ala Arg Ala Phe Leu Ser Lys Ala Lys Gly Arg Pro Asn
        195                 200                 205

Leu Arg Ile Val Thr Gly Ala Arg Ala Thr Arg Ile Ile Leu Glu Gly
    210                 215                 220

Arg Lys Ala Val Gly Val Thr Tyr Ala Ala Gly Gly Lys Leu Val Asp
225                 230                 235                 240

Val Arg Thr Arg Gly Gly Glu Val Ile Leu Ser Gly Gly Ala Val Asn
                245                 250                 255

Ser Pro Gln Leu Leu Leu Leu Ser Gly Ile Gly Gly Ala Ala Glu Leu
            260                 265                 270

Asn Ala Leu Gly Ile Pro Val Val Val Asp Leu Pro Ala Val Gly Lys
        275                 280                 285

Asn Leu Gln Asp His Leu Asp Ile Thr Ile Met His Glu Ala Asn Asp
    290                 295                 300

Arg Thr Pro Ile Gly Ile Ala Pro Ser Phe Ile Pro Arg Ala Leu Ser
305                 310                 315                 320

Gly Ala Leu Ser Tyr Ala Phe Leu Arg Lys Gly Phe Leu Thr Ser Asn

325                 330                 335

Val Ala Glu Ala Gly Gly Phe Val Lys Ser Thr Pro Ser Arg Ser Arg
            340                 345                 350

Pro Asn Leu Gln Phe His Phe Leu Pro Thr Leu Leu Lys Asp His Gly
        355                 360                 365

Arg Glu Met Ala Phe Gly Tyr Gly Tyr Thr Leu His Val Cys Asp Leu
    370                 375                 380

Leu Pro Lys Ser Arg Gly Arg Ile Gly Leu Thr Ser Pro Asp Pro Leu
385                 390                 395                 400

Asp Asp Pro Leu Ile Asp Pro Asn Tyr Leu Ser Ala Pro Glu Asp Ile
                405                 410                 415

Glu Thr Met Val Ala Ala Val Lys Ile Gly Arg Gln Ile Leu Ser Ala
            420                 425                 430

Pro Ser Met Ala Ala Phe Ser Lys Thr Glu Leu Val Pro Gly Pro Ser
        435                 440                 445

Val Gln Ser Lys Ala Asp Ile Met Ala Asp Ile Arg Arg Arg Ala Glu
    450                 455                 460

Thr Ile Tyr His Pro Val Gly Thr Cys Arg Met Gly Arg Asp Pro Gln
465                 470                 475                 480

Ser Val Val Asp Pro Ser Leu Arg Val Arg Gly Val Gln Gly Leu Arg
                485                 490                 495

Val Val Asp Ala Ser Val Met Pro Thr Leu Val Ala Gly Asn Thr Asn
            500                 505                 510

Ala Pro Thr Met Met Ile Ala Glu Arg Ala Ala Glu Leu Ile Leu Gly
        515                 520                 525

Lys Thr Lys Leu Ala Leu Ser Ala Asn Ile Glu Ala Phe Arg
    530                 535                 540

<210>  39
<211>  1629
<212>  DNA
<213>  Caulobacter sp. K31

<400>  39
atgtttgact atattattgt tggagcgggg tctgccggat gcttgttggc ggagcgcttg      60

tcagccaatc ccaggacgcg ggtctgtctg cttgaggcgg gcccgcccga ccgcagcccg     120

ctgatccaca tgcccattgg gatagcgctt ctgtcaaaga gcaaaattct caattgggca 180

ttcgagacgc agccacaggc caatctcgat ggtcgacggc tgttttggcc gcgcggcaaa 240

acccttggcg gatcgagttc gatcaatgcg atggtctata tccgcgggca ccgggatgac 300

tatgactcct ggggcgaggc agccgatccg atctggtcct atgacaatgt gctcccgctg 360

ttcaaggcga tggagtccaa cgagagattt ggaaccgacg cgtttcatgg cggcgatggt 420

gagcttcacg tcagcgacct gcgaacccgc aaccccttga gcgatgcctt cgtcgaggcc 480

ggacaacagg cccagtttcc gcatgccgtc gatttcaatg ggaagatgca ggacggcgtc 540

ggcctgtacc aggtcaccca gcacaaaggc cggcgctgga gttccgcgcg cgcctttctt 600

tccaaggcca agggccggcc caatctacgg atagtcacgg gcgcgcgggc tacccggatc 660

attctggagg gccgcaaagc ggtcggcgtg acctatgccg caggcggcaa gctggtcgat 720

gtgcgaacca ggggcggcga ggtcattctt tcgggcggcg ccgtcaattc cccgcaactg 780

ctgctgcttt ccggcatcgg cggcgcggcc gagctgaacg cactcggcat tccggtggtc 840

gtcgaccttc cggcagttgg aaaaaatctg caggatcacc tcgatatcac aatcatgcat 900

gaggcgaacg atcgtacacc gatcggcatc gcaccgtcat tcatcccgcg ggcgctgtcc 960

ggagcgctat cctacgcctt ccttcgaaag ggtttcttga cgagcaacgt cgccgaggcg 1020

ggcggcttcg tcaaaagcac accttcgcgg agtcggccga atctacagtt tcatttcctc 1080

cccacgcttt tgaaggacca tgggcgcgaa atggcgttcg ggtatggcta tacattgcat 1140

gtctgcgatc ttctgcccaa gagccgaggc cgcatcgggc tcacaagccc cgacccgctc 1200

gacgatccgc tgatcgatcc aaactatctc tcggcccccg aagacattga gaccatggtc 1260

gcggcggtga agatcggccg gcaaattctg tcggcgccgt caatggcggc cttctcgaaa 1320

accgaactgg tccctgggcc atcggtccag agcaaggcgg atatcatggc ggatatccgt 1380

cggcgagcgg agacgatcta tcatccggtg ggaacatgcc ggatgggacg agaccctcag 1440

tcggttgtcg atccgtcact ccgagtgcgt ggcgtgcaag gccttcgcgt cgtcgacgcc 1500

tcggtcatgc cgacgctggt cgccggaaac accaacgccc cgacgatgat gattgcggaa 1560

agagctgccg agctcattct tgggaagacg aaactcgcac tcagcgccaa cattgaggca 1620

ttccgctaa 1629

<210> 40
<211> 704
<212> PRT
<213> Candida tropicalis

<400> 40

Met Ala Pro Phe Leu Pro Asp Gln Val Asp Tyr Lys His Val Asp Thr
1               5                   10                  15

Leu Met Leu Leu Cys Asp Gly Ile Ile His Glu Thr Thr Val Asp Glu

20                  25                  30

Ile Lys Asp Val Ile Ala Pro Asp Phe Pro Ala Asp Lys Tyr Glu Glu
        35                  40                  45

Tyr Val Arg Thr Phe Thr Lys Pro Ser Glu Thr Pro Gly Phe Arg Glu
    50                  55                  60

Thr Val Tyr Asn Thr Val Asn Ala Asn Thr Met Asp Ala Ile His Gln
65                  70                  75                  80

Phe Ile Ile Leu Thr Asn Val Leu Gly Ser Arg Val Leu Ala Pro Ala
                85                  90                  95

Leu Thr Asn Ser Leu Thr Pro Ile Lys Asp Met Ser Leu Glu Asp Arg
            100                 105                 110

Glu Lys Leu Leu Ala Ser Trp Arg Asp Ser Pro Ile Ala Ala Lys Arg
        115                 120                 125

Lys Leu Phe Arg Leu Val Ser Thr Leu Thr Leu Val Thr Phe Thr Arg
    130                 135                 140

Leu Ala Asn Glu Leu His Leu Lys Ala Ile His Tyr Pro Gly Arg Glu
145                 150                 155                 160

Asp Arg Glu Lys Ala Tyr Glu Thr Gln Glu Ile Asp Pro Phe Lys Tyr
                165                 170                 175

Gln Phe Leu Glu Lys Pro Lys Phe Tyr Gly Ala Glu Leu Tyr Leu Pro
            180                 185                 190

Asp Ile Asp Val Ile Ile Ile Gly Ser Gly Ala Gly Ala Gly Val Val
        195                 200                 205

Ala His Thr Leu Thr Asn Asp Gly Phe Lys Ser Leu Val Leu Glu Lys
    210                 215                 220

Gly Arg Tyr Phe Ser Asn Ser Glu Leu Asn Phe Asp Asp Lys Asp Gly
225                 230                 235                 240

Val Gln Glu Leu Tyr Gln Ser Gly Gly Thr Leu Thr Thr Val Asn Gln
                245                 250                 255

Gln Leu Phe Val Leu Ala Gly Ser Thr Phe Gly Gly Gly Thr Thr Val
            260                 265                 270

Asn Trp Ser Ala Cys Leu Lys Thr Pro Phe Lys Val Arg Lys Glu Trp
        275                 280                 285

Tyr Asp Glu Phe Gly Val Asp Phe Ala Ala Asp Glu Ala Tyr Asp Lys
    290                 295                 300

Ala Gln Asp Tyr Val Trp Gln Gln Met Gly Ala Ser Thr Glu Gly Ile
305                 310                 315                 320

Thr His Ser Leu Ala Asn Glu Ile Ile Ile Glu Gly Gly Lys Lys Leu
                325                 330                 335

Gly Tyr Lys Ala Lys Val Leu Asp Gln Asn Ser Gly Gly His Pro His
            340                 345                 350

His Arg Cys Gly Phe Cys Tyr Leu Gly Cys Lys His Gly Ile Lys Gln
        355                 360                 365

Gly Ser Val Asn Asn Trp Phe Arg Asp Ala Ala Ala His Gly Ser Gln
    370                 375                 380

Phe Met Gln Gln Val Arg Val Leu Gln Ile Leu Asn Lys Lys Gly Ile
385                 390                 395                 400

Ala Tyr Gly Ile Leu Cys Glu Asp Val Val Thr Gly Ala Lys Phe Thr
                405                 410                 415

Ile Thr Gly Pro Lys Lys Phe Val Val Ala Ala Gly Ala Leu Asn Thr
            420                 425                 430

Pro Ser Val Leu Val Asn Ser Gly Phe Lys Asn Lys Asn Ile Gly Lys
        435                 440                 445

Asn Leu Thr Leu His Pro Val Ser Val Val Phe Gly Asp Phe Gly Lys
    450                 455                 460

Asp Val Gln Ala Asp His Phe His Asn Ser Ile Met Thr Ala Leu Cys
465                 470                 475                 480

Ser Glu Ala Ala Asp Leu Asp Gly Lys Gly His Gly Cys Arg Ile Glu
                485                 490                 495

Thr Ile Leu Asn Ala Pro Phe Ile Gln Ala Ser Phe Leu Pro Trp Arg
            500                 505                 510

Gly Ser Asn Glu Ala Arg Arg Asp Leu Leu Arg Tyr Asn Asn Met Val
        515                 520                 525

Ala Met Leu Leu Leu Ser Arg Asp Thr Thr Ser Gly Ser Val Ser Ser
    530                 535                 540

His Pro Thr Lys Pro Glu Ala Leu Val Val Glu Tyr Asp Val Asn Lys
545                 550                 555                 560

Phe Asp Arg Asn Ser Ile Leu Gln Ala Leu Leu Val Thr Ala Asp Leu
                565                 570                 575

Leu Tyr Ile Gln Gly Ala Lys Arg Ile Leu Ser Pro Gln Pro Trp Val
            580                 585                 590

Pro Ile Phe Glu Ser Asp Lys Pro Lys Asp Lys Arg Ser Ile Lys Asp
        595                 600                 605

Glu Asp Tyr Val Glu Trp Arg Ala Lys Val Ala Lys Ile Pro Phe Asp
    610                 615                 620

Thr Tyr Gly Ser Pro Tyr Gly Ser Ala His Gln Met Ser Ser Cys Arg
625                 630                 635                 640

Met Ser Gly Lys Gly Pro Lys Tyr Gly Ala Val Asp Thr Asp Gly Arg
                645                 650                 655

Leu Phe Glu Cys Ser Asn Val Tyr Val Ala Asp Ala Ser Leu Leu Pro
            660                 665                 670

Thr Ala Ser Gly Ala Asn Pro Met Val Thr Thr Met Thr Leu Ala Arg
        675                 680                 685

His Val Ala Leu Gly Leu Ala Asp Ser Leu Lys Thr Lys Ala Lys Leu
    690                 695                 700

<210>  41
<211>  2115
<212>  DNA
<213>  Candida tropicalis

<400>  41
atggctccat ttttgcccga ccaggtcgac tacaaacacg tcgacaccct tatgttatta      60

tgtgacggga tcatccacga aaccaccgtg gacgaaatca aagacgtcat tgcccctgac     120

ttccccgccg acaaatacga ggagtacgtc aggacattca ccaaaccctc cgaaacccca     180

gggttcaggg aaaccgtcta caacaccgtc aacgcaaaca ccatggatgc aatccaccag     240

ttcattatct tgaccaatgt tttgggatca agggtcttgg caccagcttt gaccaactcg     300

ttgactccta tcaaggacat gagcttggaa gaccgtgaaa agttgttagc ctcgtggcgt     360

gactccccta ttgctgctaa aaggaagttg ttcaggttgg tttctacgct taccttggtc     420

acgttcacga gattggccaa tgagttgcat ttgaaagcca ttcattatcc aggaagagaa     480

gaccgtgaaa aggcttatga aacccaggag attgaccctt ttaagtacca gtttttggaa     540

aaaccgaagt tttacggcgc tgagttgtac ttgccagata ttgatgtgat cattattgga 600

tctggggccg gtgctggtgt cgtggcccac actttgacca acgacggctt caagagtttg 660

gttttggaaa agggcagata ctttagcaac tccgagttga actttgatga caaggacggg 720

gttcaagaat tataccaaag tggaggtact ttgaccaccg tcaaccagca gttgtttgtt 780

cttgctggtt ccacttttgg tggtggtacc actgtcaatt ggtcggcctg tcttaaaacg 840

ccattcaagg tgcgtaagga atggtatgat gagtttggcg ttgactttgc tgccgatgaa 900

gcctacgaca aagcacagga ttatgtttgg cagcaaatgg gagcttctac cgaaggcatc 960

acccactctt tggctaacga gattattatt gaaggtggca agaaattagg ttacaaggcc 1020

aaggtattag accaaaacag cggtggtcat cctcatcaca gatgcggttt ctgttatttg 1080

ggttgtaagc acggtatcaa gcagggctct gttaataact ggtttagaga cgcagctgcc 1140

cacggttctc agttcatgca acaggttaga gttttgcaaa tccttaacaa gaagggcatc 1200

gcttatggta tcttgtgtga ggatgttgta accggtgcca agttcaccat tactggcccc 1260

aaaaagtttg ttgttgccgc cggcgcctta aacactccat ctgtgttggt caactccgga 1320

ttcaagaaca agaacatcgg taagaactta actttgcatc cagtttctgt cgtgtttggt 1380

gattttggca aagacgttca agcagatcac ttccacaact ccatcatgac tgctctttgt 1440

tcagaagccg ctgatttaga cggcaagggt catggatgca gaattgaaac catcttgaac 1500

gctccattca tccaggcttc attcttacca tggagaggta gtaacgaggc tagacgagac 1560

ttgttgcgtt acaacaacat ggtggccatg ttacttctta gtcgtgatac caccagtggt 1620

tccgtttcgt cccatccaac taaacctgaa gcattagttg tcgagtacga cgtgaacaag 1680

tttgacagaa actccatctt gcaggcattg ttggtcactg ctgacttgtt gtacattcaa 1740

ggtgccaaga gaatccttag tccccaacca tgggtgccaa tttttgaatc cgacaagcca 1800

aaggataaga gatcaatcaa ggacgaggac tatgtcgaat ggagagccaa ggttgccaag 1860

attccttttg acacctacgg ctcgccttat ggttcggcgc atcaaatgtc ttcttgtcgt 1920

atgtcaggta agggtcctaa atacggtgct gttgataccg atggtagatt gtttgaatgt 1980

tcgaatgttt atgttgctga cgctagtctt ttgccaactg ctagcggtgc taatcctatg 2040

gtcaccacca tgactcttgc aagacatgtt gcgttaggtt tggcagactc cttgaagacc 2100

aaggccaagt tgtag 2115

<210> 42
<211> 470
<212> PRT
<213> Marinobacter aquaeolei VT8

<400> 42

Met Pro Thr Leu Pro Arg Thr Phe Asp Asp Ile Gln Ser Arg Leu Ile
1               5                   10                  15

Asn Ala Thr Ser Arg Val Val Pro Met Gln Arg Gln Ile Gln Gly Leu
            20                  25                  30

Lys Phe Leu Met Ser Ala Lys Arg Lys Thr Phe Gly Pro Arg Arg Pro
        35                  40                  45

Met Pro Glu Phe Val Glu Thr Pro Ile Pro Asp Val Asn Thr Leu Ala
    50                  55                  60

Leu Glu Asp Ile Asp Val Ser Asn Pro Phe Leu Tyr Arg Gln Gly Gln
65                  70                  75                  80

Trp Arg Ala Tyr Phe Lys Arg Leu Arg Asp Glu Ala Pro Val His Tyr
                85                  90                  95

Gln Lys Asn Ser Pro Phe Gly Pro Phe Trp Ser Val Thr Arg Phe Glu
            100                 105                 110

Asp Ile Leu Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Ser Ser Val Gln
145                 150                 155                 160

Gly Val Val Ala Pro Lys Asn Leu Lys Glu Met Glu Gly Leu Ile Arg
                165                 170                 175

Ser Arg Thr Gly Asp Val Leu Asp Ser Leu Pro Thr Asp Lys Pro Phe
            180                 185                 190

Asn Trp Val Pro Ala Val Ser Lys Glu Leu Thr Gly Arg Met Leu Ala
        195                 200                 205

Thr Leu Leu Asp Phe Pro Tyr Glu Glu Arg His Lys Leu Val Glu Trp
    210                 215                 220

Ser Asp Arg Met Ala Gly Ala Ala Ser Ala Thr Gly Gly Glu Phe Ala
225                 230                 235                 240

Asp Glu Asn Ala Met Phe Asp Asp Ala Ala Asp Met Ala Arg Ser Phe
                245                 250                 255

Ser Arg Leu Trp Arg Asp Lys Glu Ala Arg Arg Ala Ala Gly Glu Glu
            260                 265                 270

Pro Gly Phe Asp Leu Ile Ser Leu Leu Gln Ser Asn Lys Glu Thr Lys
        275                 280                 285

Asp Leu Ile Asn Arg Pro Met Glu Phe Ile Gly Asn Leu Thr Leu Leu
    290                 295                 300

Ile Val Gly Gly Asn Asp Thr Thr Arg Asn Ser Met Ser Gly Gly Leu
305                 310                 315                 320

Val Ala Met Asn Glu Phe Pro Arg Glu Phe Glu Lys Leu Lys Ala Lys
                325                 330                 335

Pro Glu Leu Ile Pro Asn Met Val Ser Glu Ile Ile Arg Trp Gln Thr
            340                 345                 350

Pro Leu Ala Tyr Met Arg Arg Ile Ala Lys Gln Asp Val Glu Leu Gly
        355                 360                 365

Gly Gln Thr Ile Lys Lys Gly Asp Arg Val Val Met Trp Tyr Ala Ser
    370                 375                 380

Gly Asn Arg Asp Glu Arg Lys Phe Asp Asn Pro Asp Gln Phe Ile Ile
385                 390                 395                 400

Asp Arg Lys Asp Ala Arg Asn His Met Ser Phe Gly Tyr Gly Val His
                405                 410                 415

Arg Cys Met Gly Asn Arg Leu Ala Glu Leu Gln Leu Arg Ile Leu Trp
            420                 425                 430

Glu Glu Ile Leu Lys Arg Phe Asp Asn Ile Glu Val Val Glu Glu Pro
        435                 440                 445

Glu Arg Val Gln Ser Asn Phe Val Arg Gly Tyr Ser Arg Leu Met Val
    450                 455                 460

Lys Leu Thr Pro Asn Ser
465                 470

<210> 43
<211> 1413
<212> DNA
<213> Marinobacter aquaeolei VT8

<400> 43
atgccaacac tgcccagaac atttgacgac attcagtccc gactgattaa cgccacctcc 60

agggtggtgc cgatgcagag gcaaattcag ggactgaaat tcttaatgag cgccaagagg 120

aagaccttcg gcccacgccg accgatgccc gaattcgttg aaacacccat cccggacgtt 180

aacacgctgg cccttgagga catcgatgtc agcaatccgt ttttataccg gcagggtcag 240

tggcgcgcct atttcaaacg gttgcgtgat gaggcgccgg tccattacca gaagaacagc 300

cctttcggcc ccttctggtc ggtaactcgg tttgaagaca tcctgttcgt ggataagagt 360

cacgacctgt tttccgccga gccgcaaatc attctcggtg accctccgga ggggctgtcg 420

gtggaaatgt tcatagcgat ggatccgccg aaacacgatg tgcagcgcag ctcggtgcag 480

ggagtagtgg caccgaaaaa cctgaaggag atggaggggc tgatccgatc acgcaccggc 540

gatgtgcttg acagcctgcc tacagacaaa ccctttaact gggtacctgc tgtttccaag 600

gaactcacag gccgcatgct ggcgacgctt ctggattttc cttacgagga acgccacaag 660

ctggttgagt ggtcggacag aatggcaggt gcagcatcgg ccaccggcgg ggagtttgcc 720

gatgaaaatg ccatgtttga cgacgcggca gacatggccc ggtctttctc caggctttgg 780

cgggacaagg aggcgcgccg cgcagcaggc gaggagcccg gtttcgattt gatcagcctg 840

ttgcagagca acaaagaaac gaaagacctg atcaatcggc cgatggagtt tatcggtaat 900

ttgacgctgc tcatagtcgg cggcaacgat acgacgcgca actcgatgag tggtggcctg 960

gtggccatga acgaattccc caggaatttt gaaaaattga aggcaaaacc ggagttgatt 1020

ccgaacatgg tgtcggaaat catccgctgg caaacgccgc tggcctatat gcgccgaatc 1080

gccaagcagg atgtcgaact gggcggccag accatcaaga agggtgatcg agttgtcatg 1140

tggtacgcgt cgggtaaccg ggacgagcgc aaatttgaca accccgatca gttcatcatt 1200

gatcgcaagg acgcacgaaa ccacatgtcg ttcggctatg gggttcaccg ttgcatgggc 1260

aaccgtctgg ctgaactgca actgcgcatc ctctgggaag aaatactcaa gcgttttgac 1320

aacatcgaag tcgtcgaaga gcccgagcgg gtgcagtcca acttcgtgcg gggctattcc 1380

aggttgatgg tcaaactgac accgaacagt taa 1413

<210> 44
<211> 497
<212> PRT
<213> Acinetobacter sp. OC4

<400> 44

Met Asn Ser Val Ala Glu Ile Phe Glu Lys Ile Thr Gln Thr Val Thr
1               5                   10                  15

Ser Thr Ala Ala Asp Val Ala Thr Thr Val Thr Asp Lys Val Lys Ser
            20                  25                  30

Asn Glu Gln Phe Gln Thr Gly Lys Gln Phe Leu His Gly Gln Val Thr
        35                  40                  45

Arg Phe Val Pro Leu His Thr Gln Val Arg Gly Ile Gln Trp Met Gln
    50                  55                  60

Lys Ala Lys Phe Arg Val Phe Asn Val Gln Glu Phe Pro Ala Phe Ile
65                  70                  75                  80

Glu Gln Pro Ile Pro Glu Val Ala Thr Leu Ala Leu Ala Glu Ile Asp
                85                  90                  95

Val Ser Asn Pro Phe Leu Tyr Lys Gln Lys Lys Trp Gln Ser Tyr Phe
            100                 105                 110

Lys Arg Leu Arg Asp Glu Ala Pro Val His Tyr Gln Ala Asn Ser Pro
        115                 120                 125

Phe Gly Ala Phe Trp Ser Val Thr Arg Tyr Asp Asp Ile Val Tyr Val
    130                 135                 140

Asp Lys Asn His Glu Ile Phe Ser Ala Glu Pro Val Ile Ala Ile Gly
145                 150                 155                 160

Asn Thr Pro Pro Gly Leu Gly Ala Glu Met Phe Ile Ala Met Asp Pro
                165                 170                 175

Pro Lys His Asp Val Gln Arg Gln Ala Val Gln Asp Val Val Ala Pro
            180                 185                 190

Lys Asn Leu Lys Glu Leu Glu Gly Leu Ile Arg Leu Arg Val Gln Glu
        195                 200                 205

Val Leu Asp Gln Leu Pro Thr Asp Gln Pro Phe Asp Trp Val Gln Asn
    210                 215                 220

Val Ser Ile Glu Leu Thr Ala Arg Met Leu Ala Thr Leu Phe Asp Phe
225                 230                 235                 240

Pro Tyr Glu Lys Arg His Lys Leu Val Glu Trp Ser Asp Leu Met Ala
                245                 250                 255

Gly Thr Ala Glu Ala Thr Gly Gly Thr Val Thr Asn Leu Asp Glu Ile
            260                 265                 270

Phe Asp Ala Ala Val Asp Ala Ala Lys His Phe Ala Glu Leu Trp His
        275                 280                 285

Arg Lys Ala Ala Gln Lys Ser Ala Gly Ala Glu Met Gly Tyr Asp Leu
    290                 295                 300

Ile Ser Leu Met Gln Ser Asn Glu Ala Thr Lys Asp Leu Ile Tyr Arg
305                 310                 315                 320

Pro Met Glu Phe Met Gly Asn Leu Val Leu Leu Ile Val Gly Gly Asn

325                 330                 335

Asp Thr Thr Arg Asn Ser Met Thr Gly Gly Val Tyr Ala Leu Asn Leu
            340                 345                 350

Phe Pro Asn Glu Phe Val Lys Leu Lys Asn Asn Pro Ser Leu Ile Pro
        355                 360                 365

Asn Met Val Ser Glu Ile Ile Arg Trp Gln Thr Pro Leu Ala Tyr Met
    370                 375                 380

Arg Arg Ile Ala Lys Gln Asp Val Glu Leu Asn Gly Gln Thr Ile Lys
385                 390                 395                 400

Lys Gly Asp Lys Val Val Met Trp Tyr Val Ser Gly Asn Arg Asp Glu
                405                 410                 415

Arg Val Ile Glu Arg Pro Asp Glu Leu Ile Ile Asp Arg Lys Gly Ala
            420                 425                 430

Arg Asn His Leu Ser Phe Gly Phe Gly Val His Arg Cys Met Gly Asn
        435                 440                 445

Arg Leu Ala Glu Met Gln Leu Arg Ile Leu Trp Glu Glu Leu Leu Gln
    450                 455                 460

Arg Phe Glu Asn Ile Glu Val Leu Gly Glu Pro Glu Ile Val Gln Ser
465                 470                 475                 480

Asn Phe Val Arg Gly Tyr Ala Lys Met Met Val Lys Leu Thr Ala Lys
                485                 490                 495

Ala

<210>  45
<211>  1494
<212>  DNA
<213>  Acinetobacter sp. OC4

<400>  45
atgaacagcg tagcagaaat cttcgagaaa atcacccaga ccgtcacgag caccgcagcc      60

gacgttgcaa ccactgtcac cgacaaggtc aaatccaacg agcaattcca aacgggtaag     120

cagtttttgc acggccaggt tacgcgtttt gtgccgttgc atacgcaggt ccgtggtatt     180

cagtggatgc agaaagccaa gtttcgcgtg ttcaatgtcc aggagttccc ggcctttatt     240

gaacaaccga ttccggaagt ggcaaccctg gcgctggcgg aaattgatgt cagcaacccg     300

ttcctgtaca aacagaaaaa gtggcaaagc tacttcaaac gtctgcgtga cgaggcgcca     360

gtgcattatc aagctaacag cccgttcggt gccttttggt ctgtgacccg ttacgacgac 420

atcgtttatg ttgacaaaaa tcatgaaatc ttctccgcag aaccggtgat tgctattggt 480

aataccccgc caggcctggg tgcggaaatg ttcatcgcaa tggaccctcc gaagcacgac 540

gtccagcgcc aagcggtgca ggacgtagtt gcgccgaaga acttgaagga actggagggt 600

ctgatccgcc tgcgtgtgca ggaagtcctg gatcagctgc cgacggatca gccgtttgac 660

tgggtgcaga acgtgtcgat tgaactgacc gcacgcatgc tggcgaccct gttcgacttt 720

ccgtatgaaa aacgtcataa actggtggaa tggtccgatc tgatggcagg tacggctgag 780

gccaccggcg gtactgtgac caatctggat gagatttttg atgcggcggt tgacgcagcc 840

aagcacttcg ctgagctgtg gcaccgcaaa gcggctcaaa agagcgccgg tgcggagatg 900

ggctacgatc tgatttccct gatgcaaagc aacgaggcga cgaaagacct gatctatcgc 960

ccgatggagt tcatgggtaa cctggtcctg ctgattgttg gtggcaatga caccacccgt 1020

aatagcatga cgggcggtgt ttatgcactg aacttgtttc cgaatgagtt tgtcaaactg 1080

aagaataacc cgagcctgat ccctaacatg gttagcgaga ttatccgctg gcagacgccg 1140

ctggcttaca tgcgccgcat tgccaagcaa gacgtcgagc tgaacggtca aaccatcaaa 1200

aagggcgata aggtcgtgat gtggtacgtc agcggtaacc gcgacgaacg tgtgattgaa 1260

cgcccggacg aactgatcat tgatcgcaag ggtgcgcgta accacctgtc cttcggcttt 1320

ggcgtgcatc gctgcatggg taatcgtttg gccgagatgc aactgcgtat cctgtgggag 1380

gaactgttgc aacgcttcga gaatatcgaa gttctgggcg aaccggagat tgttcagtcc 1440

aatttcgtgc gtggctatgc gaagatgatg gttaaactga cggcgaaagc ctaa 1494

<210> 46
<211> 558
<212> PRT
<213> Pseudomonas putida

<400> 46

Met Tyr Asp Tyr Ile Ile Val Gly Ala Gly Ser Ala Gly Cys Val Leu
1               5                   10                  15

Ala Asn Arg Leu Ser Ala Asp Pro Ser Lys Arg Val Cys Leu Leu Glu
            20                  25                  30

Ala Gly Pro Arg Asp Thr Asn Pro Leu Ile His Met Pro Leu Gly Ile
        35                  40                  45

Ala Leu Leu Ser Asn Ser Lys Lys Leu Asn Trp Ala Phe Gln Thr Ala
    50                  55                  60

Pro Gln Gln Asn Leu Asn Gly Arg Ser Leu Phe Trp Pro Arg Gly Lys
65                  70                  75                  80

Thr Leu Gly Gly Ser Ser Ser Ile Asn Ala Met Val Tyr Ile Arg Gly
                85                  90                  95

His Glu Asp Asp Tyr His Ala Trp Glu Gln Ala Ala Gly Arg Tyr Trp
            100                 105                 110

Gly Trp Tyr Arg Ala Leu Glu Leu Phe Lys Arg Leu Glu Cys Asn Gln
        115                 120                 125

Arg Phe Asp Lys Ser Glu His His Gly Val Asp Gly Glu Leu Ala Val
    130                 135                 140

Ser Asp Leu Lys Tyr Ile Asn Pro Leu Ser Lys Ala Phe Val Gln Ala
145                 150                 155                 160

Gly Met Glu Ala Asn Ile Asn Phe Asn Gly Asp Phe Asn Gly Glu Tyr
                165                 170                 175

Gln Asp Gly Val Gly Phe Tyr Gln Val Thr Gln Lys Asn Gly Gln Arg
            180                 185                 190

Trp Ser Ser Ala Arg Ala Phe Leu His Gly Val Leu Ser Arg Pro Asn
        195                 200                 205

Leu Asp Ile Ile Thr Asp Ala His Ala Ser Lys Ile Leu Phe Glu Asp
    210                 215                 220

Arg Lys Ala Val Gly Val Ser Tyr Ile Lys Lys Asn Met His His Gln
225                 230                 235                 240

Val Lys Thr Thr Ser Gly Gly Glu Val Leu Leu Ser Leu Gly Ala Val
                245                 250                 255

Gly Thr Pro His Leu Leu Met Leu Ser Gly Val Gly Ala Ala Ala Glu
            260                 265                 270

Leu Lys Glu His Gly Val Ser Leu Val His Asp Leu Pro Glu Val Gly
        275                 280                 285

Lys Asn Leu Gln Asp His Leu Asp Ile Thr Leu Met Cys Ala Ala Asn
    290                 295                 300

Ser Arg Glu Pro Ile Gly Val Ala Leu Ser Phe Ile Pro Arg Gly Val
305                 310                 315                 320

Ser Gly Leu Phe Ser Tyr Val Phe Lys Arg Glu Gly Phe Leu Thr Ser
                325                 330                 335

Asn Val Ala Glu Ser Gly Gly Phe Val Lys Ser Ser Pro Asp Arg Asp
                340                 345                 350

Arg Pro Asn Leu Gln Phe His Phe Leu Pro Thr Tyr Leu Lys Asp His
            355                 360                 365

Gly Arg Lys Ile Ala Gly Gly Tyr Gly Tyr Thr Leu His Ile Cys Asp
    370                 375                 380

Leu Leu Pro Lys Ser Arg Gly Arg Ile Gly Leu Lys Ser Ala Asn Pro
385                 390                 395                 400

Leu Gln Pro Pro Leu Ile Asp Pro Asn Tyr Leu Ser Asp His Glu Asp
                405                 410                 415

Ile Lys Thr Met Ile Ala Gly Ile Lys Ile Gly Arg Ala Ile Leu Gln
                420                 425                 430

Ala Pro Ser Met Ala Lys His Phe Lys His Glu Val Val Pro Gly Gln
            435                 440                 445

Ala Val Lys Thr Asp Asp Glu Ile Ile Glu Asp Ile Arg Arg Arg Ala
    450                 455                 460

Glu Thr Ile Tyr His Pro Val Gly Thr Cys Arg Met Gly Lys Asp Pro
465                 470                 475                 480

Ala Ser Val Val Asp Pro Cys Leu Lys Ile Arg Gly Leu Ala Asn Ile
                485                 490                 495

Arg Val Val Asp Ala Ser Ile Met Pro His Leu Val Ala Gly Asn Thr
                500                 505                 510

Asn Ala Pro Thr Ile Met Ile Ala Glu Asn Ala Ala Glu Ile Ile Met
            515                 520                 525

Arg Asn Leu Asp Val Glu Ala Leu Glu Ala Ser Ala Glu Phe Ala Arg
    530                 535                 540

Glu Gly Ala Glu Leu Glu Leu Ala Met Ile Ala Val Cys Met
545                 550                 555

<210>  47
<211>  1677
<212>  DNA
<213>  Pseudomonas putida

<400>  47
atgtacgact atataatcgt tggtgctgga tctgcaggat gtgtgcttgc taatcgtctt      60

tcggccgacc cctctaaaag agtttgttta cttgaagctg ggccgcgaga tacgaatccg     120

ctaattcaca tgccgttagg tattgctttg ctttcaaata gtaaaaagtt gaattgggct 180

tttcaaactg cgccacagca aaatctcaac ggccggagcc ttttctggcc acgaggaaaa 240

acgttaggtg gttcaagctc aatcaacgca atggtctata tccgagggca tgaagacgat 300

taccacgcat gggagcaggc ggccggccgc tactggggtt ggtaccgggc tcttgagttg 360

ttcaaaaggc ttgaatgcaa ccagcgattc gataagtccg agcaccatgg ggttgacgga 420

gaattagctg ttagtgattt aaaatatatc aatccgctta gcaaagcatt cgtgcaagcc 480

ggcatggagg ccaatattaa tttcaacgga gatttcaacg gcgagtacca ggacggcgta 540

gggttctatc aagtaaccca aaaaaatgga caacgctgga gctcggcgcg tgcattcttg 600

cacggtgtac tttccagacc aaatctagac atcattactg atgcgcatgc atcaaaaatt 660

ctttttgaag accgtaaggc ggttggtgtt tcttatataa agaaaaatat gcaccatcaa 720

gtcaagacaa cgagtggtgg tgaagtactt cttagtcttg gcgcagtcgg cacgcctcac 780

cttctaatgc tttctggtgt tggggctgca gccgagctta aggaacatgg tgtttctcta 840

gtccatgatc ttcctgaggt ggggaaaaat cttcaagatc atttggacat cacattgatg 900

tgcgcagcaa attcgagaga gccgataggt gttgctcttt ctttcatccc tcgtggtgtc 960

tcgggtttgt tttcgtatgt gtttaagcgc gaggggtttc tcactagtaa cgtggcagag 1020

tcgggtggtt ttgtaaaaag ttctcctgat cgtgatcggc ccaatttgca gtttcatttc 1080

cttccaactt atcttaaaga tcacggtcga aaaatagcgg gtggttatgg ttatacgcta 1140

catatatgtg atcttttgcc taagagccga ggcagaattg gcctaaaaag cgccaatcca 1200

ttacagccgc ctttaattga cccgaactat cttagcgatc atgaagatat taaaaccatg 1260

attgcgggta ttaagatagg gcgcgctatt ttgcaggccc catcgatggc gaagcatttt 1320

aagcatgaag tagtaccggg ccaggctgtt aaaactgatg atgaaataat cgaagatatt 1380

cgtaggcgag ctgagactat ataccatccg gtaggtactt gtaggatggg taaagatcca 1440

gcttcagttg ttgatccgtg cctgaagatc cgtgggttgg caaatattag agtcgttgat 1500

gcgtcaatta tgccgcactt ggtcgcgggt aacacaaacg ctccaactat tatgattgca 1560

gaaaatgcgg cagaaataat tatgcggaat cttgatgtgg aagcattaga ggctagcgct 1620

gagtttgctc gcgagggtgc agagctagag ttggccatga tagctgtctg catgtaa 1677

<210> 48
<211> 452
<212> PRT
<213> Pseudomonas putida

<400> 48

Met Ser Glu Gln Asn Ser Gln Thr Leu Ala Trp Gln Ser Met Ser Arg
1               5                   10                  15

Asp His His Leu Ala Pro Phe Ser Asp Val Lys Gln Leu Ala Glu Lys
            20                  25                  30

Gly Pro Arg Ile Ile Thr Ser Ala Lys Gly Val Tyr Leu Trp Asp Ser
        35                  40                  45

Glu Gly Asn Lys Ile Leu Asp Gly Met Ala Gly Leu Trp Cys Val Ala
    50                  55                  60

Val Gly Tyr Gly Arg Asp Glu Leu Ala Glu Val Ala Ser Gln Gln Met
65                  70                  75                  80

Lys Gln Leu Pro Tyr Tyr Asn Leu Phe Phe Gln Thr Ala His Pro Pro
                85                  90                  95

Ala Leu Glu Leu Ala Lys Ala Ile Ala Asp Val Ala Pro Gln Gly Met
            100                 105                 110

Asn His Val Phe Phe Thr Gly Ser Gly Ser Glu Gly Asn Asp Thr Val
        115                 120                 125

Leu Arg Met Val Arg His Tyr Trp Ala Leu Lys Gly Lys Lys Asn Lys
    130                 135                 140

Asn Val Ile Ile Gly Arg Ile Asn Gly Tyr His Gly Ser Thr Val Ala
145                 150                 155                 160

Gly Ala Ala Leu Gly Gly Met Ser Gly Met His Gln Gln Gly Gly Val
                165                 170                 175

Ile Pro Asp Ile Val His Ile Pro Gln Pro Tyr Trp Phe Gly Glu Gly
            180                 185                 190

Gly Asp Met Thr Glu Ala Asp Phe Gly Val Trp Ala Ala Glu Gln Leu
        195                 200                 205

Glu Lys Lys Ile Leu Glu Val Gly Val Asp Asn Val Ala Ala Phe Ile
    210                 215                 220

Ala Glu Pro Ile Gln Gly Ala Gly Gly Val Ile Ile Pro Pro Gln Thr
225                 230                 235                 240

Tyr Trp Pro Lys Val Lys Glu Ile Leu Ala Arg Tyr Asp Ile Leu Phe
                245                 250                 255

Val Ala Asp Glu Val Ile Cys Gly Phe Gly Arg Thr Gly Glu Trp Phe
            260                 265                 270

Gly Thr Asp Tyr Tyr Asp Leu Lys Pro Asp Leu Met Thr Ile Ala Lys

275                 280                 285

Gly Leu Thr Ser Gly Tyr Ile Pro Met Gly Gly Val Ile Val Arg Asp
    290                 295                 300

Glu Val Ala Lys Val Ile Ser Glu Gly Gly Asp Phe Asn His Gly Phe
305                 310                 315                 320

Thr Tyr Ser Gly His Pro Val Ala Ala Ala Val Gly Leu Glu Asn Leu
                325                 330                 335

Arg Ile Leu Arg Asp Glu Gln Ile Ile Gln Gln Val His Asp Lys Thr
            340                 345                 350

Ala Pro Tyr Leu Gln Gln Arg Leu Arg Glu Leu Ala Asp His Pro Leu
        355                 360                 365

Val Gly Glu Val Arg Gly Leu Gly Met Leu Gly Ala Ile Glu Leu Val
    370                 375                 380

Lys Asp Lys Ala Thr Arg Ala Arg Tyr Glu Gly Lys Gly Val Gly Met
385                 390                 395                 400

Ile Cys Arg Gln His Cys Phe Asp Asn Gly Leu Ile Met Arg Ala Val
                405                 410                 415

Gly Asp Thr Met Ile Ile Ala Pro Pro Leu Val Ile Ser Ile Glu Glu
            420                 425                 430

Ile Asp Glu Leu Val Glu Lys Ala Arg Lys Cys Leu Asp Leu Thr Tyr
        435                 440                 445

Glu Ala Val Arg
    450

<210>  49
<211>  1359
<212>  DNA
<213>  Pseudomonas putida

<400>  49
atgagcgaac aaaactccca aaccctggcc tggcagagca tgagccgcga tcaccatctg      60

gcgccgttca gcgatgtcaa gcaattggct gaaaaaggtc cgcgcattat cacgtccgcg     120

aaaggcgtgt acctgtggga ttccgaaggt aacaaaatcc tggatggtat ggcgggtctg     180

tggtgtgtgg cggtcggtta cggtcgtgat gaactggctg aggtcgccag ccaacaaatg     240

aagcaactgc cgtactacaa cctgttcttc caaacggccc atccgccagc actggagctg     300

gcaaaagcca ttgccgacgt agctccgcaa ggtatgaacc atgttttctt caccggttct     360

ggcagcgaag gcaatgatac cgtgctgcgt atggtacgcc actattgggc cctgaaaggt 420

aagaagaaca agaatgtcat cattggtcgt atcaacggtt accacggtag caccgtcgct 480

ggtgcggcgc tgggtggcat gagcggcatg caccagcagg gtggtgtcat tccggacatt 540

gtccacattc cgcagccgta ttggtttggt gaaggcggtg acatgacgga agcggacttc 600

ggtgtgtggg ctgccgagca attggagaag aagatcctgg aggttggcgt tgataatgtt 660

gcggcgttca ttgcggaacc gatccaaggc gctggcggtg ttatcatccc gccacagacg 720

tattggccaa aggtgaaaga gatcctggcg cgctacgata tcttgttcgt tgctgacgag 780

gtgatctgcg gttttggtcg caccggcgaa tggtttggca ccgattacta cgacctgaaa 840

ccggatctga tgaccatcgc caaaggtctg accagcggct acattccgat gggtggtgtc 900

attgtccgtg atgaggttgc caaggttatc agcgagggcg gtgactttaa ccacggcttt 960

acctacagcg gccatccggt ggcggcagcg gtcggtctgg aaaatctgcg tattctgcgt 1020

gacgagcaga tcatccaaca agttcacgac aagaccgctc cgtatctgca acagcgcctg 1080

cgcgaactgg cggaccatcc gctggttggc gaagtccgtg gcttgggcat gctgggtgcg 1140

attgaactgg ttaaagacaa ggcaacccgt gcacgttatg agggtaaggg cgtgggcatg 1200

atctgccgtc agcactgttt cgacaacggt ctgattatgc gtgcggttgg cgataccatg 1260

attatcgcac cgccgttggt catcagcatc gaagaaatcg acgaattggt agagaaagcg 1320

cgtaagtgtc tggatctgac gtatgaggct gtgcgctaa 1359

## Beschreibung

Die Erfindung betrifft ein Verfahren zur Oxidation einer Fettsäure oder eines Esters davon, umfassend den Schritt Oxidieren der Fettsäure oder des Esters davon durch Kontaktieren mit einer Cytochrom P450-Monooxygenase der CYP153-Familie in Anwesenheit von molekularem Sauerstoff und NAD(P)H und einem Ganzzellkatalysator exprimierend eine rekombinante Cytochrom P450-Monooxygenase der CYP153-Familie, eine rekombinante Alkoholdehydrogenase, eine rekombinante Transaminase und optional ein oder mehr als ein rekombinantes Enzym aus der Gruppe umfassend Alanindehydrogenase, Ferredoxin und Ferredoxin-Reduktase sowie die Verwendung dieses Ganzzellkatalysators zur Oxidation einer Fettsäure oder eines Esters davon,
wobei die Fettsäure oder der Esters davon bevorzugt die Formel (I)

H₃C - (CH₂)ₙ - COOR (I),

wobei R aus der Gruppe ausgewählt ist, die H, Methyl, Ethyl, Propyl und Butyl umfasst,
wobei n 0 bis 30, bevorzugt 6 bis 24 ist,
aufweist.

Polyamide sind eine Klasse von Polymeren, die durch sich wiederholende Amidgruppen gekennzeichnet sind. Der Begriff "Polyamid" betrifft im Unterschied zu den chemisch verwandten Proteinen gewöhnlich synthetische, im Handel erhältliche, thermoplastische Kunststoffe. Polyamide werden von primären Aminen oder von sekundären Aminen abgeleitet, die herkömmlich beim Cracken von Kohlenwasserstoffen gewonnen werden. Es können jedoch auch Derivate, genauer Aminocarbonsäure, Lactame und Diamine, zur Polymerherstellung verwendet werden. Von Interesse sind weiterhin kurzkettige, gasförmige Alkane als Edukte, die ausgehend von nachwachsenden Rohstoffen mit biotechnologischen Verfahren gewonnen werden können.

Viele kommerziell stark nachgefragte Polyamide werden ausgehend von Lactamen hergestellt. Zum Beispiel kann man "Polyamid 6" durch Polymerisation von ε-Caprolactam und "Polyamid 12" durch Polymerisierung von Laurinlactam erhalten. Weitere kommerziell interessante Produkte umfassen Copolymere von Lactam, z. B. Copolymere von ε-Caprolactam und Laurinlactam.

Die konventionelle chemisch-technische Erzeugung von Aminen ist von der Versorgung mit fossilen Rohstoffen abhängig, ineffizient, und dabei fallen große Mengen unerwünschter Nebenprodukte an, in manchen Schritten der Synthese bis zu 80 %. Ein Beispiel für einen solchen Prozess stellt die Herstellung von Laurinlactam dar, das konventionell durch Trimerisierung von Butadien gewonnen wird. Das Trimerisierungsprodukt Cyclododekatrien wird hydriert und das daraus resultierende Cyclododekan wird zu Cyclododekanon oxidiert, das anschließend mit Hydroxylamin zu Cyclododekanoxim umgesetzt wird, welche schließlich über eine Beckmann-Umlagerung zu Laurinlactam umgewandelt wird.

Angesichts dieser Nachteile wurden Verfahren entwickelt, um Amine unter Verwendung von Biokatalysatoren ausgehend von nachwachsenden Rohstoffen zu gewinnen. Als nachwachsende Rohstoffe kommen insbesondere Quellen für Fettsäuren in Frage, die in Form von Rapsöl, Kugeldistelöl, Palmkernöl, Kokosnussöl, Sonnenblumenkernöl und ähnlichen Naturprodukten aus einer Vielzahl von biologischen Quellen, insbesondere aus Pflanzen, gewonnen werden können.

PCT/EP 2008/067447 beschreibt ein biologisches System zur Herstellung chemisch verwandter Produkte, genauer ω-Aminocarbonsäuren, unter Verwendung einer Zelle, die eine Reihe von geeigneten enzymatischen Aktivitäten aufweist und in der Lage ist, Carbonsäuren zu entsprechender ω-Aminocarbonsäure umzuwandeln.

Ein bekannter Nachteil des dabei verwendeten AlkBGT-Oxidasesystems aus *Pseudomonas putida* GPO1 besteht jedoch darin, dass es nicht in der Lage ist, eine selektive Oxidation von aliphatischen Alkanen zu primären Alkoholen zu leisten. Vielmehr treten eine Vielzahl von Oxidationsprodukten auf; insbesondere erhöht sich der Anteil an höher oxidierten Produkten wie dem entsprechenden Aldehyd, Keton oder der entsprechenden Carbonsäure mit zunehmender Reaktionsdauer (C. Grant, J. M. Woodley and F. Baganz (2011), Enzyme and Microbial Technology 48, 480-486), was die Ausbeute an erwünschtem Amin entsprechend verringert.

Das Problem der relativ unselektiven Oxidation ist dadurch verschärft, dass die entstehenden Oxidationsprodukte strukturell sehr ähnlich sind. Dies bedingt, dass es sehr schwierig ist, sie von den erwünschten Oxidationsprodukten effizient und ohne signifikanten Ausbeuteverlust abzutrennen.

Es besteht somit Bedarf an Verfahren, bei denen die enzymatisch katalysierten Reaktionen selektiver verlaufen und die Bildung irreversibel hergestellter Nebenprodukte minimiert ist.

Vor diesem Hintergrund besteht die der Erfindung zu Grunde liegende Aufgabe darin, ein verbessertes Verfahren zur Oxidation und Aminierung von Fettsäuren unter Verwendung von Biokatalysatoren bereitzustellen.

Eine weitere der Erfindung zu Grunde liegende Aufgabe besteht darin, das Verfahren dahingehend zu verbessern, dass die Ausbeute, bezogen auf die Menge des Fettsäuresubstrates oder sonstiger Substrate, die Menge des Kohlenstoffsubstrates für zur biotechnologischen Synthese verwendete Zellen erhöht und/oder die Konzentration an Nebenprodukten oder das Verhältnis von Nebenprodukten zum erwünscht Produkt verringert wird.

Eine weitere der Erfindung zu Grunde liegende Aufgabe besteht darin, das Verfahren dahingehend zu verbessern, dass die Selektivität der eingesetzten Biokatalysatoren, insbesondere von Fettsäureoxidasen, erhöht und/oder verlängert wird, entweder zu Beginn der Reaktion, d.h. vor Erreichen eines Plateaus bei der Produktkonzentration über die Zeit, oder nach Einstellung des Gleichgewichtes, d.h. nach Erreichen des Plateaus.

Eine weitere der Erfindung zu Grunde liegende Aufgabe besteht, darin, die Aufarbeitbarkeit des bei der biotechnologischen Oxidation und/Aminierung von Fettsäuren entstehenden Reaktionsgemisches zu verbessern, insbesondere mit Hinblick auf die Effizienz und Geschwindigkeit der Phasentrennung von hydrophilen und hydrophoben Stoffen.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Die der Erfindung zu Grunde liegende Aufgabe wird in einem ersten Aspekt gelöst durch ein Verfahren zur Oxidation einer Fettsäure oder eines Esters davon
umfassend den Schritt:
a) Oxidieren der Fettsäure oder des Esters davon durch Kontaktieren mit einer Cytochrom P450-Monooxygenase der CYP153-Familie in Anwesenheit von molekularem Sauerstoff und NAD(P)H,
   wobei die Fettsäure oder der Esters davon bevorzugt die Formel (I)

   H₃C - (CH₂)ₙ - COOR (I),

   wobei R aus der Gruppe ausgewählt ist, die H, Methyl, Ethyl, Propyl und Butyl umfasst,
   wobei n 0 bis 30, bevorzugt 6 bis 24 ist,
   aufweist.

In einer ersten Ausführungsform des ersten Aspekts wird die Aufgabe gelöst durch ein Verfahren weiter umfassend die Schritte:
b) Weiteres Oxidieren der oxidierten Fettsäure oder des Esters davon aus Schritt a) durch Kontaktieren mit einer Alkoholdehydrogenase,
c) Aminieren der weiter oxidierten Fettsäure oder des Esters davon aus Schritt b) durch Kontaktieren mit einer Transaminase in Anwesenheit eines Amindonors, bevorzugt Alanin,
wobei Schritt c) optional in Anwesenheit einer Alanindehydrogenase, von Ammonium und NAD(P)H erfolgt

In einer zweiten Ausführungsform, bei der es sich auch um eine Ausführungsform der ersten Ausführungsform handelt, wird die Aufgabe gelöst durch ein Verfahren, wobei die Cytochrom P450-Monooxygenase der CYP153-Familie die Peptidsequenz LL(I/L)(V/I)GGNDTTRN aufweist und es sich bevorzugt um die Cytochrom P450-Monooxygenase der CYP153-Familie aus *Alcanivorax borkumensis* SK2 (Datenbankcode YP_691921) oder eine Variante davon handelt.

Diese und sämtliche weitere in diesem Dokument verwendeten Datenbankcodes stammen aus der Genbank Protein-Datenbank des NCBI in dem am 9. März 2012 online verfügbaren Release.

In einer dritten Ausführungsform, bei der es sich auch um eine Ausführungsform der ersten bis zweiten Ausführungsform handelt, wird die Aufgabe gelöst durch ein Verfahren, wobei es sich bei der Alkoholdehydrogenase um eine NAD(P)⁺-abhängige Alkoholdehydrogenase, bevorzugt um die NAD-abhängige Alkoholdehydrogenase aus *Escherichia coli* MS 187-1 (Datenbankcode ZP_07145023) oder eine Variante davon oder die Alkoholdehydrogenase von *Bacillus stearothermophilus* (Datenbankcode P42328) oder eine Variante davon, oder um eine Oxidoreduktase der Glucose-Methanol-Cholin-Oxidoreduktase-Familie, bevorzugt die aus *Pseudomonas putida* (Datenbankcode CAB54054.1) oder eine Variante davon, oder um eine flavinhaltige Alkoholdehydrogenase, bevorzugt die flavinhaltige Alkoholdehydrogenase aus *Candida tropicalis* (Datenbankcode AAS46878.1) oder eine Variante davon, handelt.

In einer vierten Ausführungsform, bei der es sich auch um eine Ausführungsform der ersten bis dritten Ausführungsform handelt, wird die Aufgabe gelöst durch ein Verfahren, wobei in Schritt a) zusätzlich eine Ferredoxin-Reduktase, bevorzugt die Ferredoxin-Reduktase aus *Alcanivorax borkumensis* SK2 (Datenbankcode YP_691923) oder eine Variante davon, und/oder ein Ferredoxin, bevorzugt das Ferredoxin aus *Alcanivorax borkumensis* SK2 (Datenbankcode YP_691920) oder eine Variante davon, anwesend ist.

In einer fünften Ausführungsform, bei der es sich auch um eine Ausführungsform der ersten bis vierten Ausführungsform handelt, wird die Aufgabe gelöst durch ein Verfahren, wobei Schritt c) in Anwesenheit einer Alanindehydrogenase, Ammonium und NADH erfolgt und es sich bei der Alanindehydrogenase um die Alanindehydrogenase aus *Bacillus subtilis subsp. subtilis* str. 168 (Datenbankcode NP_391071) oder eine Variante davon handelt.

In einer sechsten Ausführungsform, bei der es sich auch um eine Ausführungsform der ersten bis fünften Ausführungsform handelt, wird die Aufgabe gelöst durch ein Verfahren, wobei wenigstens ein Enzym aus der Gruppe umfassend Cytochrom P450-Monooxygenase der CYP153-Familie, Alkoholdehydrogenase, Transaminase, Alanindehydrogenase, Ferredoxin und Ferredoxin-Reduktase rekombinant in Form von einem Ganzzellkatalysator bereitgestellt wird.

In einer siebten Ausführungsform, bei der es sich auch um eine Ausführungsform der sechsten Ausführungsform handelt, wird die Aufgabe gelöst durch ein Verfahren, wobei sämtliche in wenigstens einem der Schritte a), b) oder c) mit der Fettsäure oder dem Ester davon, der weiter oxidierten Fettsäure oder dem Ester davon aus Schritt b) oder der aminierten weiter oxidierten Fettsäure oder dem Ester davon aus Schritt c) kontaktierten oder anwesenden Enzyme aus der Gruppe umfassend Cytochrom P450-Monooxygenase der CYP153-Familie, Alkoholdehydrogenase, Transaminase, Alanindehydrogenase, Ferredoxin und Ferredoxin-Reduktase rekombinant in Form eines oder mehr als eines Ganzzellkatalysators bereitgestellt werden.

In einer achten Ausführungsform, bei der es sich auch um eine Ausführungsform der sechsten bis siebten Ausführungsform handelt, wird die Aufgabe gelöst durch ein Verfahren, wobei der Ganzzellkatalysator zusätzlich ein Polypeptid der AlkL-Familie, bevorzugt ein AlkL aus der Gruppe umfassend AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas* oleovorans (Datenbankcode Q00595) oder eine Variante davon exprimiert.

In einer neunten Ausführungsform, bei der es sich auch um eine Ausführungsform der ersten bis achten Ausführungsform handelt, wird die Aufgabe gelöst durch ein Verfahren, wobei es sich bei dem Ganzzellkatalysator um eine Zelle handelt, die eine ihrem Wildtyp gegenüber verringerte Aktivität wenigstens eines Enzyms aufweist, das eine der Reaktionen der β-Oxidation von Fettsäuren katalysiert, wobei das Enzym bevorzugt aus der Gruppe ausgewählt ist, die Fettsäureimporter, Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase umfasst.

In einer zehnten Ausführungsform, bei der es sich auch um eine Ausführungsform der ersten bis neunten Ausführungsform handelt, wird die Aufgabe gelöst durch ein Verfahren, wobei die Alanindehydrogenase in Schritt c) so ausgewählt ist, dass sie den von der Alkoholdehydrogenase in Schritt b) oxidierten Redoxcofaktor, bevorzugt NAD⁺ oder NADP⁺, reduziert.

In einem zweiten Aspekt wird die Aufgabe gelöst durch einen Ganzzellkatalysator exprimierend eine rekombinante Cytochrom P450-Monooxygenase der CYP153-Familie, eine rekombinante Alkoholdehydrogenase, eine rekombinante Transaminase und optional ein oder mehr als ein rekombinantes Enzym aus der Gruppe umfassend Alanindehydrogenase, Ferredoxin und Ferredoxin-Reduktase.

In einer ersten Ausführungsform des ersten Aspekts wird die Aufgabe gelöst durch einen Ganzzellkatalysator, wobei der Ganzzellkatalysator zusätzlich ein Polypeptid der AlkL-Familie, bevorzugt ein AlkL aus der Gruppe umfassend AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) oder eine Variante davon exprimiert.

In einer zweiten Ausführungsform, bei der es sich auch um eine Ausführungsform der ersten Ausführungsform handelt, wird die Aufgabe gelöst durch einen Ganzzellkatalysator, wobei es sich bei dem Ganzzellkatalysator um eine Zelle handelt, die eine ihrem Wildtyp gegenüber verringerte Aktivität wenigstens eines Enzyms aufweist, das eine der Reaktionen der β-Oxidation von Fettsäuren katalysiert, wobei das Enzym bevorzugt aus der Gruppe ausgewählt ist, die Fettsäureimporter, Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase umfasst.

In einer dritten Ausführungsform, bei der es sich auch um eine Ausführungsform der ersten bis zweiten Ausführungsform handelt, wird die Aufgabe gelöst durch einen Ganzzellkatalysator, wobei der Ganzzellkatalysator eine Ferredoxin-Reduktase und ein Ferredoxin exprimiert.

In einer vierten Ausführungsform, bei der es sich auch um eine Ausführungsform der ersten bis dritten Ausführungsform handelt, wird die Aufgabe gelöst durch einen Ganzzellkatalysator, wobei der Ganzzellkatalysator eine Alanindehydrogenase exprimiert, wobei es sich um die Alanindehydrogenase aus *Bacillus subtilis subsp. subtilis* str. 168 (Datenbankcode NP_391071) oder eine Variante davon handelt.

In einer fünften Ausführungsform, bei der es sich auch um eine Ausführungsform der ersten bis vierten Ausführungsform handelt, wird die Aufgabe gelöst durch einen Ganzzellkatalysator, wobei das Cytochrom P450-Monooxygenase der CYP153-Familie die Peptidsequenz LL(I/L)(V/I)GGNDTTRN aufweist und/oder es sich um die Cytochrom P450-Monooxygenase der CYP153-Familie aus *Alcanivorax borkumensis* SK2 (Datenbankcode YP_691921) oder eine Variante davon handelt, und es sich bei der Ferredoxin-Reduktase um die Ferredoxin-Reduktase aus *Alcanivorax borkumensis* SK2 (Datenbankcode YP_691923) oder eine Variante davon handelt und es sich bei dem Ferredoxin um das Ferredoxin aus *Alcanivorax borkumensis* SK2 (Datenbankcode YP_691920) oder eine Variante davon handelt.

In einer sechsten Ausführungsform, bei der es sich auch um eine Ausführungsform der ersten bis fünften Ausführungsform handelt, wird die Aufgabe gelöst durch einen Ganzzellkatalysator, wobei es sich bei der Alkoholdehydrogenase um eine NAD(P)⁺-abhängige Alkoholdehydrogenase, um eine Oxidoreduktase der Glucose-Methanol-Cholin-Oxidoreduktase-Familie oder um eine flavinhaltige Alkoholoxidase handelt, bevorzugt um eine NAD(P)⁺-abhängige Alkoholdehydrogenase, am bevorzugtesten um die NAD-abhängige Alkoholdehydrogenase aus *Escherichia coli* MS 187-1 (Datenbankcode ZP_07145023) oder eine Variante davon handelt.

In einer siebten Ausführungsform, die auch eine weitere Ausführungsform des ersten oder zweiten Aspektes und deren Ausführungsformen darstellt, wird die Aufgabe gelöst durch einen Ganzzellkatalysator nach dem zweiten Aspekt oder einer Ausführungsform des zweiten Aspektes oder Verfahren nach dem ersten Aspekt oder einer Ausführungsform des ersten Aspektes, wobei es sich bei der Transaminase um die Transaminase aus *Pseudomonas putida* GB-1 (Datenbankcode YP_001668026.1) oder eine Variante davon handelt.

In einer achten Ausführungsform, die auch eine weitere Ausführungsform des ersten oder zweiten Aspekts darstellt, handelt es sich bei der Fettsäureoder den Ester davon um eine ungesättigte oder verzweigte Fettsäureoder Ester davon.

In einer neunten Ausführungsform, die auch eine weitere Ausführungsform des ersten oder zweiten Aspekts darstellt, wird die Aufgabe gelöst durch ein Verfahren, wobei wenigstens ein Enzym aus der Gruppe umfassend Cytochrom P450-Monooxygenase der CYP153-Familie, Alkoholdehydrogenase, Transaminase, Alanindehydrogenase, Ferredoxin und Ferredoxin-Reduktase rekombinant in Form von einem Ganzzellkatalysator bereitgestellt wird, oder wird die Aufgabe gelöst durch einen Ganzzellkatalysator, wobei es sich bei dem Ganzzellkatalysator um eine Zelle handelt, die eine relativ zum Wildtyp der Zelle eine verringerte Aktivität wenigstens einer endogenen Aldehyddehydrogenase aufweist.

In einem dritten Aspekt wird die der Erfindung zu Grunde liegende Aufgabe gelöst durch die Verwendung des Ganzzellkatalysators nach dem zweiten Aspekt der Erfindung oder einer ihrer Ausführungsformen zur Oxidation und/oder Aminierung einer Fettsäure oder eines Esters davon, wobei die Fettsäure oder der Ester davon bevorzugt die Formel (I)

H₃C - (CH₂)ₙ - COOR (I),

wobei R aus der Gruppe ausgewählt ist, die H, Methyl, Ethyl, Propyl und Butyl umfasst,
wobei n 0 bis 30, bevorzugt 6 bis 24 ist,
aufweist

In einer ersten Ausführungsform des dritten Aspekts wird das Problem gelöst durch eine Verwendung, wobei die Oxidation eine Mischung von Oxidationsprodukten ergibt, die, bezogen auf die Stoffmenge der umgesetzten Fettsäure oder des Esters davon, wenigstens 90 % des entsprechenden Alkohols, weniger als 1 % des entsprechenden Aldehydes und weniger als 10 % der entsprechenden Säure umfasst.

In einer zweiten Ausführungsform des dritten Aspekts, die auch eine weitere Ausführungsform der Ausführungsformen des dritten Aspekts darstellt, handelt es sich bei der Fettsäure um eine ungesättigte oder verzweigte Fettsäure oder Ester davon.

Die vorliegende Erfindung beruht auf der Erkenntnis der Erfinder, dass die Verwendung spezifischer Monooxygenasen oder von Ganzzellkatalysatoren exprimierend solche Monooxygenasen, genauer Cytochrom P450-Monooxygenase der CYP153-Familie, überraschend zur Oxidation und/oder Aminierung von Fettsäuren zur Bildung der erwünschten Produkte mit höherer Selektivität und besserer relativer Ausbeute führt.

Ohne an irgendeine Theorie gebunden sein zu wollen, vermuten die Erfinder, dass das aktive katalytische Zentrum dieser Monooxygenasen derart beschaffen ist, dass bereits hydroxylierte Produkte, nicht erst zum Aldehyd oder gar zur Säure oxidierte Produkte, mit geringerer Affinität gebunden und weiter oxidiert werden als es bei vergleichbaren, aus dem Stand der Technik bekannten Monooxygenasen der Fall ist.

Das erfindungsgemäße Verfahren sieht in Schritt a) die Oxidation von Fettsäuren durch eine Cytochrom P450-Monooxygenase der CYP153-Familie vor. In einer Ausführungsform wird unter dem Begriff "Fettsäure oder Ester davon" eine Verbindung der Formel H₃C-(CH₂)ₓ-COOR, wobei x 4, 5, 6, 7, 8, 9, 10, 11, 12, 13, 14, 15, 16, 17, 18, 19, 20, 21, 22, 23, 24, 25, 26, 27, 28, 29 oder 30 und R Wasserstoff, Methyl, Ethyl oder Propyl, bevorzugt Wasserstoff ist. In einer besonders bevorzugten Ausführungsform ist x 6, 7, 8, 9, 10, 11, 12, 13, 14, 15, 16, 17, 18, 19, 20, 21, 22, 23 oder 24 und R Wasserstoff. In einer bevorzugtesten Ausführungsform handelt es sich dabei um Laurinsäure oder Laurinsäuremethylester. In einer bevorzugten Ausführungsform handelt es sich um eine ungesättigte Fettsäure aus der Gruppe umfassend Myristoleinsäure, Palmitoleinsäure, Petroselinsäure, Ölsäure, Elaidinsäure, Vaccensäure, Gadoleinsäure, Icosensäure oder Erucasäure. Ebenso möglich sind Mischungen verschiedener Fettsäuren, beispielweise durch Hydrolyse und ggf. Veresterung von Kugeldistelöl, Kokosöl, Cuphea-Öl oder Palmkernöl gewonnene Mischungen von Fettsäuren oder Fettsäureestern,. Da nicht alle Fettsäuren in nennenswertem Maß bei Raumtemperatur löslich sind, kann es notwendig sein, weitere Maßnahmen zu ergreifen, wie beispielsweise die Erhöhung der Temperatur oder, bevorzugterweise, die Zugabe eines organischen Lösungsmittels, um sie der wässrigen Phase zugänglich zu machen. In einer besonders bevorzugten Ausführungsform findet eine Fettsäure oder ein Ester davon, am bevorzugtesten Laurinsäuremethylester oder Ölsäure, als ein solches weiteres Lösungsmittel Verwendung.

Wie bei allen in dieser Anmeldung genannten Verbindungen umfassen Fettsäuren nicht nur die protonierte Form der Fettsäure, sondern auch sämtliche in wässriger Lösung dissoziierte Formen, Formulierungen oder Salze. Beispielsweise umfasst der Begriff Laurinsäure auch Laureat oder Natriumlaureat. Als weiteres Beispiel umfasst die Aminosäure Alanin die in Wasser an der Carboxylgruppe deprotonierten oder protonierten und die an der Aminogruppe protonierten oder deprotonierten Formen.

Das erfindungsgemäße Verfahren sieht nicht nur die Oxidation zur hydroxylierten Fettsäure vor, sondern ermöglicht die effiziente Umsetzung von Fettsäuren zur entsprechenden ω-Aminocarbonsäure mittels eines Enzymsystems umfassend eine Cytochrom P450-Monooxygenase der CYP153-Familie, eine Alkoholdehydrogenase, eine Transaminase und optional eine Aminosäuredehydrogenase. Die Verwendung derartiger Enzyme erfolgt unter Bedingungen, die mit deren enzymatischer Aktivität kompatibel sind. Dazu gehört zunächst die Auswahl eines geeigneten wässrigen Puffersystems umfassend wenigstens einen pHstabilisierenden Puffer, beispielsweise Natriumphosphat, optional zusätzlich wenigstens ein Salz, beispielsweise Natriumchlorid, bei einem geeigneten pH-Wert. In einer bevorzugtesten Ausführungsform beträgt der pH-Wert 5, 5,5, 6, 6,5, 7, 7, 5, 8, 8,5, 9 oder 10, bevorzugt 2,5 bis 7,5 besonders bevorzugt 5,5 bis 7,5. Auch die Temperatur muss auf die Aktivität des zu verwendenden Enzyms oder der zu verwendenden Enzyme abgestimmt sein. In einer bevorzugten Ausführungsform beträgt die Temperatur 1 bis 45, noch bevorzugter 20 bis 45, am bevorzugtesten 28 bis 42 °C. Die Auswahl eines geeigneten Puffersystems und die Stabilisierung der Aktivität kann der Fachmann anhand von Standardverfahren, siehe beispielsweise A Cornish-Bowden (1995), Fundamentals of Enzym Kinetics, Portland Press Limited, 1995, vornehmen. Die Aktivität der Cytochrom P450-Monooxygenase der CYP153-Familie, ohne oder in Kombination mit Ferredoxin und Ferredoxin-Reduktase, kann mittels des von Scheps, D., Malca, H., Hoffmann, B., Nestl, B. M, und Hauer, B. (2011) Org. Biomol. Chem., 9, 6727 beschriebenen Assays bestimmt werden. Ob Ferredoxin und/oder Ferredoxin-Reduktase aktiv sind, erkennt der Fachmann durch eine erhebliche Aktivitätssteigerung der Monooxygenase in Anwesenheit der beiden aktiven Enzyme gegenüber deren Abwesenheit.

Ein Enzymtest für die Aktivität von Transaminasen wird von Cayman Chemical Company, Ann Arbor, MI, vertrieben ("Alanine Transaminase Activity Assay Kit, Item No. 700260"). Die Aktivität einer Aminosäuredehydrogenase lässt sich nach Germano, H. J., und Anderson, K. E. (1968), J. Bact. 96 (1), Seite 55-60, bestimmen.

Die Verwendung von Enzymen erfordert weiterhin die Anwesenheit aller notwendigen Substrate. So ist für die Aktivität der Cytochrom P450-Monooxygenase der CYP153-Familie neben der erfindungsgemäß umzusetzenden Fettsäure oder des Esters davon die Anwesenheit von Sauerstoff und eines Elektronendonors notwendig. Vorzugsweise wird Sauerstoff durch Kontakt der Reaktionsmischung umfassend Enzyme(e) oder Zellen und Substrate mit atmosphärischer Luft, alternativ mit reinem Sauerstoff oder mit Sauerstoff angereicherter atmosphärischer Luft verfügbar gemacht, besonders bevorzugt durch Rühren der Reaktionsmischung, während diese mit atmosphärischer Luft, alternativ mit reinem Sauerstoff oder mit Sauerstoff angereicherter atmosphärischer Luft, in Kontakt steht.

Zur optimalen Versorgung der Cytochrom P450-Monooxygenase der CYP153-Familie mit Elektronen aus dem Reduktionsmittel, bevorzugt NADH, ist es bevorzugt, dass die Monoxygenase zusammen mit funktionell mit ihr wechselwirkender Ferredoxin-Reduktase und funktionell mit ihr wechselwirkendem Ferredoxin verwendet wird. Dabei kann es sich um isolierte oder bei der Verwendung eines Ganzzellkatalysators um co-exprimierte Polypeptide oder um N- oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

Bei allen erfindungsgemäß eingesetzten Enzymen kann es sich um Zellen umfassend entsprechende enzymatisch aktive Polypeptide oder deren Lysate oder Präparationen der Polypeptide in sämtlichen Aufreinigungsstufen, vom kruden Lysat bis zum reinen Polypeptid, oder Ganzzellkatalysatoren handeln. Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. Zur Expression der Polypeptide können sämtliche dem Fachmann zur Verfügung stehende Expressionssysteme verwendet werden. Zur Aufreinigung kommen chromatographische Verfahren in Frage, beispielsweise die affinitätschromatographische Aufreinigung eines mit einem Tag versehenen rekombinanten Proteins unter Verwendungen eines immobilisierten Liganden, beispielsweise eines Nickelions im Falle eines Histidin-Tags, von immobilisiertem Glutathion im Falle einer an das Zielprotein fusionierten Glutathion-S-Transferase oder von immobilisierter Maltose im Falle eines Tags umfassend Maltose-bindendes Protein. Für zahlreiche biotechnologisch bedeutsame Arten von Zellen, z.B. *E. coli,* sind geeignete Verfahren und Vektoren bekannt, die für die Expression oder Überexpression eines Nukleinsäuremoleküls verwendet werden können, beispielsweise die Vektoren vom Typ pET oder pGEX und für deren Expression geeignete Zellen (B A Moffatt, and F W Studier (1986) J. Mol. Biol. 189, 113-130, A H Rosenberg, B N Lade, D Chui, S Lin, J J Dunn, and F W Studier (1987) Gene 56, 125-135 und F W Studier, A H Rosenberg, J J Dunn, and J W Dubendorff (1990) Meth. Enzymol. 185, 60-89.

Die aufgereinigten Enzyme können entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, beispielsweise durch Sulfhydryl-Kopplungs-Chemie (z.B. Kits der Firma Pierce). Zellmembranassoziierte oder in Zellmembranen eingebettete Enzyme können in Form von Membranpräparationen oder solubilisiert verwendet werden.

Im Falle der Verwendung wenigstens eines Ganzzellkatalysators ist bei längerer Reaktionszeit zu beachten, dass die Bedingungen mit der Lebensfähigkeit der wenigstens einen als Ganzzellkatalysator eingesetzten Zelle kompatibel sind. Der Fachmann kann Standardwerken, beispielsweise Fuchs/Schlegel (2007) Allgemeine Mikrobiologie, 2008, Georg Thieme Verlag, Bedingungen und Lösungen entnehmen, die die Erhaltung solcher Zellen in einem lebensfähigen Zustand ermöglichen.

In einer bevorzugten Ausführungsform versteht man unter dem Begriff "Ganzzellkatalysator", wie hierin verwendet, eine intakte, lebensfähige und metabolisch aktive Zelle, die eine erwünschte enzymatische Aktivität bereitstellt. Der Ganzzellkatalysator kann das zu verstoffwechselnde Substrat, im Falle der vorliegenden Erfindung den Alkohol oder das daraus entstehende Oxidationsprodukt, entweder ins Zellinnere transportieren, wo es von cytosolischen Enzymen verstoffwechselt wird, oder er kann das Enzym von Interesse auf seiner Oberfläche präsentieren, wo es gegenüber Substraten im Medium direkt exponiert ist. Dem Fachmann sind zahlreiche Systeme zur Herstellung von Ganzzellkatalysatoren bekannt, beispielsweise aus der DE 60216245.

Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. In einer bevorzugten Ausführungsform handelt es sich bei einem "Polypeptid der AlkL-Familie", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) und Varianten davon.

Für eine Reihe von Anwendungen empfiehlt sich die Verwendung isolierter Enzyme. In einer bevorzugten Ausführungsform bedeutet der Begriff "isoliert", wie hierin verwendet, dass das Enzym in reinerer und/oder aufkonzentrierter Form vorliegt als in seiner natürlichen Quelle. In einer bevorzugten Ausführungsform gilt das Enzym als isoliert, wenn es ein Polypeptidenzym ist und mehr als 60, 70, 80, 90 oder bevorzugt 95 % des Massenproteinanteils der entsprechenden Präparation ausmacht. Dem Fachmann sind zahlreiche Verfahren zur Messung der Masse eines Proteins in einer Lösung bekannt, beispielsweise die visuelle Abschätzung anhand der Dicke von entsprechenden Proteinbanden auf SDS-Polyacrylamidgelen, NMR-Spektroskopie oder massenspektrometriebasierten Verfahren.

Bei den erfindungsgemäß verwendeten Enzymen handelt es sich bevorzugt um rekombinante Enzyme. In einer bevorzugten Ausführungsform wird unter dem Begriff "rekombinant", wie hierin verwendet, verstanden, dass das entsprechende Nukleinsäure-Molekül in der Natur nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen.

In einer bevorzugten Ausführungsform handelt es sich bei der als Ganzzellkatalysator oder bei der als Expressionssystem verwendeten Zelle um eine prokaryotische, bevorzugt um eine bakterielle Zelle. Es ist erfindungsgemäß bevorzugt, dass aufgrund der guten genetischen Zugänglichkeit Mikroorganismen eingesetzt werden; ausgewählt aus der Gruppe der Bakterien, besonders aus der Gruppe enthaltend, bevorzugt bestehend aus, , *Magnetococcus, Mariprofundus, Acetobacter, Acidiphilium, Afipia, Ahrensia, Asticcacaulis, Aurantimonas, Azorhizobium, Azospirillum, Bacillus, Bartonella, tribocorum, Beijerinckia, Bradyrhizobium, Brevundimonas, subvibrioides, Brucella, Caulobacter, Chelativorans, Citreicella, Citromicrobium, Corynebacterium, Dinoroseobacter, Erythrobacter, Fulvimarina, Gluconacetobacter, Granulibacter, Hirschia, Hoeflea, Hyphomicrobium, Hyphomonas, Ketogulonicigenium, Labrenzia, Loktanella, Magnetospirillum, Maricaulis, Maritimibacter, Mesorhizobium, Methylobacterium, Methylocystis, Methylosinus, Nitrobacter, Novosphingobium, Oceanibulbus, Oceanicaulis, Oceanicola, Ochrobactrum, Octadecabacter, Oligotropha, Paracoccus, Parvibaculum, Parvularcula, Pelagibaca, Phaeobacter, Phenylobacterium, Polymorphum, Pseudovibrio, Rhodobacter, Rhodomicrobium, Rhodopseudomonas, Rhodospirillum, Roseibium, Roseobacter, Roseomonas, Roseovarius, Ruegeria, Sagittula, Silicibacter, Sphingobium, Sphingomonas, Sphingopyxis, Starkeya, Sulfitobacter, Thalassiobium, Xanthobacter, Zymomonas, Agrobacterium, Rhizobium, Sinorhizobium, Anaplasma, Ehrlichia, Neorickettsia, Orientia, Rickettsia, Wolbachia, Bordetella, Burkholderia, Cupriavidus, taiwanensis, Lautropia, Limnobacter, Polynucleobacter, Ralstonia, Chromobacterium, Eikenella, corrodens, Basfia, Kingella, Laribacter, Lutiella, Neisseria, Simonsiella, Achromobacter, Acidovorax, Alicycliphilus, Aromatoleum, Azoarcus, Comamonas, Dechloromonas, Delftia, Gallionella, Herbaspirillum, Herminiimonas, Hylemonella, Janthinobacterium, Leptothrix, Methylibium, Methylobacillus, Methylophilales, Methyloversatilis, Methylovorus, Nitrosomonas, Nitrosospira, Oxalobacter, Parasutterella, Polaromonas, Polaromonas, Pusillimonas, Rhodoferax, Rubrivivax, Sideroxydans, Sutterella, wadsworthensis, Taylorella, Thauera, Thiobacillus, Thiomonas, Variovorax, Verminephrobacter, Anaeromyxobacter, Bdellovibrio, bacteriovorus, Bilophila, Desulfarculus, Desulfatibacillum, Desulfobacca, Desulfobacterium, Desulfobulbus, Desulfococcus, Desulfohalobium, Desulfitobacterium, Desulfomicrobium, Desulfonatronospira, Desulfotalea, Desulfovibrio, Desulfuromonas, Geobacter, Haliangium, Hippea, Lawsonia, Myxococcus, Pelobacter, Plesiocystis, Sorangium, Stigmatella, Syntrophobacter, Syntrophus, Arcobacter, Caminibacter, Campylobacter, Helicobacter, Nitratifractor, Nitratiruptor, Sulfuricurvum, Sulfurimonas, Sulfurospirillum, Sulfurovum, Wolinella, Buchnera, Blochmannia, Hamiltonella, Regiella, Riesia, Citrobacter, Cronobacter, Dickeya, Edwardsiella, Enterobacter, Erwinia, Escherichia, Klebsiella, Pantoea, Pectobacterium, Proteus, Providencia, Rahnella, Salmonella, Serratia, Shigella, Sodalis, Wigglesworthia, Glossina, Xenorhabdus, Yersinia, Acidithiobacillus, Acinetobacter, Aeromonas, Alcanivorax, Alkalilimnicola, Allochromatium, Alteromonadales, Alteromonas, Baumannia, Beggiatoa, Bermanella, Carsonella, Ruthia, Vesicomyosocius, Cardiobacterium, Chromohalobacter, Colwellia, Congregibacter, Coxiella, Dichelobacter, Endoriftia, Enhydrobacter, Ferrimonas, Francisella, Glaciecola, Hahella, Halomonas, Halorhodospira, Halothiobacillus, Idiomarina, Kangiella, Legionella, Marinobacter, Marinomonas, Methylobacter, Methylococcus, Methylomicrobium, Methylophaga, Moraxella, Moritella, Neptuniibacter, Nitrococcus, Pseudoalteromonas, Psychrobacter, Psychromonas, Reinekea, Rickettsiella, Saccharophagus, Shewanella, Succinatimonas, Teredinibacter, Thioalkalimicrobium, Thioalkalivibrio, Thiomicrospira, Tolumonas, Vibrionales, Actinobacillus, Aggregatibacter, Gallibacterium, Haemophilus, Histophilus, Mannheimia, Pasteurella, Azotobacter, Cellvibrio, Pseudomonas, Aliivibrio, Grimontia, Photobacterium, Photobacterium, Vibrio, Pseudoxanthomonas, Stenotrophomonas, Xanthomonas, Xylella, Borrelia, Brachyspira, Leptospira, Spirochaeta, Treponema, Hodgkinia, Puniceispirillum, Liberibacter, Pelagibacter, Odyssella, Accumulibacter,* insbesondere B. *subtilis,* B. *megaterium,* C. *glutamicum, E. coli, Pseudomonas sp., Pseudomonas fluorescens, Pseudomonas putida, Pseudomonas stutzeri" Acinetobacter* sp., *Burkholderia* sp., *Burkholderia thailandensis,* Cyanobakterien, *Klebsiella* sp., *Klebsiella oxytoca, Salmonella sp., Rhizobium sp.* und *Rhizobium meliloti,* wobei E. *coli* besonders bevorzugt ist.

In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. In einer bevorzugten Ausführungsform ist unter dem Begriff "Alkanhydroxylase-Aktivität", wie hierin verwendet, die Fähigkeit zu verstehen, die Hydroxylierung von Alkanen oder unsubstituierten linearen Alkylresten umfassend wenigstens fünf, bevorzugt zwölf Kohlenstoffstoffreste zu katalysieren. In einer weiteren bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante, die bevorzugt Alkanhydroxylaseaktivität aufweist.

Die Verwendung von Cytochrom P450-Monooxygenasen der CYP153-Familie zur Hydroxylierung von Alkanen ist im Stand der Technik beschrieben, ebenso Enzymtests zur Bestimmung der Enzymaktivität und Verfahren zur Expression und Aufreinigung (Scheps, D., Malca, H., Hoffmann, B., Nestl, B. M, und Hauer, B. (2011) Org. Biomol. Chem., 9, 6727). Neben einem zu oxidierenden Alkan oder unsubstituiertem linearen Alkylrest umfassend wenigstens fünf, bevorzugt zwölf Kohlenstoffstoffreste umfassen die für die Reaktion des Enzyms umfassenden Substrate Sauerstoff und Elektronen, die in Form von NADH bevorzugt über die anderen beiden Komponenten, Ferredoxin und eine Ferredoxin-Reduktase, auf die Oxidase übertragen werden. Scheps *et al.* (2011) sowie Roome, P. W., Jr., Philley, J. C., und Peterson (1983) J. Biol. Chem. 258, 2593, Roome, P.W., and Peterson, J. A. (1988), Arch. Biochem. Biophys., 266, 41 und Peterson, J. A., Lorence, M. C., und Amarneh, B. (1990) J. Biol. Chem, 265, 6066 offenbaren auch Verfahren zur Gewinnung von Ferredoxin und Ferredoxin-Reduktase in funktioneller Form.

Erfindungsgemäß wird in Schritt b) eine Alkoholdehydrogenase zur Oxidation des in Schritt a) entstandenen Fettsäurealkohols eingesetzt. Alkoholdehydrogenasen stellen eine seit Jahrzehnten in der Biochemie im Zusammenhang mit brauereitechnischen Fermentationsprozessen stark beachtete und biotechnologisch hochrelevante Enzymklasse dar, die verschiedene Gruppen von Isoformen umfasst. So existieren membrangebundene, flavinabhängige Alkoholdehydrogenasen vom *Pseudomonas putida* GPO1 AlkJ-Typ, die Flavocofaktoren statt NAD⁺ verwenden. Eine weitere Gruppe umfasst eisenhaltige, gegenüber Sauerstoff empfindliche Alkoholdehydrogenasen, die in Bakterien und in inaktiver Form in Hefe gefunden werden. Eine andere Gruppe umfasst NAD⁺-abhängige Alkoholdehydrogenasen, unter ihnen zinkhaltige Alkoholdehydrogenasen, bei denen das aktive Zentrum ein cysteinkoordiniertes Zinkatom aufweist, das das Alkoholsubstrat fixiert. In einer bevorzugten Ausführungsform wird unter dem Begriff "Alkoholdehydrogenase", wie hierin verwendet, ein Enzym verstanden, das ein Aldehyd bzw. Keton zu dem entsprechenden primären bzw. sekundären Alkohol oxidiert. Bevorzugt handelt es sich bei der Alkoholdehydrogenase im erfindungsgemäßen Verfahren um eine NAD⁺-abhängige Alkoholdehydrogenase, d.h. eine Alkoholdehydrogenase, die NAD⁺ als Cofaktor zur Oxidation des Alkohols bzw. NADH zur Reduktion des entsprechenden Aldehyds bzw. Ketons verwendet. In der bevorzugtesten Ausführungsform handelt es sich bei der Alkoholdehydrogenase um eine NAD⁺-abhängige, zinkhaltige Alkoholdehydrogenase.

In einer weiteren bevorzugten Ausführungsform handelt es sich bei der Alkoholdehydrogenase um eine Alkoholdehydrogenase Oxidoreduktase der Glucose-Methanol-Cholin-Oxidoreduktase-Familie. In einer bevorzugten Ausführungsform wird unter dem Begriff "Oxidoreduktase der Glucose-Methanol-Cholin-Oxidoreduktase-Familie", wie hierin verwendet, eine Alkoholdehydrogenase verstanden, die FAD als Cofaktor umfasst und bevorzugt das Enzym aus *Pseudomonas putida* (Datenbankcode CAB69081) oder eine Variante davon darstellt. Beispiele umfassen SEQ ID NO 26, 27, 28 und 29 sowie das Enzym aus *Pseudomonas putida* (Datenbankcode CAB69081).

In einer weiteren bevorzugten Ausführungsform handelt es sich bei der Alkoholdehydrogenase um eine Alkoholdehydrogenase der Familie der flavinhaltigen Alkoholdehydrogenasen. In bevorzugten Ausführungsform wird unter dem Begriff "Familie der flavinhaltigen Alkoholdehydrogenasen", wie hierin verwendet, die Gruppe von Alkoholoxidasen verstanden, die ein Hämprotein vom c-Typ darstellen und FAD als Cofaktor enthalten und bevorzugt zusätzlich zur Gruppe des Enzyms mit dem Datenbankcode AAS46878.1 und Varianten davon gehören. Beispielhafte flavinhaltige Alkoholdehydrogenasen umfassen die Enzyme mit den Datenbankcodes AAS46878.1, AAS46880.1 aus *Candida tropicalis* und das Enzyme mit dem Datenbankcode CAB75351.1 aus *Candida cloacae.*

Erfindungsgemäß wird in Schritt c) eine Transaminase verwendet. In einer bevorzugten Ausführungsform wird unter dem Begriff "Transaminase", wie hierin verwendet, ein Enzym verstanden, das die Übertragung von α-Aminogruppen von einem Donor-, bevorzugt eine Aminosäure, auf ein Akzeptormolekül, bevorzugt eine α-Ketocarbonsäure, katalysiert. In einer bevorzugten Ausführungsform wird unter dem Begriff "Amindonor", wie hierin verwendet, eine L-Aminosäure verstanden, deren Aminogruppe von der Transaminase auf die α-Ketocarbonsäure unter Bildung einer Aminosäure übertragen werden kann. In einer besonders bevorzugten Ausführungsform handelt es sich bei dem Amindonor um L-Alanin. In einer bevorzugten Ausführungsform ist die Transaminase aus der Gruppe von Transaminasen und deren Varianten ausgewählt, die dadurch gekennzeichnet sind, dass sie an der Position der Aminosäuresequenz, die Val224 aus der Transaminase von *Pseudomonas putida* GB-1 (Datenbankcode YP_001668026.1) entspricht, eine Aminosäure ausgewählt aus der Gruppe umfassend Isoleucin, Valin, Phenylalanin, Methionin und Leucin aufweist, und an der Position der Aminosäuresequenz, die Gly230 aus der Transaminase von *Pseudomonas putida* GB-1 (Datenbankcode YP_001668026.1) entspricht, eine andere Aminosäure als Threonin und bevorzugt eine Aminosäure aus der Gruppe umfassend Serin, Cystein, Glycin und Alanin aufweist. In einer besonders bevorzugten Ausführungsform ist die Transaminase aus der Gruppe ausgewählt, die die ω-Transaminase aus *Chromobacterium violaceum* DSM30191, Transaminasen aus *Pseudomonas putida* GB-1, *Pseudomonas putida* W619, aus *Pseudomonas aeruginosa* PA01, *Streptomyces coelicolor* A3(2), *Pseudomonas putida* (Datenbankcode YP_001668026), *Pseudomonas putida* (Datenbankencode YP_001668026.1 or YP_001671460); Rhodobacter sphaeroides (strain ATCC 17023; Datenbankcode YP_353455) und *Streptomyces avermitilis* MA 4680 sowie Varianten davon umfasst.

In einer bevorzugten Ausführungsform wird unter dem Begriff "Alanindehydrogenase", wie hierein verwendet, ein Enzym verstanden, das die Umwandlung von L-Alanin unter Verbrauch von Wasser und NAD(P)⁺ zu Pyruvat, Ammoniak und NAD(P)H katalysiert. Bevorzugt handelt es sich bei der Alanindehydrogenase um eine intrazelluläre Alanindehydrogenase, noch bevorzugter um eine rekombinante intrazelluläre Alanindehydrogenase eines bakteriellen Ganzzellkatalysators. Beispiele umfassen die Enzyme aus *Rhizobium leguminosarum* (Datenbankcode YP_002975437), *Bacillus megaterium* (Datenbankcode YP_003565624), *Rhodobacter capsulatus* (Datenbankcode ADE84249.1) und *Bacillus subtilis* (Datenbankcode NP_391071).

Während die Alkoholdehydrogenase in Schritt b), sofern es sich um eine NAD(P)-abhängige handelt, pro umgesetztes Substratmolekül ein Molekül des Redoxcofaktors NAD(P)H verbraucht, oxidiert die Aminodehydrogenase NAD(P)H. Besonders vorteilhaft ist daher die Verwendung eines Systems, bei dem Alkoholdehydrogenase und Aminosäuredehydrogenase den gleichen Redoxcofaktor umsetzen. NADP-abhängige Alkoholdehydrogenasen umfassen das Enzym aus *E. coli* (YjgB, Datenbankcode ZP_07117674) und ein weiteres Enzym aus E. *coli* (YahK, Datenbankcode BAE76108.1). NAD-abhängige Alkoholdehydrogenasen umfassen ein Enzym aus *E. coli* (AdhP, Datenbankcode ZP_07145023), das Enzym aus *Bacillus subtilis* (Datenbankcode NP_391071), das Enzym aus *Bacillus stearothermophilus* (Datenbankcode P42328.1) und das Enzym aus *Rhizobium leguminosarum* (Datenbankcode YP_002975437). NADP-abhängige Alanindehydrogenasen umfassen das Enzym aus *Rhodobacter capsulatus* (Datenbankcode ADE84249.1). NAD-abhängige Alanindehydrogenasen umfassen die Alanindehydrogenase aus *Bacillus subtilis subsp. subtilis* str. 168 (Datenbankcode NP_391071).

Die Lehre der vorliegenden Erfindung kann nicht nur unter Verwendung der bzw. auf die exakten Aminosäure- oder Nukleinsäuresequenzen der hierin beschriebenen biologischen Makromoleküle ausgeführt bzw. angewendet werden, sondern auch unter Verwendung von oder auf Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Wird erfindungsgemäß ein Ganzzellkatalysator eingesetzt, so ist bevorzugt, dass es sich dabei um eine Zelle handelt, die eine ihrem Wildtyp gegenüber verringerte Aktivität wenigstens eines Enzyms aufweist, das eine der Reaktionen der β-Oxidation von Fettsäuren katalysiert, wobei das Enzym bevorzugt aus der Gruppe ausgewählt ist, die Fettsäureimporter, Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase umfasst. Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen (Y Fujita, H Matsuoka, and K Hirooka (2007) Mol. Microbiology 66(4), 829-839). Im weiteren Sinn beginnt sie mit der Aufnahme einer Fettsäure in die Zelle, im Falle von *E. coli* durch den Transporter FadL (P N Black (1991) J. Bacteriol. 173, 435-442), der sie durch die äußere bzw. innere Membran der Gram-negativen Bakterienzelle schleust und das FadD-Genprodukt (P N Black, C C DiRusso, A K Metzger, and T L Heimert (1992) J. Biol. Chem. 267, 25513-25520), das die Fettsäure in Form des CoA-Esters ins Cytosol freisetzt. Dort wird die Fettsäure, sofern die Bedingungen es erfordern, zunächst an der β-Position des CoA-Fettsäureesters durch eine Acyl-CoA-Dehydrogenase, im Falle von *E. coli* FadE (J. W. Campbell & J. E. Cronan (200) J. Bacteriol. 184, 3759-3764), oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E. coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E. coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. In einer bevorzugten Ausführungsform wird unter dem Begriff "Enzym, das eine der Reaktionen der β-Oxidation von Fettsäuren katalysiert" ein jegliches Enzym verstanden, das direkt mit dem Fettsäuresubstrat oder einem auf dem Weg zum Acetyl-CoA daraus entstehenden Molekül wechselwirkt, bevorzugt es als Substrat erkennt, und seine Umwandlung zu einem auf diesem Abbauweg näher am Acetyl-CoA liegenden Stoffwechselprodukt katalysiert, bevorzugt einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. In einer besonders bevorzugten Ausführungsform wird unter dem Begriff "Enzym, das eine der Reaktionen der β-Oxidation von Fettsäuren katalysiert", wie hierin verwendet, jedes Enzym aus der Gruppe verstanden, die die Genprodukte FadA, FadB, FadD, FadL und FadE aus *E. coli* und/oder deren Varianten oder Homologa aus anderen Organismen umfasst. Die Genprodukte FadA, FadB, FadD, FadL und FadE aus *E. coli* ebenso wie Varianten und Homologa aus zahlreichen weiteren biotechnologisch nutzbaren Organismen und ihre Nukleinsäure- und Polypeptidssequenzen sind im Stand der Technik beschrieben, beispielsweise FadA unter Zugangsnummer AP009048.1, FadB unter Zugangsnummer BAE77457.1, FadD unter Zugangsnummer BAA15609.1, FadE unter Zugangsnummer BAA77891.2 und FadL unter Zugangsnummer BAA16205.1

Mit der Entwicklung moderner genetischer, mikrobiologischer und molekularbiologischer Methoden stehen dem Fachmann zahlreiche Werkzeuge zur Verfügung, mit denen er die Aktivität von in lebenden Zellen vorhandenen Enzymen routinemäßig messen und beeinflussen kann. Zur Bestimmung der Aktivität eines Enzyms, die in Form einer Suspension, eines Pellets vorliegt oder in prozessierter Form einer Zellkultur entnommen sein kann, können enzymatische Standardtests verwendet und ausgewertet werden, wie es in Lehrbüchern, beispielsweise A Cornish-Bowden (1995), Fundamentals of Enzym Kinetics, Portland Press Limited, beschrieben ist. Der Stand der Technik offenbart zahlreiche Tests, die speziell für die Messung der Aktivität von Enzymen geeignet sind, die eine der Reaktionen der β-Oxidation von Fettsäuren katalysieren, beispielsweise in K Kameda & W D Nunn (1981) J. Biol. Chem. 256, 5702-5707, Hi Marrakchi, W E DeWolf, C Quinn, J West, B J Polizzi, C Y So et al. (2003) Biochem. J. 370, 1055-1062, S Lobo, G Florova, and K A Reynolds (2001) Biochemistry 40 (39), 11955-64 und X Yu, T Liu, F Zhu, and C Khosla (2011) PNAS, *elektronische Veröffentlichung vor Drucklegung.* Auch routinemäßig anwendbare Verfahren zur Verringerung der Aktivität eines Enzyms in einer Zelle, beispielsweise durch ungerichtete Mutagenese von Zellen durch Exposition gegenüber radioaktiver Strahlung gefolgt von Anreicherung oder Screening der Mutanten, durch ortsgerichtete Einführung von Punktmutationen oder durch den Knock-out eines chromosomal in eine Zelle integrierten für ein aktives Enzym kodierendes Gen sind im Stand der Technik beschrieben, beispielsweise in Sambrook/Fritsch/Maniatis (1989): Molecular Cloning: A Laboratory Manual, Cold Spring Harbor Laboratory Press, 2nd edition oder in Fuchs/Schlegel (2007) Allgemeine Mikrobiologie, 2008, Georg Thieme Verlag. Im besonderen Fall der Fad-Genprodukt bietet sich zu Verringerung der Aktivität auch die Überexpression eines transkriptionellen Repressors, beispielsweise von FadR (Y Fujita, H Matsuoka, and K Hirooka (2007) Mol. Microbiology 66(4), 829-839). an. Auch eine Aktivitätsverringerung auf der Basis von RNA-Interferenz (T Tuschl (2001) ChemBioChem 2: 239-145) oder unter Verwendung von spezifischen Inhibitoren ist möglich. In einer bevorzugten Ausführungsform bedeutet die Formulierung "wobei die Zelle eine ihrem Wildtyp gegenüber verringerte Aktivität" eines Enzyms aufweist, wie hierin verwendet, dass die Aktivität des Enzyms in der veränderten Zelle gegenüber der Aktivität des gleichen Enzyms in einer Wildtyp-Zelle verringert ist. In einer bevorzugten Ausführungsform beträgt die relative Verringerung in der Reihenfolge zunehmender Bevorzugung 5, 10, 20, 40, 50, 75, 90, 95, 99 oder mehr Prozent der Aktivität. In einer besonders bevorzugten Ausführungsform ist keine Aktivität des Enzyms gegenüber dem Hintergrund mehr nachweisbar.

Wird erfindungsgemäß ein Ganzzellkatalysator verwendet, so ist es weiterhin vorteilhaft, wenn es sich bei dem Ganzzellkatalysator um eine Zelle handelt, die eine relativ zum Wildtyp der Zelle verringerte Aktivität wenigstens einer endogenen Aldehyddehydrogenase handelt. In einer bevorzugten Ausführungsform wird unter dem Begriff "endogene Aldehyddehydrogenase", wie hierin verwendet, ein Enzym verstanden, das in der Lage ist, die Oxidation eines Aldehyds zur entsprechenden Carbonsäure zu katalysieren, und das natürlich im Genom des Wildtyps der verwendeten Zelle vorhanden ist. Ein Beispiel für eine für *E. coli* endogene Alkoholdehydrogenase stellen das Enzym mit dem Datenbankcode BAA15032.1 (AldA) und Varianten davon dar.

Die vorliegenden Anmeldung umfasst ein Sequenzprotokoll mit den folgenden Polypeptid (Polyp)- und Nukleotid (DNA)-Sequenzen:

| SEQ ID NO | Typ | Beschreibung |
|---|---|---|
| 1 | Polyp | AlkL aus *Pseudomonas oleovorans* (Datenbankcode Q00595) |
| 2 | DNA | AlkL aus *Pseudomonas oleovorans* (Datenbankcode Q00595) |
| 3 | Polyp | AlkL aus *Pseudomonas putida* (Datenbankcode CAB69081) |
| 4 | DNA | AlkL aus *Pseudomonas putida* (Datenbankcode CAB69081) |
| 5 | Polyp | AlkL aus *Marinobacter aquaeolei* VT8 (Datenbankcode YP_957722) |
| 6 | DNA | AlkL aus *Marinobacter aquaeolei* VT8 (Datenbankcode YP_957722) |
| 7 | Polyp | AlkL aus *Oceanicaulis alexandrii* HTCC2633 (Datenbankcode ZP_00953584) |
| 8 | DNA | AlkL aus *Oceanicaulis alexandrii* HTCC2633 (Datenbankcode ZP_00953584) |
| 9 | Polyp | AlkL aus *Marinobacter manganoxydans* Mnl7-9 (Datenbankcode ZP_09158756) |
| 10 | DNA | AlkL aus *Marinobacter manganoxydans* Mnl7-9 (Datenbankcode ZP_09158756) |
| 11 | Polyp | AlkL aus *Caulobacter* sp. K31 (Datenbankcode YP_001672217) |
| 12 | DNA | AlkL aus *Caulobacter* sp. K31 (Datenbankcode YP_001672217) |
| 13 | Polyp | Ferredoxin-Reduktase aus *Alcanivorax borkumensis* SK2 (Datenbankcode YP_691923) |
| 14 | DNA | Ferredoxin-Reduktase aus *Alcanivorax borkumensis* SK2 (Datenbankcode YP_691923) |
| 15 | Polyp | Ferredoxin aus *Alcanivorax borkumensis* SK2 (Datenbankcode YP_691920) |
| 16 | DNA | Ferredoxin aus *Alcanivorax borkumensis* SK2 (Datenbankcode YP_691920) |
| 17 | Polyp | NAD-abhängige Alkoholdehydrogenase aus *Escherichia coli* MS 187-1 (Datenbankcode ZP_07145023) |
| 18 | DNA | NAD-abhängige Alkoholdehydrogenase aus *Escherichia coli* MS 187-1 (Datenbankcode ZP_07145023) |
| 19 | Polyp | CYP153-Familie aus *Alcanivorax borkumensis* SK2 (Datenbankcode YP_691921) |
| 20 | DNA | CYP153-Familie aus *Alcanivorax borkumensis* SK2 (Datenbankcode YP_691921) |
| 21 | Polyp | LL(I/L)(V/I)GGNDTTRN |
| 22 | Polyp | Alanindehydrogenase aus *Bacillus subtilis subsp. subtilis* str. 168 (Datenbankcode NP_391071) |
| 23 | DNA | Alanindehydrogenase aus *Bacillus subtilis subsp. subtilis* str. 168 (Datenbankcode NP_391071) |
| 24 | Polyp | Transaminase aus *Chromobacterium violaceum* ATCC 12472 (Datenbankcode NP_901695) |
| 25 | DNA | Transaminase aus *Chromobacterium violaceum* ATCC 12472 (Datenbankcode NP_901695) |
| 26 | Polyp | Oxidoreduktase der Glucose-Methanol-Cholin-Oxidoreduktase-Familie (Deletion von AlkJ Q00593) |
| 27 | DNA | Oxidoreduktase der Glucose-Methanol-Cholin-Oxidoreduktase-Familie (Deletion von AlkJ Q00593) |
| 28 | Polyp | Oxidoreduktase der Glucose-Methanol-Cholin-Oxidoreduktase-Familie YP_694430 (Ab_AlkJ) |
| 29 | DNA | Oxidoreduktase der Glucose-Methanol-Cholin-Oxidoreduktase-Familie YP_694430 (Ab_AlkJ) |
| 30 | Polyp | Ferredoxin-Reduktase (YP_957889(Maqu_FdOR) |
| 31 | DNA | Ferredoxin-Reduktase (YP_957889(Maqu_FdOR) |
| 32 | Polyp | Ferredoxin-Reduktase (BAE78453 (Ac_FdOR)) |
| 33 | DNA | Ferredoxin-Reduktase (BAE78453 (Ac_FdOR)) |
| 34 | Polyp | Ferredoxin YP_957887 (Maqu_Fd) |
| 35 | DNA | Ferredoxin YP_957887 (Maqu_Fd) |
| 36 | Polyp | Ferredoxin BAE78451 (Ac_Fd) |
| 37 | DNA | Ferredoxin BAE78451 (Ac_Fd) |
| 38 | Polyp | Oxidoreduktase der Glucose-Methanol-Cholin-Oxidoreduktase-Familie aus *Caulobacter sp.* K31 (Datenbankcode ABZ74557.1) |
| 39 | DNA | Oxidoreduktase der Glucose-Methanol-Cholin-Oxidoreduktase-Familie aus *Caulobacter sp.* K31 (Datenbankcode ABZ74557.1) |
| 40 | Polyp | Flavinhaltige Alkoholdehydrogenase aus *Candida tropicalis* (Datenbankcode AAS46878.1) |
| 41 | DNA | Flavinhaltige Alkoholdehydrogenase aus *Candida tropicalis* (Datenbankcode AAS46878.1) |
| 42 | Polyp | CYP153-Familie aus *Marinobacter aquaeolei* VT8 (Datenbankcode YP_ YP_957888) |
| 43 | DNA | CYP153-Familie aus *Marinobacter aquaeolei* VT8 (Datenbankcode YP_ YP_957888) |
| 44 | Polyp | CYP153-Familie aus Acinetobacter sp. OC4 (Datenbankcode YP_ YP_957888) |
| 45 | DNA | CYP153-Familie aus Acinetobacter sp. OC4 (Datenbankcode YP_ YP_957888) |
| 46 | Polyp | Alkoholdehydrogenase von *Pseudomonas putida* (Datenbankcode CAB54054) |
| 47 | DNA | Alkoholdehydrogenase von *Pseudomonas putida* (Datenbankcode CAB54054) |
| 48 | Polyp | Aminotransferase von *Pseudomonase putida* (Datenbankcode YP_001668026) |
| 49 | DNA | Aminotransferase von *Pseudomonase putida* (Datenbankcode YP_001668026) |

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Verfahren zur Oxidation einer Fettsäure oder eines Esters davon der Formel (I)
H₃C - (CH₂)ₙ - COOR (I),
wobei R aus der Gruppe ausgewählt ist, die H, Methyl, Ethyl, Propyl und Butyl umfasst, wobei n 0 bis 30, bevorzugt 6 bis 24 ist,
umfassend den Schritt:
a) Oxidieren der Fettsäure oder des Esters davon durch Kontaktieren mit einer Cytochrom P450-Monooxygenase der CYP153-Familie in Anwesenheit von molekularem Sauerstoff und NAD(P)H,
wobei die Fettsäure oder der Ester davon bevorzugt die Formel (I)
H₃C - (CH₂)ₙ - COOR (I),
wobei R aus der Gruppe ausgewählt ist, die H, Methyl, Ethyl, Propyl und Butyl umfasst, wobei n 0 bis 30, bevorzugt 6 bis 24 ist,
aufweist.

2. Verfahren nach Anspruch 1, weiter umfassend die Schritte:
b) Weiteres Oxidieren der oxidierten Fettsäure oder des Esters davon aus Schritt a) durch Kontaktieren mit einer Alkoholdehydrogenase,
c) Aminieren der weiter oxidierten Fettsäure oder des Esters davon aus Schritt b) durch Kontaktieren mit einer Transaminase in Anwesenheit eines Amindonors, bevorzugt Alanin,
wobei Schritt c) optional in Anwesenheit einer Alanindehydrogenase, von Ammonium und NAD(P)H erfolgt

3. Verfahren nach einem der Ansprüche 1 oder 2, wobei die Cytochrom P450-Monooxygenase der CYP153-Familie die Peptidsequenz LL(I/L)(V/I)GGNDTTRN aufweist und es sich bevorzugt um die Cytochrom P450-Monooxygenase der CYP153-Familie aus *Alcanivorax borkumensis* SK2 (Datenbankcode YP_691921) oder eine Variante davon handelt.

4. Verfahren nach einem der Ansprüche 1 bis 3, wobei es sich bei der Alkoholdehydrogenase um eine NAD(P)⁺-abhängige Alkoholdehydrogenase, bevorzugt um die NAD-abhängige Alkoholdehydrogenase aus *Escherichia coli* MS 187-1 (Datenbankcode ZP_07145023) oder eine Variante davon, oder um eine Oxidoreduktase der Glucose-Methanol-Cholin-Oxidoreduktase-Familie, bevorzugt die aus *Pseudomonas putida* GB-1 (Datenbankcode CAB54054.1) oder eine Variante davon, oder um eine flavinhaltige Alkoholdehydrogenase, bevorzugt die flavinhaltige Alkoholdehydrogenase aus *Candida tropicalis* (Datenbankcode AAS46878.1) oder eine Variante davon, handelt.

5. Verfahren nach einem der Ansprüche 1 bis 4, wobei in Schritt a) zusätzlich eine Ferredoxin-Reduktase, bevorzugt die Ferredoxin-Reduktase aus *Alcanivorax borkumensis* SK2 (Datenbankcode YP_691923) oder eine Variante davon, und/oder ein Ferredoxin, bevorzugt das Ferredoxin aus *Alcanivorax borkumensis* SK2 (Datenbankcode YP_691920) oder eine Variante davon, anwesend ist.

6. Verfahren nach einem der Ansprüche 1 bis 5, wobei Schritt c) in Anwesenheit einer Alanindehydrogenase, Ammonium und NADH erfolgt und es sich bei der Alanindehydrogenase um die Alanindehydrogenase aus *Bacillus subtilis subsp. subtilis* str. 168 (Datenbankcode NP_391071) oder eine Variante davon handelt.

7. Verfahren nach einem der Ansprüche 1 bis 6, wobei wenigstens ein Enzym aus der Gruppe umfassend Cytochrom P450-Monooxygenase der CYP153-Familie, Alkoholdehydrogenase, Transaminase, Alanindehydrogenase, Ferredoxin und Ferredoxin-Reduktase rekombinant in Form von einem Ganzzellkatalysator bereitgestellt wird.

8. Verfahren nach Anspruch 7, wobei sämtliche in wenigstens einem der Schritte a), b) oder c) mit der Fettsäure oder dem Ester davon, der weiter oxidierten Fettsäure oder dem Ester davon aus Schritt b) oder der aminierten weiter oxidierten Fettsäure oder dem Ester davon aus Schritt c) kontaktierten oder anwesenden Enzyme aus der Gruppe umfassend Cytochrom P450-Monooxygenase der CYP153-Familie, Alkoholdehydrogenase, Transaminase, Alanindehydrogenase, Ferredoxin und Ferredoxin-Reduktase rekombinant in Form eines oder mehr als eines Ganzzellkatalysators bereitgestellt werden.

9. Verfahren nach einem der Ansprüche 7 bis 8, wobei der Ganzzellkatalysator zusätzlich ein Polypeptid der AlkL-Familie, bevorzugt ein AlkL aus der Gruppe umfassend AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) oder eine Variante davon exprimiert.

10. Verfahren nach einem der Ansprüche 7 bis 9, wobei es sich bei dem Ganzzellkatalysator um eine Zelle handelt, die eine ihrem Wildtyp gegenüber verringerte Aktivität wenigstens eines Enzyms aufweist, das eine der Reaktionen der β-Oxidation von Fettsäuren katalysiert, wobei das Enzym bevorzugt aus der Gruppe ausgewählt ist, die Fettsäureimporter, Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase umfasst.

11. Verfahren nach einem der Ansprüche 6 bis 10, wobei die Alanindehydrogenase in Schritt c) so ausgewählt ist, dass sie den von der Alkoholdehydrogenase in Schritt b) oxidierten Redoxcofaktor, bevorzugt NAD⁺ oder NADP⁺, reduziert.

12. Ganzzellkatalysator exprimierend eine rekombinante Cytochrom P450-Monooxygenase der CYP153-Familie, eine rekombinante Alkoholdehydrogenase, eine rekombinante Transaminase und optional ein oder mehr als ein rekombinantes Enzym aus der Gruppe umfassend Alanindehydrogenase, Ferredoxin und Ferredoxin-Reduktase.

13. Ganzzellkatalysator nach Anspruch 12, wobei der Ganzzellkatalysator zusätzlich ein Polypeptid der AlkL-Familie, bevorzugt ein AlkL aus der Gruppe umfassend AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) oder eine Variante davon, exprimiert.

14. Ganzzellkatalysator nach einem der Ansprüche 12 bis 13, wobei es sich bei dem Ganzzellkatalysator um eine Zelle handelt, die eine ihrem Wildtyp gegenüber verringerte Aktivität wenigstens eines Enzyms aufweist, das eine der Reaktionen der β-Oxidation von Fettsäuren katalysiert, wobei das Enzym bevorzugt aus der Gruppe ausgewählt ist, die Fettsäureimporter, Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase umfasst.

15. Ganzzellkatalysator nach einem der Ansprüche 12 bis 14, wobei der Ganzzellkatalysator eine Ferredoxin-Reduktase und ein Ferredoxin exprimiert.

16. Ganzzellkatalysator nach einem der Ansprüche 12 bis 15, wobei der Ganzzellkatalysator eine Alanindehydrogenase epxrimiert, wobei es sich um die Alanindehydrogenase aus *Bacillus subtilis subsp. subtilis* str. 168 (Datenbankcode NP_391071) oder eine Variante davon handelt.

17. Ganzzellkatalysator nach einem der Ansprüche 12 bis 16, wobei die Cytochrom P450-Monooxygenase der CYP153-Familie die Peptidsequenz LL(I/L)(V/I)GGNDTTRN aufweist und/oder es sich um die Cytochrom P450-Monooxygenase der CYP153-Familie aus *Alcanivorax borkumensis* SK2 (Datenbankcode YP_691921) oder eine Variante davon handelt, und es sich bei der Ferredoxin-Reduktase um die Ferredoxin-Reduktase aus *Alcanivorax borkumensis* SK2 (Datenbankcode YP_691923) oder eine Variante davon handelt und es sich bei dem Ferredoxin um das Ferredoxin aus *Alcanivorax borkumensis* SK2 (Datenbankcode YP_691920) oder eine Variante davon handelt.

18. Ganzzellkatalysator oder Verfahren nach einem der Ansprüche 1 bis 17, wobei es sich bei der Alkoholdehydrogenase um eine NAD(P)⁺-abhängige Alkoholdehydrogenase, um eine Oxidoreduktase der Glucose-Methanol-Cholin-Oxidoreduktase-Familie oder um eine flavinhaltige Alkoholoxidase handelt, bevorzugt um eine NAD(P)⁺-abhängige Alkoholdehydrogenase, am bevorzugtesten um die NAD-abhängige Alkoholdehydrogenase aus *Escherichia coli* MS 187-1 (Datenbankcode ZP_07145023) oder eine Variante davon handelt.

19. Ganzzellkatalysator oder Verfahren nach einem der Ansprüche 1 bis 18, wobei es sich bei der Transaminase um die Transaminase aus *Pseudomonas putida* GB-1 (Datenbankcode YP_001668026.1) oder eine Variante davon handelt.

20. Ganzzellkatalysator oder Verfahren nach einem der Ansprüche 1 bis 19, wobei es sich bei der Fettsäure um eine ungesättigte oder verzweigte Fettsäure handelt.

21. Ganzzellkatalysator oder Verfahren nach einem der Ansprüche 1 bis 20, wobei wenigstens ein Enzym aus der Gruppe umfassend Cytochrom P450-Monooxygenase der CYP153-Familie, Alkoholdehydrogenase, Transaminase, Alanindehydrogenase, Ferredoxin und Ferredoxin-Reduktase rekombinant in Form von einem Ganzzellkatalysator bereitgestellt wird, oder wird die Aufgabe gelöst durch einen Ganzzellkatalysator, wobei es sich bei dem Ganzzellkatalysator um eine Zelle handelt, die eine relativ zum Wildtyp der Zelle verringerte Aktivität wenigstens einer endogenen Aldehyddehydrogenase handelt.

22. Verwendung des Ganzzellkatalysators nach einem der Ansprüche 12 bis 21 zur Oxidation und/oder Aminierung einer Fettsäure oder eines Esters davon, wobei die Fettsäure oder der Ester davon bevorzugt die Formel (I)
H₃C - (CH₂)ₙ - COOR (I),
wobei R aus der Gruppe ausgewählt ist, die H, Methyl, Ethyl, Propyl und Butyl umfasst,
wobei n 0 bis 30, bevorzugt 6 bis 24 ist,
aufweist

23. Verwendung des Ganzzellkatalysators nach Anspruch 22, wobei die Oxidation eine Mischung von Oxidationsprodukten ergibt, die, bezogen auf die Stoffmenge der umgesetzten Fettsäure oder des Esters davon, wenigstens 90 % des entsprechenden Alkohols, weniger als 1 % des entsprechenden Aldehydes und weniger als 10 % der entsprechenden Säure umfasst.

24. Verwendung des Ganzzellkatalysators nach einem der Ansprüche 22 bis 23, wobei es sich es sich bei der Fettsäure um eine ungesättigte oder verzweigte Fettsäure handelt.
